(19) Europäisches Patentamt
European Patent Office
Office européen des brevets

(11) EP 0 874 629 B1

(12) **EUROPEAN PATENT SPECIFICATION**

(45) Date of publication and mention of the grant of the patent:
**19.05.2004 Bulletin 2004/21**

(21) Application number: **96944844.8**

(22) Date of filing: **17.12.1996**

(51) Int Cl.[7]: **A61K 31/42**, C07D 261/04

(86) International application number:
**PCT/US1996/020076**

(87) International publication number:
**WO 1997/023212 (03.07.1997 Gazette 1997/29)**

(54) **ISOXAZOLINE, ISOTHIAZOLINE AND PYRAZOLINE FACTOR Xa INHIBITORS**

ISOXAZOLIN, ISOTHIAZOLIN UND PYRAZOLIN ALS FAKTOR Xa INHIBITOREN

ISOXAZOLINE, ISOTHIAZOLINE ET PYRAZOLINE INHIBITEURS DU FACTEUR Xa

(84) Designated Contracting States:
**AT BE CH DE DK ES FI FR GB GR IE IT LI LU NL PT SE**

(30) Priority: **21.12.1995 US 9508 P**
**08.05.1996 US 646903**
**12.11.1996 US 30666 P**

(43) Date of publication of application:
**04.11.1998 Bulletin 1998/45**

(73) Proprietor: **Bristol-Myers Squibb Pharma Company**
**Princeton, New Jersey 08543-4000 (US)**

(72) Inventors:
- **QUAN, Mimi, Lifen**
  **Newark, DE 19711-3019 (US)**
- **WITYAK, John**
  **West Grove, PA 19390-9439 (US)**
- **GALEMMO, Robert, Anthony, Jr.**
  **Collegeville, PA 19426-1411 (US)**
- **STOUTEN, Petrus, F., W.**
  **Wilmington, DE 19808-4051 (US)**
- **PRUITT, James, Russell**
  **Landenberg, PA 19350-9662 (US)**

(74) Representative: **Beacham, Annabel Rose et al**
**Frank B. Dehn & Co.,**
**European Patent Attorneys,**
**179 Queen Victoria Street**
**London EC4V 4EL (GB)**

(56) References cited:
**WO-A-95/14682**
**WO-A-95/14683**
**US-A- 5 262 388**

Remarks:
The file contains technical information submitted after the application was filed and not included in this specification

EP 0 874 629 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

FIELD OF THE INVENTION

This invention relates to isoxazolines which are inhibitors of Factor Xa, to pharmaceutical compositions containing these compounds, and to these compounds for use as anticoagulant agents for treatment and prevention of thromboembolic disorders.

BACKGROUND OF THE INVENTION

Stuerzebecher et al., Thrombosis Research, vol. 9, 637-646 (1976) describes comparative studies of a number of benzamidine derivatives as Factor Xa inhibitors. The most active inhibitors were 3-amidino-phenylaryl derivatives.

Tidwell et al., Thrombosis Research, vol. 19, 339-349 (1980) describes Factor Xa inhibitory activity of a series of heterocyclic aromatic mono- and di-amidines.

Stuerzebecher et al., Thrombosis Research, vol. 17, 545-548 (1980) describes Factor Xa inhibitory activity of a series of a,a'-bis-(4-amidinobenzyl)cycloalkanones, a,a'-bis-(4-aminobenzylidene)- and a,a'-bis-(3-aminobenzylidene) cycloalkanones with 5 to 8-membered rings, the corresponding non-cyclic derivatives, and derivatives containing only one amidino group.

Hauptmann et al., Blood Coagulation and Fibrinolysis, vol. 4, 577-582(1993) and Hauptmann et al., Thromb. Haemostasis, vol. 63(2), 220-223(1990) report testing of several synthetic compounds as Factor Xa inhibitors: Na-tosylglycyl-3-amidinophenylalanine methyl ester; 2,7-bis(4-amidinobenzylidene)-cycloheptanone-(1); Na-tosyl-4-amidinophenylalanine piperidide; Na-naphthylsulphonylglycyl-4-amidinophenylalanine piperidide; 4-methyl-1-$N^2$-(methyl-1,2,3,4-tethydro-8-quinolinesulphonyl-L-arginyl-2-piperidine carbonic acid; and D-phenylalanyl-L-propyl-L-arginine chloromethyl ketone.

Nagahara et al., J. Med. Chem., vol. 37, 1200-1207(1994) describes several dibasic (amidinoaryl)propanoic acid derivatives as Factor Xa inhibitors.

Daiichi EPA 0 540 051 A1, published May 5, 1993, discloses aromatic amidine derivatives, including amidino naphthylenes, amidino-indoles, amidino-benzimidazoles, and amidino-benzothiophenes, which have Factor Xa inhibitory activity.

DuPont Merck WO 95/14683 and WO 95/14682, published June 1, 1995, disclose isoxazoles and isoxaolines as antagonists of the platelet glycoprotein IIb/IIIa fibrinogen receptor. The isoxazoles and isoxaolines of WO 95/14683 are represented by the formula:

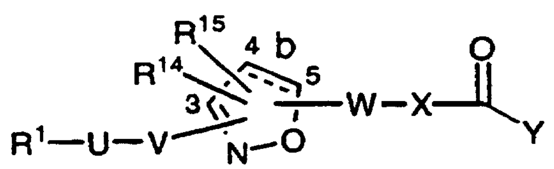

The isoxazolines of WO 95/14682 are represented by the formula:

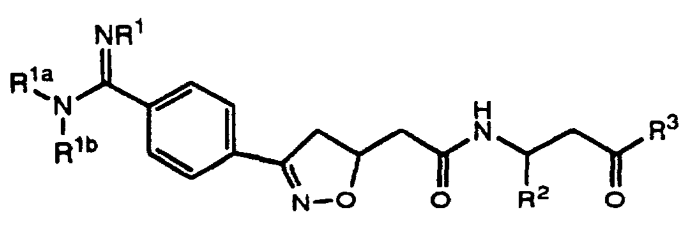

Copending, commonly assigned U.S. Patent Application Serial Number 08/449597, filed May 24, 1995, discloses isoxazoline antagonists of the platelet glycoprotein IIb/IIIa fibrinogen receptor having the formula:

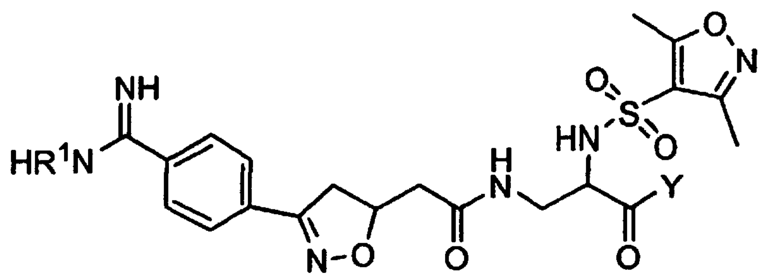

EP 53095 A and other references disclose various dianilino-pyrazoline as components of photosensitive systems.

EP 438690 and other references disclose various 1-amido-pyrazolines as pesticides, e.g., insecticides, fungicides and acaricides.

To date there have been no isoxazoline, isothiazoline or pyrazoline derivatives described as Factor Xa inhibitors.

SUMMARY OF THE INVENTION

This invention provides novel compounds of Formula (V) and (VI):

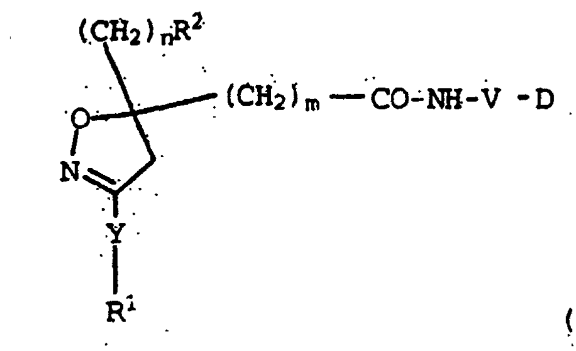

(V)

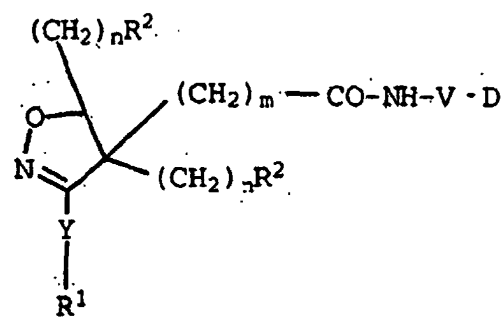

(VI)

including pharmaceutically acceptable salts thereof, and all stereoisomeric forms thereof and mixtures of such stereoisomeric forms, wherein:

$R^1$ is $C(NR^{14})NR^5R^6$ and

D is selected from

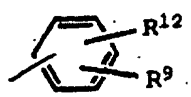 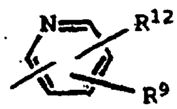 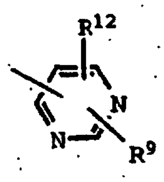

V is selected from the following:

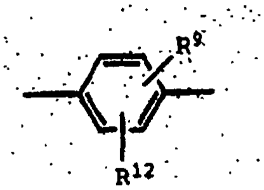 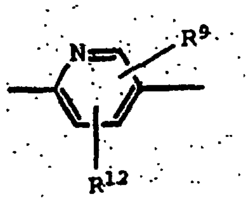 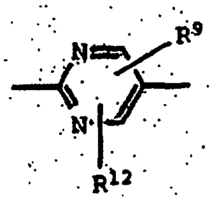

Y is selected from

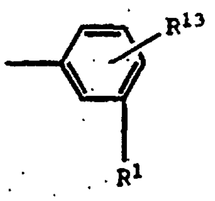 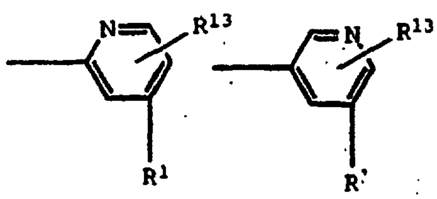 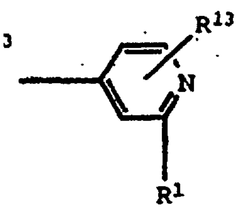

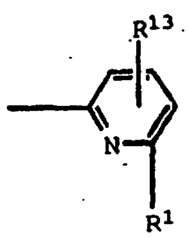 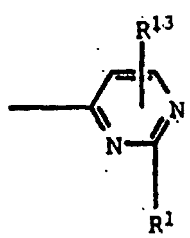 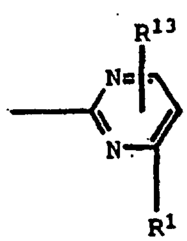

$R^2$ is selected from

H $C_1$-$C_6$ alkyl
$C_1$-$C_6$ alkoxy
$CO_2R^5$
$CONHR^5$
$CONHCH_2CO_2R^5$
$CONH(CH_2)_q$-$R^{10}$
$R^{10}$
CO-$R^5$
$COCO_2R^5$
$COCONHR^5$
$SO_nR^5$
$SO_2NHR^5$
$NHR^7$
$CH=CHCO_2R^5$
$CH=CHCONHR^5$
O-$(CH_2)_n$-$R^{10}$

$SO_n$-$(CH_2)_n$-$R^{10}$
NH-$(CH_2)_n$-$R^{10}$

$R^5$ and $R^6$ at each appearance are independently
H
$C_1$-$C_6$ alkyl
$(CH_2)_n$-phenyl

$R^7$ is selected from
H
$C_1$-$C_6$ alkyl
$SO_2R^5$
$COR^5$
$(CH_2)_r$-$R^{10}$
$(CH_2)_n$-phenyl

$R^8$ is selected from
H
$C_1$-$C_6$ alkyl
halogen
$NO_2$
$CF_3$
$OR^5$

$R^9$ is selected from
H
$C_1$-$C_6$ alkyl
halogen
$NO_2$
$NHR^7$
$SO_2NHR^{11}$
$CF_3$
$OR^5$
$CO_2R^5$
$CONR^5R^7$
CN
$(CH_2)_pNR^5R^6$
$C(NR^{14})NR^5R^6$
$NHC(NR^{14})NR^5R^6$
$NHC(NR^{14})H$
$SO_n$-$R^5$
$SO_n$-$CF_3$
imidazole, pyrazole, 1,2,3-triazole, 1,2,4-triazole and tetrazole, each optionally substituted with $CF_3$, halogen, $NO_2$, $C_1$-$C_5$ alkyl, or $C_1$-$C_5$ alkoxy;

$R^{10}$ is selected from

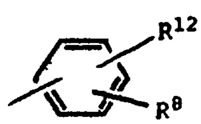
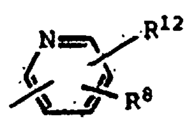
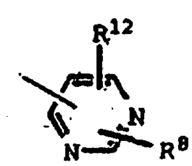
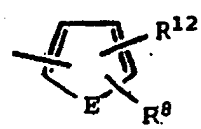

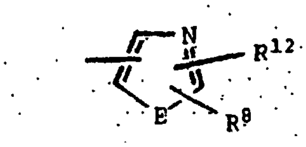 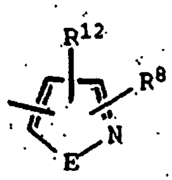 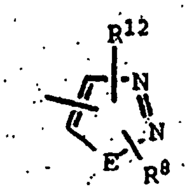 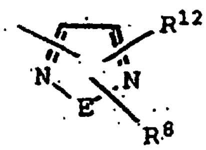

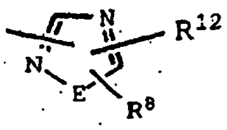 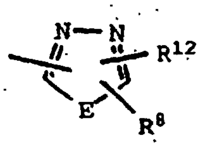 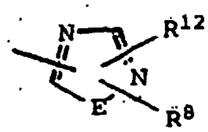 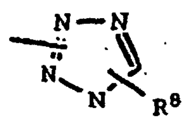

$R^{11}$ is selected from
- H
- $C_1$-$C_6$ alkyl
- $(CH_2)_n$-phenyl
- $COR^5$
- $CO_2R^5$

$R^{12}$ is selected from
- H
- $C_1$-$C_6$ alkyl
- $C_1$-$C_6$ alkoxy
- halogen
- $NO_2$
- $NHR^7$
- CN
- $CF_3$
- $SONHR^{11}$

$R^{13}$ is selected from
- H
- OH
- $C_1$-$C_{10}$ alkyl
- $C_1$-$C_{10}$ alkoxy
- nitro
- halo
- $CF_3$

$R^{14}$ is selected from
- H
- OH
- $C_1$-$C_{10}$ alkyl
- $C_1$-$C_{10}$ alkoxy
- $CO_2$-$C_1$-$C_{10}$ alkyl
- CO-$C_1$-$C_{10}$ alkyl
- CONH-$C_1$-$C_{10}$ alkyl
- CONH-phenyl
- $CO_2(CH_2)_n$-phenyl;

at each appearance each of the following are independently:

m = 0-2

n = 0-4, except that in $-SO_n-$, n = 0-2;

p = 0-1

q = 0-4

r = 1-2.

As used in this specification and the claims:

the terms "alkyl" and "alkoxy" mean straight or branched chain alkyl and straight or branched chain alkoxy, each optionally substituted with 1 to 3 substituents independently selected from halo, $C_1$-$C_6$ straight or branched alkoxy, $S(O)_n$-alkyl where alkyl is $C_1$-$C_6$ straight or branched alkyl and n is 0-2, morpholino, $C_1$-$C_6$ alkylacyloxy, $NR^5R^7$ where $R^5$ and $R^7$ are as defined in claim 1, CN, $NO_2$, and $CF_3$;
the term "phenyl" means phenyl optionally substituted with 1 to 3 substituents independently selected from halo, $C_1$-$C_6$ straight or branched alkoxy, $S(O)_n$-alkyl where alkyl is $C_1$-$C_6$ straight or branched alkyl and n is 0-2, morpholino, $C_1$-$C_6$ alkylacyloxy, $NR^5R^7$ where $R^5$ and $R^7$ are as defined in claim 1, CN, $NO_2$, and $CF_3$;
the terms "halo" and "halogen" mean chloro, fluoro, bromo and iodo.

Many compounds of this invention have one or more asymmetric centers or planes. All chiral (enantiomeric and diastereomeric) and racemic forms are included in the present invention. Many geometric isomers of olefins, C=N double bonds, and the like can also be present in the compounds, and all such stable isomers are contemplated in the present invention. The compounds may be isolated in optically active or racemic forms. It is well known in the art how to prepare optically active forms, such as by resolution of racemic forms or by asymmetric synthesis, or synthesis from optically active starting materials. All chiral, (enantiomeric and diastereomeric) and racemic forms and all geometric isomeric forms of a structure are intended, unless the specific stereochemistry or isomer form is specifically indicated.

Specific preferred compounds of this invention include the following and pharmaceutically acceptable salt forms thereof:

3-(3-amidinophenyl)-5-[[(2'-aminosulfonyl-[1,1']-biphenyl-4-yl)-methyl]aminocarbonyl]-5-(carbomethoxymethyl) isoxazoline

3-(3-amidinophenyl)-5-[[(2'-aminosulfonyl-[1,1']-biphenyl-4-yl)methyl]aminocarbonyl]-5-(aminocarbonylmethyl) isoxazoline

3-(3-amidinophenyl)-5-[(([1,1']-biphenyl-4-yl)aminocarbonyl]-5-(carbomethoxymethyl)isoxazoline

3-(3-amidinophenyl)-5-[(([1,1']-biphenyl-4-yl)aminocarbonyl]-5-(carboxymethyl)isoxazoline

3-(3-amidinophenyl)-5-[(([1,1']-biphenyl-4-yl)aminocarbonyl]-5-(aminocarbonylmethyl)isoxazoline

3-(3-amidinophenyl)-5-[(([1,1']-biphenyl-4-yl)aminocarbonyl]-5-(hydroxymethyl)isoxazoline

3-(3-amidinophenyl)-5-[(2'-aminosulfonyl-3'-n-propyl-[1,1']-biphenyl-4-yl)aminocarbonyl]-5-(carbomethoxymethyl)isoxazoline

3-(3-amidinophenyl)-5-[(2'-t-butylaminosulfonyl-[1,1']-biphenyl-4-yl)aminocarbonyl]-5-(carbomethoxymethyl)isoxazoline

3-(3-amidinophenyl)-5-[(2'-t-butylaminosulfonyl-[1,1']-biphenyl-4-yl)aminocarbonyl]-5-(aminocarbonylmethyl)isoxazoline

3-(3-amidinophenyl)-5-[(4'-amino-[1,1']-biphenyl-4-yl)aminocarbonyl]-5-(carbomethoxymethyl)isoxazoline

3-(3-amidinophenyl)-5-[(2'-trifluoromethyl-[1,1']-biphenyl-4-yl)aminocarbonyl]-5-(carbomethoxymethyl)isoxazoline

3-(3-amidinophenyl)-5-[(2'-aminosulfonyl-3-methyl-[1,1']-biphenyl-4-yl)aminocarbonyl)]-5-(carbomethoxyethyl) isoxazoline

3-(3-amidinophenyl)-5-[(2'-aminosulfonyl-3-methyl-[1,1']-biphenyl-4-yl)aminocarbonyl]-5-(carboxyethyl)isoxazoline

3-(3-amidinophenyl)-5-[(2'-aminosulfonyl-3-methyl-[1,1']-biphenyl-4-yl)aminocarbonyl]-5-(carbomethoxyethylene)isoxazoline

3-(3-amidinophenyl)-5-[[5-(2'-aminosulfonylphenyl-1-yl)pyrimidin-2-yl]aminocarbonyl]-5-(aminocarbonylmethyl) isoxazoline

3-(3-amidinophenyl)-5-[[5-(2'-aminosulfonylphenyl-1-yl)pyrimidin-2-yl]aminocarbonyl]-5-(hydroxyethyl)isoxazoline

3-(3-amidinophenyl)-5-[[5-(2'-aminosulfonylphenyl-1-yl)pyrimidin-2-yl]aminocarbonyl]-5-(methoxyethyl)isoxazoline

3-(3-amidinophenyl)-5-[[5-(2'-aminosulfonylphenyl-1-yl)pyrimidin-2-yl]aminocarbonyl]-5-(methyl)isoxazoline

3-(3-amidinophenyl)-5-[(2'-aminosulfonyl-[1,1']-biphenyl-4-yl)aminocarbonyl]-5-(carbomethoxymethyl)isoxazoline

3-(3-amidinophenyl)-5-[(2'-aminosulfonyl-[1,1']-biphenyl-4-yl)aminocarbonyl]-5-(carboxymethyl)isoxazoline

3-(3-amidinophenyl)-5-[(2'-aminosulfonyl-[1,1']-biphenyl-4-yl)aminocarbonyl]-5-(aminocarbonylmethyl)isoxazoline

3-(3-amidinophenyl)-5-[(2'-aminosulfonyl-[1,1']-biphenyl-4-yl)aminocarbonyl)-5-(hydroxyethyl)isoxazoline

3-(3-amidinophenyl)-5-[(2'-aminosulfonyl-[1,1']-biphenyl-4-yl)aminocarbonyl]-5-(carbomethoxymethylaminocarbonylmethyl)isoxazoline

3-(3-amidinophenyl)-5-[(2'-aminosulfonyl-[1,1']-biphenyl-4-yl)aminocarbonyl]-5-[(imidazole-4-yl)ethylaminocarbonylmethyl]isoxazoline

3-(3-amidinophenyl)-5-[(2'-aminosulfonyl-[1,1']-biphenyl-4-yl)aminocarbonyl]-5-(methoxyethyl)isoxazoline

3-(3-amidinophenyl)-5-[(2'-aminosulfonyl-[1,1']-biphenyl-4-yl)aminocarbonyl]-5-(methyl)isoxazoline

3-(3-amidinophenyl)-5-[(2'-aminosulfonyl-3-methyl-[1,1']-biphenyl-4-yl)aminocarbonyl]-5-(carbomethoxymethyl) isoxazoline

3-(3-amidinophenyl)-5-[(2'-aminosulfonyl-3-methyl-[1,1']-biphenyl-4-yl)aminocarbonyl]-5-(carboxymethyl)isoxazoline

3-(3-amidinophenyl)-5-[(2'-aminosulfonyl-3-methyl-[1,1']-biphenyl-4-yl)aminocarbonyl]-5-(aminocarbonylmethyl) isoxazoline

3-(3-amidinophenyl)-5-[(2'-aminosulfonyl-3-methyl[1,1']-biphenyl-4-yl)aminocarbonyl]-5-(carbomethoxymethylaminocarbonylmethyl)isoxazoline

3-(3-amidinophenyl)-5-[(2'-aminosulfonyl-3-methyl-[1,1,]-biphenyl-4-yl)aminocarbonyl]-5-(hydroxyethyl)isoxazoline

3-(3-amidinophenyl)-5-[(2'-aminosulfonyl-3-methyl-[1,1']-biphenyl-4-yl)aminocarbonyl]-5-(methoxyethyl)isoxazoline

3-(3-amidinophenyl)-5-[(2'-aminosulfonyl-3-methyl-[1,1']-biphenyl-4-yl)aminocarbonyl]-5-(methyl)isoxazoline

3-(3-amidinophenyl)-5-[(2'-aminosulfonyl-3-fluoro-[1,1']-biphenyl-4-yl)aminocarbonyl]-5-(carbomethoxymethyl) isoxazoline

3-(3-amidinophenyl)-5-[(2'-aminosulfonyl-3-fluoro-[1,1']-biphenyl-4-yl)aminocarbonyl]-5-(carboxymethyl)isoxazoline

3-(3-amidinophenyl)-5-[(2'-aminosulfonyl-3-fluoro-[1,1']-biphenyl-4-yl)aminocarbonyl]-5-(aminocarbonylmethyl) isoxazoline

3-(3-amidinophenyl)-5-[(2'-aminosulfonyl-3-fluoro-[1,1']-biphenyl-4-yl)aminocarbonyl]-5-(hydroxyethyl)isoxazoline

3-(3-amidinophenyl)-5-[(2'-aminosulfonyl-3-fluoro-[1,1']-biphenyl-4-yl)aminocarbonyl]-5-(methoxyethyl)isoxazoline

3-(3-amidinophenyl)-5-[(2'-aminosulfonyl-3-fluoro-[1,1']-biphenyl-4-yl)aminocarbonyl]-5-(methyl)isoxazoline

3-(3-amidinophenyl)-5-[[2-(2'-aminosulfonylphenyl-1-yl)pyridin-5-yl]aminocarbonyl]-5-(carbomethoxymethyl)isoxazoline

3-(3-amidinophenyl)-5-[[2-(2'-aminosulfonylphenyl-1-yl)pyridin-5-yl]aminocarbonyl]-5-(carboxymethyl)isoxazoline

3-(3-amidinophenyl)-5-[[2-(2'-aminosulfonylphenyl-1-yl)pyridin-5-yl]aminocarbonyl]-5-(aminocarbonylmethyl)isoxazoline

3-(3-amidinophenyl)-5-[[2-(2'-aminosulfonylphenyl-1-yl)pyridin-5-yl]aminocarbonyl]-5-(hydroxyethyl)isoxazoline

3-(3-amidinophenyl)-5-[[2-(2'-aminosulfonylphenyl-1-yl)pyridin-5-yl]aminocarbonyl]-5-(methoxyethyl)isoxazoline

3-(3-amidinophenyl)-5-[[2-(2'-aminosulfonylphenyl-1-yl)pyridin-5-yl]aminocarbonyl]-5-(methyl)isoxazoline

3-(3-amidinophenyl)-5-[[5-(2'-aminosulfonylphenyl-1-yl)pyridin-2-yl]aminocarbonyl]-5-(carbomethoxymethyl)isoxazoline

3-(3-amidinophenyl)-5-[[5-(2'-aminosulfonylphenyl-1-yl)pyridin-2-yl]aminocarbonyl]-5-(carboxymethyl)isoxazoline

3-(3-amidinophenyl)-5-[[5-(2'-aminosulfonylphenyl-1-yl)pyridin-2-yl]aminocarbonyl]-5-(aminocarbonylmethyl)isoxazoline

3-(3-amidinophenyl)-5-[[5-(2'-aminosulfonylphenyl-1-yl)pyridin-2-yl]aminocarbonyl]-5-(hydroxyethyl)isoxazoline

3-(3-amidinophenyl)-5-[[5-(2'-aminosulfonylphenyl-1-yl)pyridin-2-yl]aminocarbonyl]-5-(methoxyethyl)isoxazoline

3-(3-amidinophenyl)-5-[[5-(2'-aminosulfonylphenyl-1-yl)pyridin-2-yl]aminocarbonyl]-5-(methyl)isoxazoline

3-(3-amidinophenyl)-5-[[5-(2'-aminosulfonylphenyl-1-yl)pyrimidin-2-yl]aminocarbonyl]-5-(carbomethoxymethyl) isoxazoline

3-(3-amidinophenyl)-5-[[5-(2'-aminosulfonylphenyl-1-yl)pyrimidin-2-yl]aminocarbonyl]-5-(carboxymethyl)isoxazoline

3-(3-amidinophenyl)-5-[5-(2'-aminosulfonylphenyl-1-yl)pyrimidin-2-yl]aminocarbonyl-5-carbomethoxymethyl-isoxazoline

3-(3-amidinophenyl)-5-[5-(2'-aminosulfonylphenyl-1-yl)pyridin-2-yl]aminocarbonyl-5-carbomethoxymethyl-isoxazoline

3-(3-amidinophenyl)-5-[2'-aminosulfonyl-[1,1']-biphenyl-4-yl]aminocarbonyl-5-(tetrazol-1-yl)methyl-isoxazoline

3-(3-amidinophenyl)-5-[5-(2'-aminosulfonylphenyl-1-yl)pyridin-2-yl]aminocarbonyl-5-(tetrazol-1-yl)methyl-isoxazoline

3-(3-amidinophenyl)-5-[5-(2'-aminosulfonylphenyl-1-yl)pyrimidin-2-yl]aminocarbonyl-5-(tetrazol-1-yl)methyl-isoxazoline

3-(3-amidinophenyl)-5-[2'-trifluoromethyl-[1,1']-biphenyl-4-yl]aminocarbonyl-5-(tetrazol-1-yl)methyl-isoxazoline

3-(3-amidinophenyl)-5-[5-(2'-trifluoromethylphenyl-1-yl)pyridin-2-yl]aminocarbonyl-5-(tetrazol-1-yl)methyl-isoxazoline

3-(3-amidinophenyl)-5-[5-(2'-trifluoromethylphenyl-1-yl)pyrimidin-2-yl]aminocarbonyl-5-(tetrazol-1-yl)methyl-isoxazoline

3-(3-amidinophenyl)-5-[5-(2'-trifluoromethylsulfonylphenyl-1-yl)pyridin-2-yl]aminocarbonyl-5-(tetrazol-1-yl)methyl-isoxazoline

3-(3-amidinophenyl)-5-[5-(2'-trifluoromethylsulfonylphenyl-1-yl)pyrimidin-2-yl]aminocarbonyl-5-(tetrazol-1-yl)methyl-isoxazoline

3-(3-amidinophenyl)-5-[2'-aminosulfonyl-3-flouro-[1,1']-biphenyl-4-yl]aminocarbonyl-5-(tetrazol-1-yl)methyl-isoxazoline

3-(3-amidinophenyl)-5-[2'-aminosulfonyl-3-chloro-[1,1']-biphenyl-4-yl]aminocarbonyl-5-(tetrazol-1-yl)methyl-isoxazoline

3-(3-amidinophenyl)-5-[2'-trifluoromethyl-3-flouro-[1,1']-biphenyl-4-yl]aminocarbonyl-5-(tetrazol-1-yl)methyl-isoxazoline

3-(3-amidinophenyl)-5-(2'-trifluoromethyl-3-chloro-[1,1']-biphenyl-4-yl]aminocarbonyl-5-(tetrazol-1-yl)methyl-isoxazoline

3-(3-amidinophenyl)-5-[5-(2'-aminosulfonylphenyl-1-yl)pyridin-2-yl]aminocarbonyl-5-methoxymethyl-isoxazoline

3-(3-amidinophenyl)-5-[2'-methylaminosulfonyl-[1,1']-biphenyl-4-yl]aminocarbonyl-5-(tetrazol-1-yl)methyl-isoxazoline

3-(3-amidinophenyl)-5-[5-(2'-methylaminosulfonylphenyl-1-yl)pyridin-2-yl]aminocarbonyl-5-(tetrazol-1-yl)methyl-isoxazoline

3-(3-amidinophenyl)-5-[2'-methylsulfonyl-[1,1']-biphenyl-4-yl]aminocarbonyl-5-(tetrazol-1-yl)methyl-isoxazoline

3-(3-amidinophenyl)-5-[2'-methylsulfonyl-fluoro-[1,1']-biphenyl-4-yl]aminocarbonyl-5-(tetrazol-1-yl)methyl-isoxazoline

3-(3-amidinophenyl)-5-[2'-methylsulfonyl-chloro-[1,1']-biphenyl-4-yl]aminocarbonyl-5-(tetrazol-1-yl)methyl-isoxazoline

3-(3-amidinophenyl)-5-[2'-trifluoromethylsulfonyl-[1,1']-biphenyl-4-yl]aminocarbonyl-5-(tetrazol-1-yl)methyl-isoxazoline

3-(3-amidinophenyl)-5-[2'-aminosulfonyl-[1,1']-biphenyl-4-yl]aminocarbonyl-5-(imidazol-1-yl)methyl-isoxazoline

3-(3-amidinophenyl)-5-[2'-trifluoromethylsulfonyl-3-fluoro-[1,1']-biphenyl-4-yl]aminocarbonyl-5-(tetrazol-1-yl)methyl-isoxazoline

3-(3-amidinophenyl)-5-[2'-trifluoromethylsulfonyl-3-chloro-[1,1']-biphenyl-4-yl]aminocarbonyl-5-(tetrazol-1-yl)methyl-isoxazoline

3-(3-amidinophenyl)-5-[2'-aminosulfonyl-[1,1']-biphenyl-4-yl]aminocarbonyl-5-(imidazol-1-yl)methyl-isoxazoline

3-(3-amidinophenyl)-4-(2'-aminosulfonyl-[1,1']-biphenyl-4-yl)aminocarbonyl-5-methoxymethyl-isoxazoline

3-(3-amidinophenyl)-4-(2'-aminosulfonyl-[1,1']-biphenyl-4-yl)aminocarbonyl-5-trifluoromethyl-isoxazoline

3-(3-amidinophenyl)-5-(2'-aminosulfonyl-[1,1']-biphenyl-4-yl)aminocarbonyl-4-methoxymethyl-isoxazoline

## DETAILED DESCRIPTION OF THE INVENTION

### Synthesis

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Preferred methods include, but are not limited to, those described below.

A convenient method for the synthesis of the isoxazoline compounds of this invention utilizes a dipolar cycloaddition of nitrile oxides with appropriate dipolarophiles to prepare the isoxazoline rings present in compounds of Formula I (for reviews of 1,3-dipolar cycloaddition chemistry, see 1,3-Dipolar Cycloaddition Chemistry (Padwa, ed.), Wiley, New York, 1984; Kanemasa and Tsuge, Heterocycles 1990, 30, 719). Scheme 1 shows a general synthesis of 3,5-substituted-isoxazolines. An appropriately substituted hydroxylamine is treated with NCS in DMF according to the method of Liu, et al. (J. Org. Chem. 1980, 45, 3916). The resulting hydroximinoyl chloride is then dehydrohalogenated in situ using TEA to give a nitrile oxide, which undergoes a 1,3-dipolar cycloaddition to a suitably substituted alkene to afford the isoxazoline. Alternatively, the oxime may be oxidatively chlorinated, dehydrochlorinated and the resulting nitrile oxide trapped by a suitable alkene under phase transfer conditions according to the method of Lee (Synthesis 1982, 508). The isoxazoline compounds of the general formula (I) wherein the 4 and 5 positions are substituted can be prepared following the 1,3-dipolar cycloaddition methodology using a suitable 1,2-disubstituted olefin as a dipolarophile. A mixture of regioisomers is formed and the regioisomers can be separated by column chromatography. An example is shown in Scheme 2. Optically active isoxazolines can be obtained by chiral HPLC separation of the two enantiomers, or enzymatic resolution on the regioisomeric esters. It can also be obtained by the use of an appropriate chiral auxilliary on the dipolarophile as described by Olsson (J. Org. Chem. 53, 2468, 1988). The synthetic methods described above may also be used for the synthesis of compounds of this invention where Y is pyridyl or pyrimidyl derivatives in formula (I).

**Scheme 1**

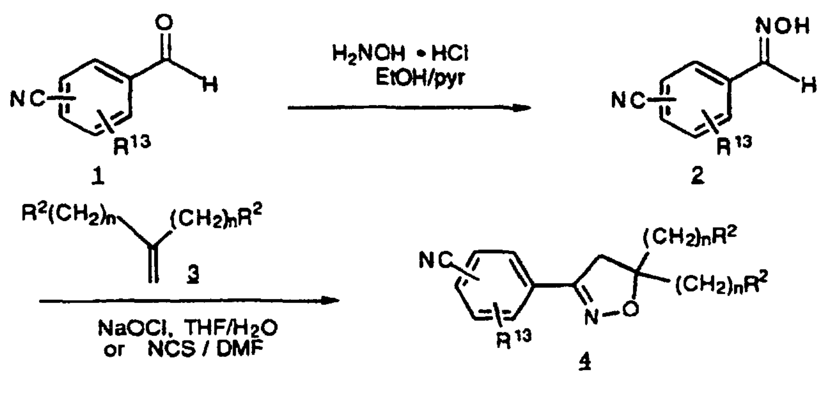

## Scheme 2

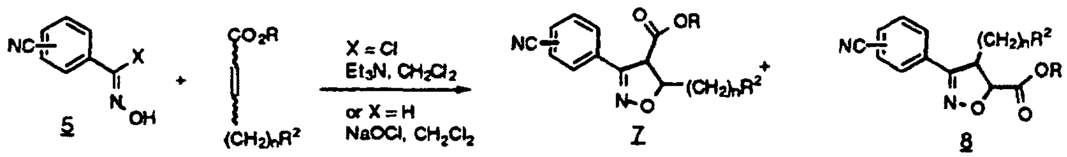


5 6 7 8

X = H, Cl
R = H, alkyl

Many isoxazoline compounds of this invention can use commercially available substituted alkenes as starting materials. Compounds with $R^2$ is acid or amide can be prepared from the commercially available alkene-esters or alkene acids. The transformations of the functional groups can be done either at the alkene stage or after the isoxazoline ring is formed. Compounds with $R^2$ is $O(CH_2)_nR$, $NH(CH_2)_nR$, $S(CH_2)_nR$, where R is $R^5$ or $R^{10}$, can be prepared from substituted allyl bromide. An example is shown in Scheme 3. The sulfoxides and sulfones can be prepared from oxidation of the thio-compounds (Scheme 3). Compounds wh $R^2$ is aromatics and heteroaromatics ($R^{10}$) can be prepared from the reaction of the bromide or iodide of the aromatics and hetreoaromatics with allyl of vinyl bromide. The C-linked aromatic and hetreoaromatic compounds can be synthesized using Zinc and Copper organometallics shown by Knochel ( Tet. Lett. 31, 4413-4416, 1990), or using palladium-catalyzed coupling of an a-stannyl acrylate to aryl iodides or triflates by Levin (Tet. Lett. 34, 6211-6214, 1993). These reactions are exemplified in Scheme 4. The N-linked heteroaromatic compounds can be prepared by alkylation of the hetroaromatics with allyl bromides. An example is shown in Scheme 5. Compounds with $R^2$ is $COCO_2R$ or COCONHR can be prepared by the method discribed by Iwanowicz (Bioorg, & Med. Chem. Lett., 2, 1607-1612, 1992).

## Scheme 3

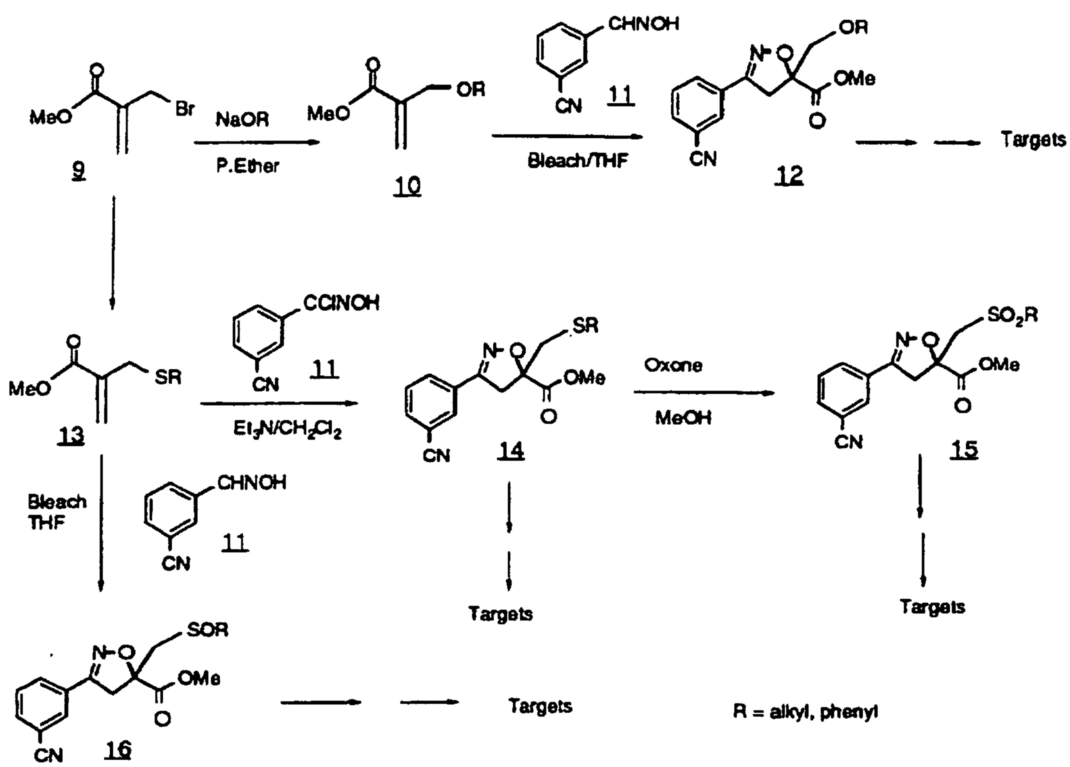


9 10 11 12 13 14 15 16

## Scheme 4

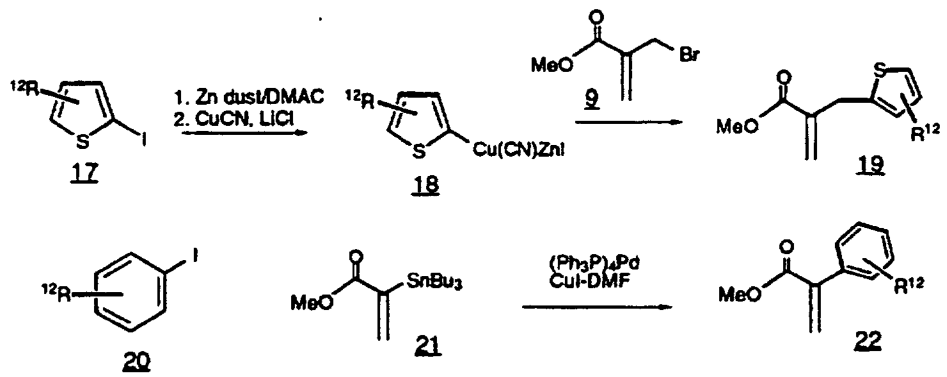


17 18 9 19

20 21 22

## Scheme 5

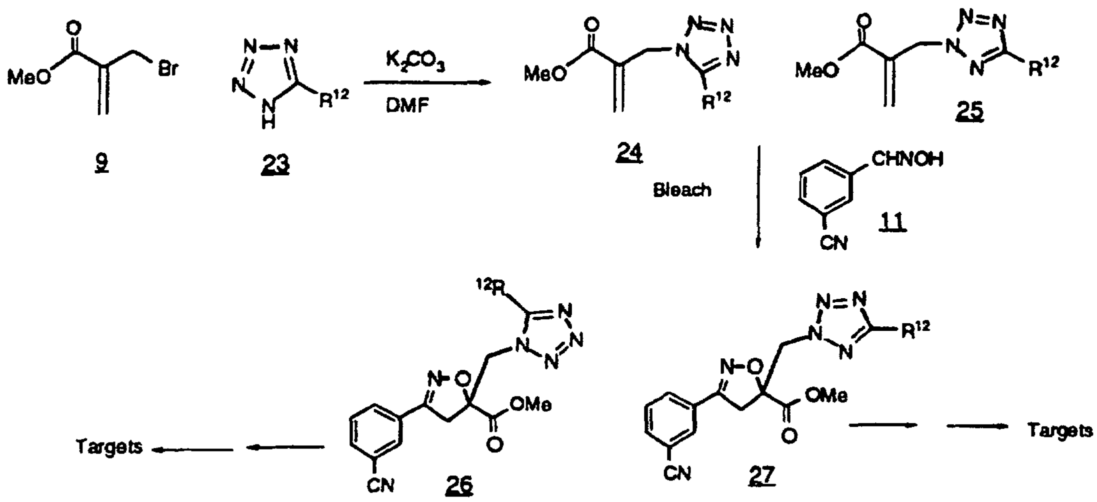


9 23 24 25 11

26 27

Appropriately substituted crotonate ester can be used as starting material for 4,5-disubstituted isoxazolines. The crotonate esters can be obtained from commertial sources or can be obtained from ethyl-4-bromocrotonate by nucleophilic displacement reactions shown in Scheme 6. Trisubstituted olefins as diplolarophiles can be obtained from ethylpropiolate by the cuprate chemistry (Scheme 7) according to the method described by Deslongchamps (Synlett, 660, 1994).

## Scheme 6

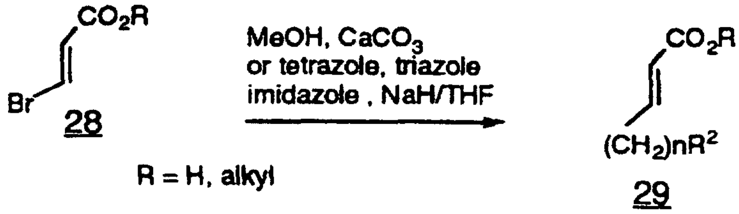


28 29

R = H, alkyl

**Scheme 7**

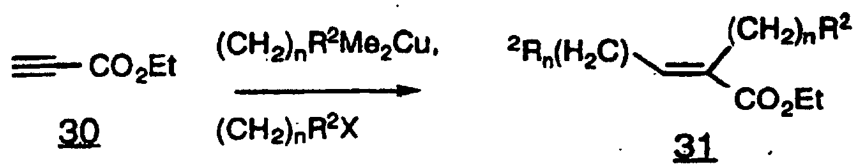


30 31

Compounds of this invention may be prepared using substituted acrylates, vinyl acetate, or crotonate as starting materials. The core ring structures can be synthesized as described above and the easter group is then coupled with an appropiate amine using standard conditions for the formation of amide bonds. The nitrile is then converted to the amidine via the imidate or thioimidate under standard conditions. Some of the compounds are prepared following the procedures described in copending commonly-assigned US. Patent application USSN 08/337920. An example of these compounds is shown in Scheme 11. The 3-substituted-isoxazoline-5-ylcarboxylic acids or 3-substituted-isoxazoline-5-ylacetic acids can be converted to the corresponding amidines first, followed by protection as the Boc-derivatives or CBZ-derivatives. They were then coupled with appropiate amines as exemplified in Scheme 12.

**Scheme 11**

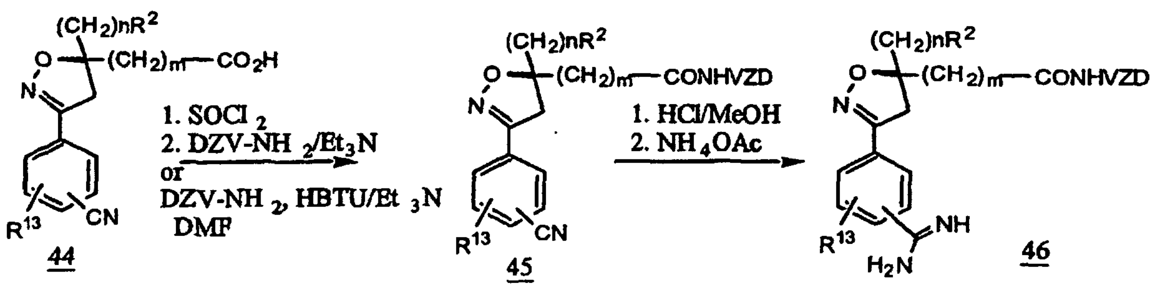


44 45 46

**Scheme 12**

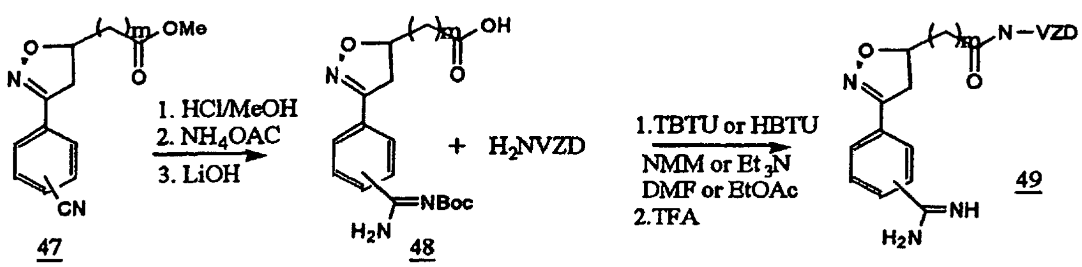


47 48 49

Some of the compounds of this invention may also be prepared as shown in Scheme 21. Itaconic anhydride

reacts with appropiate amine to give 3-carboxy-3-butenamide. The benzaldehyde oxime is oxidatively chlorinated and dehydrochlorinated. The resulting nitrile oxide then reacted with 3-carboxy-3-butenamide to yield the 3,5,5-trisubstituted isoxazoline which was then converted to the final benzamidine as described above.

**Scheme 21**

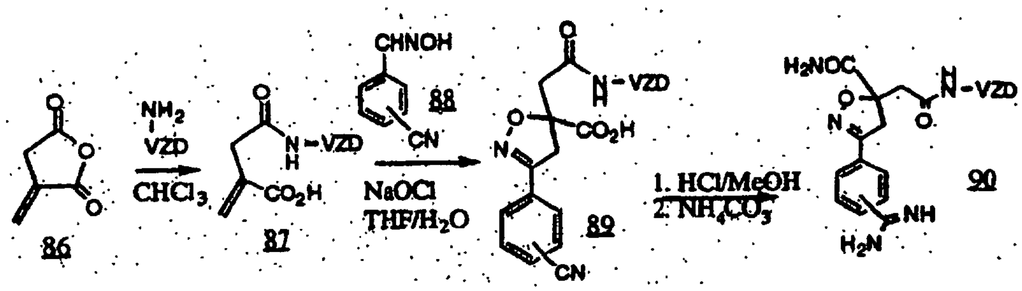


As used herein, the term "compound of formula I " or "compounds of this invention" includes pharmaceutically acceptable salts of the compounds of formula I.

The pharmaceutically acceptable salts of the compounds of Formula I include the conventional non-toxic salts or the quaternary ammonium salts of the compounds of Formula I formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the compounds of Formula I which contain a basic or acidic moiety by conventional chemical methods. Generally, the salts are prepared by reacting the free base or acid with stoichiometric amounts or with an excess of the desired salt-forming inorganic or organic acid or base in a suitable solvent or various combinations of solvents.

The pharmaceutically acceptable salts of the acids of Formula I with an appropriate amount of a base, such as an alkali or alkaline earth metal hydroxide e.g. sodium, potassium, lithium, calcium, or magnesium, or an organic base such as an amine, e.g., dibenzylethylenediamine, trimethylamine, piperidine, pyrrolidine, benzylamine and the like, or a quaternary ammonium hydroxide such as tetramethylammoinum hydroxide and the like.

As discussed above, pharmaceutically acceptable salts of the compounds of the invention can be prepared by reacting the free acid or base forms of these compounds with a stoichiometric amount of the appropriate base or acid, respectively, in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

The compounds of this invention and their preparations can be understood further by the following examples which do not constitute a limitation of the invention. In these examples, unless otherwise indicated, all temperatures are in degrees centigrade and parts and percentages are by weight.

**Example 4**

3-(3-amidinophenyl)-5-[2'-aminosulfonyl-[1,1']-biphenyl-4-yl]aminocarbonyl-5-carbomethoxy methyl-isoxazoline, Trifluoroacetic Acid Salt

Part A. Preparation of 2-(t-butylamino)sulfonylphenylboronic acid

To a solution of 34.0 g (0.16 mol) of benzene-N-(t-butylsulfonamide in 500 mL of THF under $N_2$ was added 160 mL (0.36 mol) of 2.25M n-butyllithium in hexane over 35 min, keeping the temperature between 0°-2°C. The reaction mixture was allowed to warm to room temperature over 1.5h, during which time a thick precipitate formed. Triisopropylborate (46 mL, 0.20 mol) was added, keeping the temperature below 35°C. After 1h, the reaction mixture

was cooled, 1N HCl (260 mL) was added, and the mixture was stirred for 30 min. After diluted with 520 mL of water, the mixture was extracted with 3x400 mL of ether. The combined organic extracts were extracted with 3x250 mL of 1N NaOH. The aqueous extracts were acidified to pH 1 with 6N HCl, and then extracted with 3x250 mL of ether. The ether extracts were washed with 250 mL of brine, dried over $MgSO_4$, and the solvents were removed *in vacuo* to yield 45 g of a thick oil. After addition of Toluene (45 mL), the mixture was agitated for 1h on the rotary evaporator. A small quantity of solid formed, which was used to induce partial solidification of the remaining crude product. Addition toluene (150 mL) was added, and the mixture was reduced to 1/2 volume *in vacuo*, keeping the temperature from 0°-10°C. The resulting precipitate was collected and washed with hexane, then dried under vacuum to give 24.6 g (60%) of the title compound as white crystals. m.p. 118°-119°C. $^1$HNMR ($CDCl_3$): d 1.18 (s, 9H); 5.13 (s, 1H); 6.29 (br s, 2H); 7.53 (m, 2H); 7.82 (d, 1H); 8.00 (d, 1H).

Part B. Preparation of 2'-t-butylaminosulfonyl-4-nitro-[1,1']-biphenyl

A mixture of 4.4 g (0.020 mol) of 1-bromo-4-nitrobenzene and 5.14 g (0.020 mol) of 2-(t-butylamino)sulfonylphenylboronic acid , 1.16 g of tetrakis(triphenylphosphine) palladium(0) (0.001 mol), 0.32 g of tetrabutylammonium bromide (0.001 mol), and 20 mL of 2M aqueous sodium carbonate were refluxed with 180 mL of benzene under $N_2$ for 5.5h. After cooling the mixture was diluted with methylene chloride and water. the two phases were separated and organic phase was washed with water and brine, dried over $MgSO_4$ and concentrated. The resulting solid was recrystallized from EtOAc/hexane to afford 3.25 g of the desired biphenyl.(49%). $^1$HNMR ($CDCl_3$): d 1.07(s, 9H); 3.60 (br s, 2H); 7.29 (d, 1H); 7.59 (m, 2H); 7.69 (d, 2H); 8.20 (d, 2H); 8.30 (d, 2H).

Part C. Preparation of 1-Bromo-4-t-butoxycarbonylaminobenzene

To a mixture of NaH (4.13, 0.14 mol) in THF was added 4-bromoaniline. The resulting mixture was refluxed under $N_2$ for 1h. It was then cooled and di-t-butyl dicarbonate (33 g, 0.15 mol) was added. After stirred for 1/2h, more NaH (4.13 g, 0.14 mol) was added and the reaction mixture was refluxed under $N_2$ overnight. The reaction mixture was cooled and carefully quenched with water. The mixture was extracted with ether. The combined organic solution was washed with saturated aqueous $NH_4Cl$ and saturated aqueous $NaHCO_3$, dried over $MgSO_4$, and concentrated. It was then purified by chromatography on silica gel eluted with hexane to yield 27.2 g of the desired product (80%). $^1$HNMR ($CDCl_3$): d 1.52 (s, 9H); 6.48 (br s, 1H); 7.27 (d, 2H); 7.40 (d, 2H).

Part D. Preparation of 2'-t-butylaminosulfonyl-4-amino-[1,1']-biphenyl

Method A:

A suspension of 3.00 g (0.009 mol) of 2'-t-butylaminosulfonyl-4-nitro-[1,1']-biphenyl and 0,30 g of 10% Pd/C in 90 mL of methanol was stirred at room temperature under $H_2$ (gas) (1 atm) for 1/2h. The solubility of the starting material was very poor in methanol, so 60 mL of ethyl acetate was added and the mixture was stirred for 4h. The reaction mixture was filtered through celite and the filtrate was concentrated. The crude product was recrystallized from benzene/hexane to give 2.32 g (85%) of the aniline.
$^1$HNMR ($CDCl_3$): d 0.99 (s, 9H); 3.72 (br s, 1H);3.83 (br s, 2H); 6.76 (d, 1H); 7.27 (d, 1H); 7.33 (d, 2H); 7.43 (t, 1H); 7.53 (t, 1H); 8.14 (d, 1H). MS m/e 305 $(M+H)^+$.

Method B:

A mixture of 12.8 g (0.047 mol) of 1-Bromo-4-t-butoxycarbonylaminobenzene and 12.3 g (0.048 mol) of 2-(t-butylamino)sulfonylphenylboronic acid , 3.0 g of tetrakis(triphenylphosphine) palladium(0) (0.0026 mol), 0.80 g of tetrabutylammonium bromide (0.0024 mol), and 13.8 g (0.10 mol, in 30 ml of water) potassium carbonate were refluxed with 300 mL of toluene under $N_2$ for 6h. The toluene was removed in vacuo and the residue was dissolved in methylene chloride and water. The two phases were separated and organic phase was washed with water and brine, dried over $MgSO_4$ and concentrated. the crude product was purified by chromatography on silica gel eluted with EtOAc/hexane (1:3) to afford 12.66 g of the desired biphenyl.(67%).
The protected aminobiphenyl compound (2.80 g, 6.9 mmol) was stirred with 10 mL of triflouroacetic acid and 20 mL of methylene chloride at room temperature for 2h. The solvents were removed *in vacuo*. The residue was dissolved in methylene chloride and precipitated with hexane to give 1.20 g of the desired product as the TFA salt. $^1$HNMR (DMSO-$d_6$): d 1.01(s, 9H); 6.80 (s, 1H); 7.20-7.68 (m, 8H);8.03 (d, 1H).

Part E. Preparation of 3-(3-cyanophenyl)-5-N-[2'-t-butylaminosulfonyl-[1,1']-biphenyl-4-yl]aminocarbonyl-5-carbomethoxy methyl-isoxazoline

3-(3-cyanophenyl)-5-carbomethoxy methyl-isoxazolin-5-ylcarboxylic acid (0.50 g, 1.73 mmol) was refluxed with 10 mL of acetonitrile and 0.76 mL (10.4 mmol) of thionyl chloride for 1h under $N_2$. The solvent was removed in vacuo. Residual thionyl chloride was removed by adding toluene and then evaporating to dryness. The resulting solid was dissolved in 20 mL of THF and 2'-t-butylaminosulfonyl-4-amino-[1,1']-biphenyl, TFA salt (0.60 g, 1.40 mmol) was added followed by triethylamine (1.5 mL, 10.4 mmol). The reaction mixture was stirred at RT and the reaction was completed in less than 30 min. The mixture was diluted with ethyl acetate and the solution was washed with water and brine. It was dried over $MgSO_4$ and concentrated. The crude product mixture was chromatographed on silica gel eluted with methylene chloride/ethyl acetate (9:1) to give 0.57 g of the desired product (71%). MS 575.2, $(M+H)^+$. $^1$HNMR ($CDCl_3$): d 0.95 (s, 9H); 3.03 (d, 1H); 3.27 (d, 1H);3.60 (d, 1H); 3.66 (s, 3H); 3.78 (d, 1H); 7.19 (d, 1H); 7.39-7.71 (m, 8H); 7.83 (d, 1H); 7.92 (s, 1H); 8.09 (d, 1H); 8.68 (s, 1H).

Part F. Preparation of 3-(3-cyanophenyl)-5-N-[2'-aminosulfonyl-[1,1']-biphenyl-4-yl]aminocarbonyl-5-carbomethoxy methyl-isoxazoline

3-(3-Cyanophenyl)-5-N-[2'-t-butylaminosulfonyl-[1,1']-biphenyl-4-yl]aminocarbonyl-5-carbomethoxy methyl-isoxazoline (1.12 g, 1.95 mmol) was refluxed with 25 ml of trifluoroacetic acid under $N_2$ for 1/2h. The TFA was removed in vacuo, the residue was dissolved in methylene chloride and then precipitated with ether to give 1.0 g of white solid (99%). MS 519.2, $(M+H)^+$. $^1$HNMR ($CDCl_3$): d 3.14 (d, 1H); 3.40 (d, 1H); 3.76 (s, 3H); 3.85 (dd, 2H); 4.40 (br s, 2H); 7.35 (d, 1H); 7.48-7.80 (m, 8H); 7.83 (d, 1H); 8.01 (s, 1H); 8.18 (d, 1H); 8.82 (s, 1H).

Part G. Preparation of 3-(3-amidinophenyl)-5-N-[2'-aminosulfonyl-[1,1']-biphenyl-4-yl]aminocarbonyl-5-carbomethoxy methyl-isoxazoline, Trifluoroacetic Acid Salt

3-(3-Cyanophenyl)-5-N-[2'-aminosulfonyl-[1,1']-biphenyl-4-yl]aminocarbonyl-5-carbomethoxy methyl-isoxazoline (1.2 g, 1.93 mmol) was dissolved in 90 mL of $CHCl_3$ and 20 mL of MeOH. The reaction mixture was cooled in an ice-bath, and HCl gas was bubbled-in for 30 min until the solution was saturated. The mixture was sealed and placed at 0°C for 12h. The solvents were removed in vacuo and the resulting solid was dried and used in the next step.

The imidate formed above was added with 0.92 g (12.0 mmol) of ammonium acetate and 30 mL of methanol. The mixture was sealed and stirred at RT for 12h. The crude benzamidine was purified by HPLC (C18 reversed phased) eluted with 0.5% TFA in $H_2O/CH_3CN$ to give 0.47 g of the benzamidine TFA salt (37%). MS 536.4, $(M+H)^+$. $^1$HNMR (DMSO-$d_6$): d 3.20 (m, 2H); 3.48 (s, 3H); 3.70-4.01(m, 2H); 7.20-7.32 (m, 4H); 7.52 (m, 2H); 7.72 (d, 2H); 7.88 (d, 1H); 7.98 (d, 1H); 8.05 (d, 1H); 8.07 (s, 1H); 9.24 (s, 2H); 9.40 (s, 2H) ; 10.05 (s, 1H).

**Example 5**

3-(3-amidinophenyl)-5-[[(2'-aminosulfonyl-[1,1']-biphenyl-4-yl)-methyl]aminocarbonyl]-5-(carbomethoxymethyl) isoxazoline, trifluoroacetic acid salt **(Ex.5)**

Part A. Preparation of 2'-t-butylaminosulfonyl-4-aminomethyl-[1,1']-biphenyl

2'-t-butylaminosulfonyl-4-methyl-[1,1']-biphenyl (prepared by the same method described in Part B of Exaple 1) (1.57 g, 5.18 mmol) was refluxed with N-bromosuccinamide (0.92 g, 5.18 mmol) and AIBN (0.10 g) in 50 mL of $CCl_4$ for 2h. The mixture was cooled and the precipitae was filtered-off. The filtrate was concentrated to an off-white solid. The resulting solid was dissovled in 20 mL of DMF and sodium azide (0.67 g, 10.3 mmol) was added. the mixture was heated to 100 °C for 6 h under $N_2$. The reaction mixture was cooled and pourted into water. It was extracted with EtOAc. The combined organic solution was washed with brine and dried over $MgSO_4$. It was the concentrated to a white solid. this solid was the added together with 0.2 g of Pd(OH), 0.5 mL of concentrated HCl, and 100 mL of MeOH. The mixture was placed under balloon $H_2$ for 5 h. The resulting mixture was filtered through celite and washed with MeOH. The filtrate was concentrated and precipitated with $Et_2O$ to give 1.32 g of white solid (72 %). MS (DCI) 336 $(M+NH_4)^+$, 319 $(M+H)^+$.

Part B. Preparation of 3-(3-cyanophenyl)-5-N-[2'-t-butylaminosulfonyl-[1,1']-biphenyl-4-methyl]aminocarbonyl-5-carbomethoxy methyl-isoxazoline

This compound was prepared by the same method described in Part G of Example 4 using 2'-t-butylaminosulfonyl-4-aminomethyl-[1,1']-biphenyl and 3-(3-cyanophenyl)-5-carbomethoxy methyl-isoxazolin-5-ylcarboxylic acid as the starting materials. MS (DCI) 606 $(M+NH_4)^+$.

Part C. Preparation of 3-(3-amidinophenyl)-5-[[(2'-aminosulfonyl-[1,1']-biphenyl-4-yl)-methyl]aminocarbonyl]-5-(carbomethoxymethyl)isoxazoline, trifluoroacetic acid salt (**EX 5)**.

3-(3-cyanophenyl)-5-N-[2'-t-butylaminosulfonyl-[1,1']-biphenyl-4-methyl]aminocarbonyl-5-carbomethoxy methyl-isoxazoline was subjected to the Pinner - amidine reaction protocol described in Part D of Example 1. The crude product mixture was purified by HPLC (C18 reversed phased) eluted with 0.5 % TFA in $H_2O$ and $CH_3CN$ to give Compounds Ex 5 as the TFA salt. Ex 5: MS (ESI) 518.4, $(M+H)^+$.

**Example 8**

3-(3-amidinophenyl)-5-(2'-aminosulfonyl-[1,1']-biphenyl-4-yl)aminocarbonyl-5-(tetrazol-1-yl)methyl-isoxazoline, Trifluoroacetic Acid Salt

Part A. Preparation of 3-(3-cyanophenyl)-5-Carbomethoxy-5-(tetrazol-1-yl)methyl-isoxazoline

1*H*-Tetrazole(0.89 g, 14.0 mmol) and $K_2CO_3$ were added together with 50 mL of DMF. Methyl 2-(bromomethyl) acrylate (2.5 g, 14.0 mmol) was added. The mixture was stirred at room temperature under $N_2$ for 12h. The mixture was poured into water and extracted with EtOAc. The combined organic solution was washed with brine, dried over $MgSO_4$, and then concentrated to give 1.63 g of methyl 2-(tetrazolemethyl)acrylate. This crude product mixtre was added together with 3-cyanobenzaldehyde oxime prepared as described in Example 1 (1.42 g, 9.69 mmol) and THF (50 mL). To the above mixture was added dropwise bleach (25 mL of 0.67M solution). The resulting mixture was stirred at room temperature under $N_2$ for 3h. The THF was removed. The mixture was diluted with water and extracted with EtOAc. The combined organic solution was washed with brine, dried over $MgSO_4$, and concentrated. It was purified by chromatography (silica gel, 5-15% EtOAc in $CH_2Cl_2$) to give 1.61 g of the desired product and 0.50 g of the regioisomer 3-(3-cyanophenyl)-5-Carbomethoxy-5-(tetrazol-2-yl)methyl-isoxazoline. $^1$HNMR (DMSO-$d_6$): $\delta$ 3.78 (s, 3H); 3.80-4.10 (q, 2H); 5.09-5.20 (q, 2H); 7.68 (t, 1H); 7.98 (d, 1H); 8.07 (s, 1H); 9.45 (s, 1H). MS($ES^+$) 313.1 $(M+H)^+$.

Part B. Preparation of 3-(3-cyanophenyl)-5-Carboxylic acid-5-(tetrazol-1-yl)methyl-isoxazoline

3-(3-Cyanophenyl)-5-Carbomethoxy-5-(tetrazol-1-yl-methyl)-isoxazoline (1.60 g, 5.12 mmol) was added together with 75 mL of THF. LiOH ( 12 mL of 0.5 M aqueous solution) was added. The mixture was stirred at room temperature under $N_2$ for 1h. The THF was removed. The mixture was diluted with water and acidified with concentrated HCl. It was extracted with EtOAc. The combined organic solution was washed with brine, dried over $MgSO_4$, and concentrated to a white solid (1.54 g). $^1$HNMR (DMSO-$d_6$): $\delta$ 3.70-4.02 (q, 2H); 5.02-5.18 (q, 2H); 7.67 (t, 1H); 7.97 (d, 1H); 8.04 (s, 1H); 9.42 (s, 1H). MS($ES^+$) 299 $(M+H)^+$.

Part C. Preparation of 3-(3-cyanophenyl)-5-[2'-t-Butylaminosulfonyl-[1,1']-biphenyl-4-yl]aminocarbonyl-5-(tetrazol-1-yl)methyl-isoxazoline

3-(3-Cyanophenyl)-5-Carboxylic acid-5-(tetrazol-1-yl)methyl-isoxazoline (0.55 g, 1.84 mmol) was refluxed with $CH_3CN$ (20 mL) and $SOCl_2$ (1.34 mL, 18.4 mmol) under $N_2$ for 1h. The solvent was removed. Residual $SOCl_2$ was removed by dissolving in toluene and then removing the solvent to dryness. The resulting solid was dissolved in $CH_2Cl_2$ (20 mL). 2'-t-Butylaminosulfonyl-4-amino-[1,1']-biphenyl prepared as described in Example 4 (0.28 g, 0.92 mmol) was added followed by $Et_3N$ (1.5 mL, 18.4 mmol). The mixture was stirred at room temperature under $N_2$ for 1/2 h. It was diluted with $CH_2Cl_2$ and washed with water and brine. It was dried over $MgSO_4$ and concentrated.
The desired product was the purified by chromatography (silica gel, 20% EtOAc in $CH_2Cl_2$) to give 0.59 g off-white solid. $^1$HNMR (DMSO-$d_6$): $\delta$ 1.01 (s, 9H); 3.90-4.10 (q, 2H); 5.08-5.16 (q, 2H); 6.70 (s, 1H), 7.24-7.38 (m, 3H), 7.50-7.77 (m, 5H), 7.98-8,03 (m, 3H); 8.12 (s, 1H); 9.42 (s, 1H). MS($ES^+$) 585.2 (M+H)+.

Part D. Preparation of 3-(3-amidinophenyl)-5-[2'-aminosulfonyl-[1,1']-biphenyl-4-yl]aminocarbonyl-5-(tetrazol-1-yl) methyl-isoxazoline, Trifluoroacetic Acid Salt

3-(3-Cyanophenyl)-5-[2'-t-Butylaminosulfonyl-[1,1']-biphenyl-4-yl]aminocarbonyl-5-(tetrazol-1-yl)methyl-isoxazoline (0.41 g, 0.70 mmol) was dissolved in anhydrous $CHCl_3$ (20 mL) and anhydrous $CH_3OH$ (5 mL). HCl gas was bubbled-in until the solution was saturated (about 15 min). The reaction mixture was sealed and placed in a refrigerator

for 12 h. The solvents were removed. The resulting solid was dried under vacuum. The imidate formed above was dissolved in 20 mL of anhydrous $CH_3OH$. Ammonium acetate (0.55 g, 7.0 mmol) was added. The mixture was sealed and stirred at room temperature for 12 h. The solvent was removed. The solid was dissolved in $CH_3CN/H_2O$/TFA, and purifed by reversed phase HPLC ($C_{18}$ reversed phase column, 0.5% TFA in $H_2O/CH_3CN$) to give the desired TFA salt (0.15 g). $^1$HNMR (DMSO-$d_6$) : δ 3.89-4.16 (q, 2H); 5.13-5.31 (q, 2H); 7.22-7.48 (m, 5H), 7.52-7.78(m, 5H), 7.91 (d, 1H); 8.00-8,08 (m, 3H); 9.12 (s, 2H); 9.41 (s, 2H); 9.43 (s, 1H). MS($ES^+$) 546.3 $(M+H)^+$.

**Example 10 and Example 11**

3-(3-amidinophenyl)-4-(2'-aminosulfonyl-[1,1']-biphenyl-4-yl)aminocarbonyl-5-methyl-isoxazoline, Trifluoroacetic Acid Salt

3-(3-amidinophenyl)-5-(2'-aminosulfonyl-[1,1']-biphenyl-4-yl)aminocarbonyl-4-methyl-isoxazoline, Trifluoroacetic Acid Salt

Part A. Preparation of 3-(3-cyanophenyl)-5-Carbomethoxy-4-methyl-isoxazoline and 3-(3-cyanophenyl)-4-Carbomethoxy-5-methyl-isoxazoline

To a dichloromethane ( 100mL) solution of 3-cyanophenyl-oximinochloride (2.30 g, 13.65 mmol) and methyl crotonate (1.71 g, 17.05 mmol) was added triethylamine (1.39 g, 13.65 mmol) in dichloromethane (5mL) dropwise over 0.5 h. The reaction mixture was stirred at room temperature for 12 h. It was then concentrated to a viscous oil. Chromatography (silica gel, hexane : ethyl acetate 8:2) afforded the desired 4-methylcarboxylate-isoxazoline (0.82g, 25% yield) as a colorless oil. $^1$HNMR($CDCl_3$) δ 1.47 (d, J = 9Hz, 3H), 3.77 (s, 3H), 4.09 (d, J = 4.2Hz, 1H), 5.15 (m, 1H), 7.54 (t, 1H), 7.68 (d, J = 7.8Hz, 2H), 7.94 (d, J = 8Hz, 2H). MS(ESI) 245, $(M+H)^+$. The 5-methylcarboxylate isoxazoline was also obtained (0.53g, 16% yield) as a colorless oil. $^1$HNMR($CDCl_3$) δ 1.42 (d, J = 8.5Hz, 3H), 3.81 (s, 3H), 3.96 (m, 1H), 4.83 (d, J = 4.5Hz, 1H), 7.55 (t, 1H), 7.70 (d, J = 8.0Hz, 2H), 7.95 (d, J = 7.9Hz, 2H). MS(ESI) 245, $(M+H)^+$.

Part B. Preparation of 3-(3-cyanophenyl)-4-Carboxylic acid-5-methyl-isoxazoline

The 4-isoxazoline ester was then carefully hydrolyzed (LiOH, leq.) in THF:water (4:1, 20 mL) to the carboxylic acid (0.75g, 97% yield). $^1$HNMR($CDCl_3$) δ 1.50 (d, J = 8Hz, 3H), 4.07 (d, J = 7 Hz, 1H), 5.18, (m, 1H), 7.50 (t, 1H), 7.68 (d, J = 8Hz, 2H), 7.97 (d, J = 8Hz, 2H). MS(ESI) 231 $(M+H)^+$.

Part C. Preparation of 3-(3-cyanophenyl)-4-(2'-t-butylaminosulfonyl-[1,1']-biphenyl-4-yl)aminocarbonyl-5-methyl-isoxazoline

Treatment of the acid from Part C with oxalyl chloride (leq) in dichloromethane followed by addition of a drop of DMF. Reaction mixture was stirred at room temperature for 1.5h and then concentrated to a yelloow oil. This was then redissolved in dichloromethane followed by treatment with 2'-t-Butylaminosulfonyl-4-amino-[1,1']-biphenyl prepared as described in Example 4 (1 eq) and triethyl amine (3 eq.). The reaction mixture was stirred at room temperature overnight The reaction mixture was poured into water (100mL) and then extracted with ethyl acetate (2X100mL), It was washed with brine (50mL) and dried (magnesium sulfate). Evaporation of the solvent afforded crude amide which was purified (column chromatography, silica gel $CH_2Cl_2$:MeOH, 9:1) to give 0.35 g (20% yield) colorless oil. $^1$HNMR ($CDCl_3$) δ 1.01 (s, 9H), 1.52 (d, J = 6.5Hz, 3H), 3.70 (s, 1H), 4.18 (d, J = 5.4Hz, 1H), 5.18 (m, 1H), 7.27 (dd, J = 3 and 8Hz, 1H), 7.43-7.54 (m, 7H), 7.68 (d, J = 8.5Hz, 1H), 8.03 (ds, 2H), 8.17 (sd, 2H). MS (DCI-$NH_3$) 534 $(M+NH_4)^+$.

Part D. Preparation of 3-(3-amidinophenyl)-4-(2'-aminosulfonyl-[1,1']-biphenyl-4-yl)aminocarbonyl-5-methyl-isoxazoline

The nitrile obtained in Part D was then subjected to the Pinner - amidine reaction protocol described previously to afford 0.15g (colorless crystals) of the desired benzamidine compound after reversed phase HPLC purification. $^1$HNMR(DMSO $d_6$) δ 1.44 (d, J = 7.5Hz, 3H), 4.53 (d, J = 6Hz, 1H), 5.02 (m, 1H), 7.27-7.38 (m, 5H), 7.55-7.63 (m, 3H), 7.70 (t, 1H0, 7.80 (d, J = 8.5Hz, 1H), 7.91 (d, J = 8.2hz, 1H), 8.00 (dd, J = 1.8 and 7.9Hz, 1H), 9.10 (bs, 2H), 9.44 (bs, 2H), 10.30 (s, 1H). MS (ESI) 478.3, $(M+H)^+$.

Part E. Preparation of 3-(3-amidinophenyl)-5-(2'-aminosulfonyl-[1,1']-biphenyl-4-yl)aminocarbonyl-4-methyl-isoxazoline

This compound was obtained by the same procedure described above using 3-(3-cyanophenyl)-4-Carbomethoxy-5-methyl-isoxazoline as starting material. $^1$HNMR(DMSO $d_6$) δ 1.38 (d, J = 7.7Hz, 3H), 4.31 (m, 1H), 5.08 (d, J = 5.4Hz, 1H), 7.23-7.38 (m, 5H), 7.55-7.66 (m, 2H), 7.69-7.70 (m, 2H), 7.88 (d, j = 8Hz, 1H), 8.00 (d, j = 8 Hz, 1H), 8.10 (ds, 2H), 9.20 (bs, 2H0, 9.40 (bs, 2H), 10.3 (s, 1H). MS(ESI) 478.4, $(M+H)^+$.

The compounds of Tables 1-6 were prepared by the methods of Examples 4-11. The compounds in Tables 1-6 which have asymmetric centers are racemates except where indicated otherwise by (+) or (-) in the column headed o.r. (for optical rotation) in Table 2.

## TABLE 1

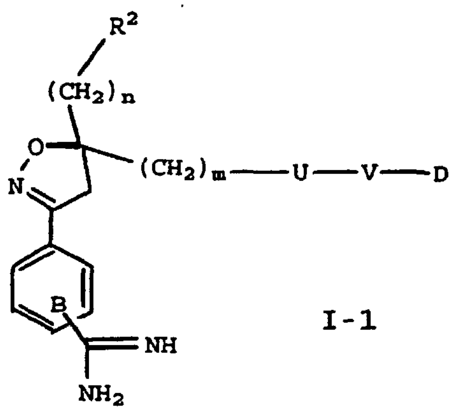

I-1

| EX# | B | m | $(CH_2)_nR^2$ | -U-V-D | MS $(M+H)^+$ |
|---|---|---|---|---|---|
| 16 | *p* | 1 | H | 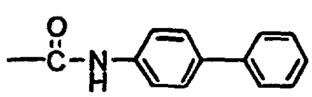 | 399.0 |
| 17 | *p* | 1 | H | 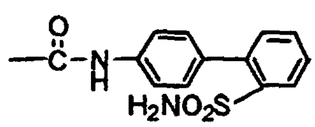 | 478.3 |
| 18 | *p* | 1 | H | 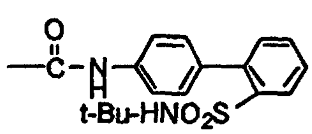 | 534.3 |

| | | | | | |
|---|---|---|---|---|---|
| 36 | *m* | 0 | $CH_2CO_2Me$ | 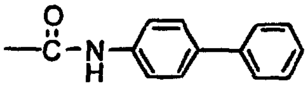 | 457.4 |
| 37 | *m* | 0 | $CH_2CO_2H$ | 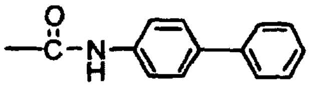 | 443.4 |
| 38 | *m* | 0 | $CH_2CONH_2$ | 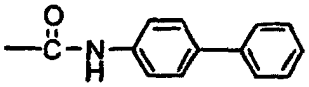 | 442.4 |
| 39 | *m* | 0 | $CH_2CH_2OH$ | 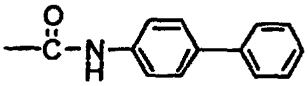 | 429.3 |
| 40 | *m* | 0 | $CH_2CO_2Me$ | 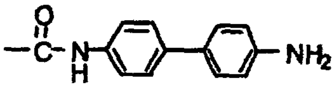 | 236.8 $(M+2H)^{2+}$ |

| | | | | | |
|---|---|---|---|---|---|
| 42 | *m* | 0 | $CH_2CO_2Me$ | 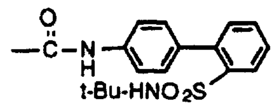 | 592.5 |
| 43 | *m* | 0 | $CH_2CONH_2$ | 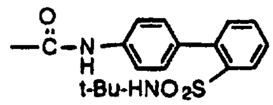 | 577.5 |
| 44 | *m* | 0 | $CH_2CO_2H$ | 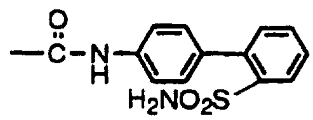 | 522.4 |
| 45 | *m* | 0 | $CH_2CONH_2$ | 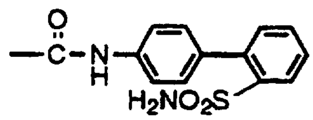 | 521.4 |
| 46 | *m* | 0 | $CH_2CH_2OH$ | 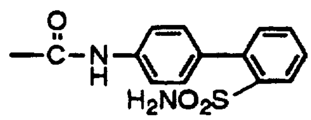 | 508.2 |
| 47 | *m* | 0 | $CH_2CH_2OMe$ | 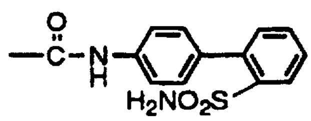 | |
| 48 | *m* | 0 | $CH_2CONHCH_2CO_2Me$ | 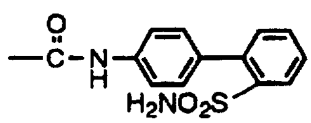 | 593.3 |
| 49 | *m* | 0 | $CH_2CONH(CH_2)_2$-4-imidazole | 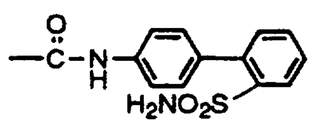 | 308.2 $(M+2H)^{2+}$ |
| 50 | *m* | 0 | $CH_2CO_2Me$ | 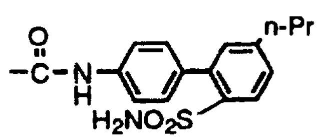 | 578.3 |
| 51 | *m* | 0 | $CH_2CO_2Me$ | 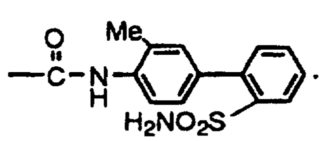 | 550.3 |

| | | | | | |
|---|---|---|---|---|---|
| 52 | *m* | 0 | $CH_2CO_2H$ | 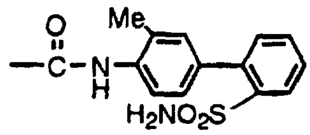 | 536.5 |
| 53 | *m* | 0 | $CH_2CONH_2$ | 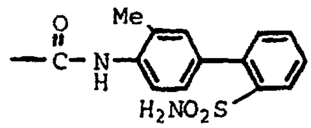 | 535.3 |
| 54 | *m* | 0 | $CH_2CONHCH_2CO_2Me$ | 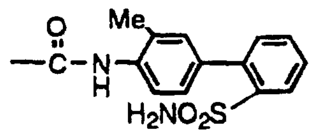 | 607.3 |
| 55 | *m* | 0 | $CH_2CO_2Me$ | 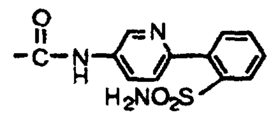 | 537.2 |
| 56 | *m* | 0 | $CH_2CO_2Me$ | 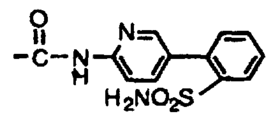 | 537.2 |
| 57 | *m* | 0 | $CH_2CO_2Me$ | 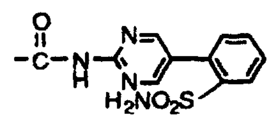 | 538.2 |
| 58 | *m* | 0 | $CH_2CO_2Me$ | 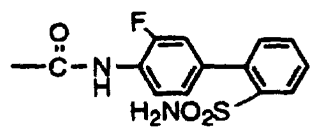 | 554.2 |
| 59 | *m* | 0 | $CH_2CO_2Me$ | 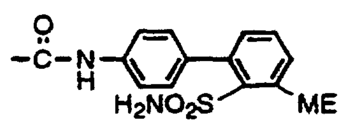 | |
| 60 | *m* | 0 | $CH_2CO_2Me$ | 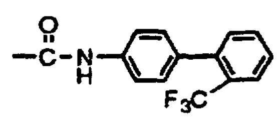 | 525.3 |
| 61 | *m* | 0 | $CH=CHCO_2Me$ | 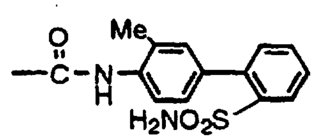 | 562.3 |

| | | | | | |
|---|---|---|---|---|---|
| 62 | *m* | 0 | $CH_2CH_2CO_2Me$ | 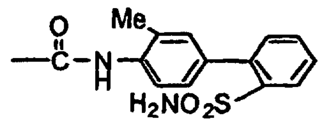 | 564.2 |

**TABLE 2**

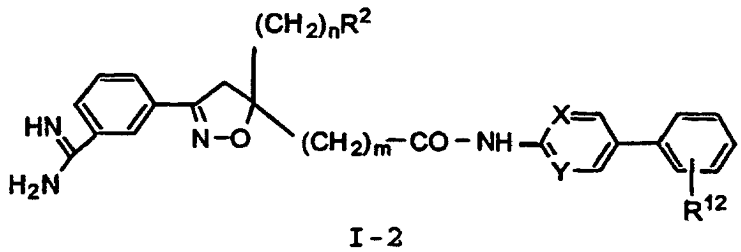

I-2

| EX# | o.r. | m | X | Y | $(CH_2)_nR^2$ | $R^{12}$ | MS $(M+H)^+$ |
|---|---|---|---|---|---|---|---|
| 68 | | 0 | N | N | $CH_2CO_2Me$ | *o*-$SO_2NH_2$ | 538.2 |
| 69 | | 0 | CH | N | $CH_2CO_2Me$ | *o*-$SO_2NH_2$ | 537.2 |
| 70 | (+) | 0 | N | N | $CH_2CO_2Me$ | *o*-$SO_2NH_2$ | 538.2 |
| 71 | (-) | 0 | N | N | $CH_2CO_2Me$ | *o*-$SO_2NH_2$ | 538.2 |

| | | | | | | | |
|---|---|---|---|---|---|---|---|
| 72 | | 0 | CF | CH | $CH_2CO_2Me$ | $o$-$SO_2NH_2$ | 554.2 |
| 73 | | 0 | CF | CH | $CH_2CO_2H$ | $o$-$SO_2NH_2$ | 540.2 |
| 74 | | 0 | CH | CH | H | $o$-$SO_2NH_2$ | 464.2 |
| 75 | | 0 | CH | N | $CH_3$ | $o$-$SO_2NH_2$ | 479.3 |
| 76 | | 0 | CH | CH | $CH_3$ | $o$-$SO_2NH_2$ | 478.2 |
| 77 | | 0 | CH | N | $CH_2OMe$ | $o$-$SO_2NH_2$ | 509.2 |
| 78 | | 0 | N | N | $CH_2SEt$ | $o$-$SO_2NH_2$ | 540.3 |
| 79 | | 0 | N | N | $CH_2SO_2Et$ | $o$-$SO_2NH_2$ | 572.4 |
| 80 | | 0 | CH | N | $CH_2SO_2Et$ | $o$-$SO_2NH_2$ | 571.3 |
| 81 | | 0 | CH | CH | $CH_2SO_2Et$ | $o$-$SO_2NH_2$ | 570.4 |
| 8 | | 0 | CH | CH | $CH_2$-tetrazol-1-yl | $o$-$SO_2NH_2$ | 546.3 |
| 82 | | 0 | CH | CH | $CH_2$-tetrazol-1-yl | $o$-$SO_2NH$-t-Bu | 602.3 |
| 83 | | 0 | CH | CH | $CH_2$-tetrazol-2-yl | $o$-$SO_2NH_2$ | 546.5 |
| 84 | | 0 | CH | CH | $CH_2$-tetrazol-2-yl | $o$-$SO_2NH$-t-Bu | 602.6 |
| 85 | | 0 | CH | N | $CH_2CO_2H$ | $o$-$SO_2NH_2$ | 523.1 |
| 86 | (-) | 0 | CH | N | $CH_2CO_2Me$ | $o$-$SO_2NH_2$ | 537.1 |
| 87 | (+) | 0 | CH | N | $CH_2CO_2Me$ | $o$-$SO_2NH_2$ | 537.3 |
| 88 | | 0 | N | N | $CH_2OMe$ | $o$-$SO_2NH_2$ | 510.3 |
| 89 | | 0 | N | N | $CH_2OEt$ | $o$-$SO_2NH_2$ | 524.3 |
| 90 | | 0 | CH | N | $CH_2OEt$ | $o$-$SO_2NH_2$ | 523.3 |
| 91 | | 0 | CH | CH | $CH_2CONH_2$ | $o$-$SO_2NH_2$ | 520.6 |
| 92 | | 0 | CH | CH | $CH_2OMe$ | $o$-$SO_2NH_2$ | 508.3 |
| 93 | | 0 | CH | CH | $CH_2OEt$ | $o$-$SO_2NH_2$ | 522.3 |
| 94 | (-) | 0 | CH | CH | $CH_2OEt$ | $o$-$SO_2NH_2$ | 522.4 |
| 95 | (+) | 0 | CH | CH | $CH_2OEt$ | $o$-$SO_2NH_2$ | 522.4 |
| 96 | (+) | 0 | CH | CH | $CH_2OEt$ | $o$-$SO_2NH$-t-Bu | 578.5 |
| 97 | | 0 | CH | N | $CH_2CO_2H$ | $o$-$SO_2NH_2$ | 524.4 |

| | | | | | | | |
|---|---|---|---|---|---|---|---|
| 98 | | 0 | CH | CH | $CH_2O$-i-Pen | *o*-$SO_2NH_2$ | 564.4 |
| 99 | | 0 | CH | CH | $CH_2Br$ | *o*-$SO_2NH_2$ | 556.3 |
| 100 | | 0 | CH | CH | $CH_2Br$ | *o*-$SO_2NH$-t-Bu | 612.4 |
| 101 | | 0 | CH | CH | $CH_2OEt$ | *o*-$SO_2NHMe$ | 536.4 |
| 102 | (-) | 0 | CH | N | $CH_2OEt$ | *o*-$SO_2NH_2$ | 523.3 |
| 103 | (-) | 0 | CH | N | $CH_2OEt$ | *o*-$SO_2NH$-t-Bu | 579.3 |
| 104 | | 0 | CH | CH | $CH_2O$-n-Pr | *o*-$SO_2NH_2$ | 536.4 |
| 105 | | 0 | CH | CH | $CH_2O$-n-Bu | *o*-$SO_2NH_2$ | 550.5 |
| 106 | | 0 | CH | N | $CH_2SEt$ | *o*-$SO_2NH_2$ | 539.3 |
| 107 | | 0 | CH | CH | $CF_3$ | *o*-$SO_2NH$-t-Bu | 588.2 |
| 108 | | 0 | CH | CH | $CF_3$ | *o*-$SO_2NH_2$ | 532.3 |
| 109 | (-) | 0 | CH | CH | $CH_2$-tetrazol-1-yl | *o*-$SO_2NH_2$ | 546.4 |
| 110 | (+) | 0 | CH | CH | $CH_2$-tetrazol-1-yl | *o*-$SO_2NH_2$ | 546.2 |
| 111 | (-) | 0 | CCl | CH | $CH_2$-tetrazol-1-yl | *o*-$SO_2NH_2$ | 580.1 |
| 112 | | 0 | CCl | CH | $CH_2$-tetrazol-1-yl | *o*-$SO_2NH_2$ | 580.1 |
| 113 | | 0 | CF | CH | $CH_2$-tetrazol-1-yl | *o*-$SO_2NH_2$ | 564.4 |
| 114 | (-) | 0 | CF | CH | $CH_2$-tetrazol-1-yl | *o*-$SO_2NH_2$ | 564.4 |
| 115 | | 0 | CH | N | $CH_2$-tetrazol-1-yl | *o*-$SO_2NH$-t-Bu | 603.5 |
| 116 | | 0 | CH | N | $CH_2$-tetrazol-1-yl | *o*-$SO_2NH_2$ | 547.4 |
| 117 | (-) | 0 | CH | N | $CH_2$-tetrazol-1-yl | *o*-$SO_2NH_2$ | 547.4 |
| 118 | | 0 | CH | CH | $CH_2CH_2OMe$ | *o*-$SO_2NH_2$ | 522.4 |
| 119 | | 0 | CH | CH | $CH_2CH_2OMe$ | *o*-$SO_2NH$-t-Bu | 578.5 |
| 120 | (-) | 0 | CH | N | $CH_2$-tetrazol-1-yl | *o*-$SO_2NH$-t-Bu | 603.6 |
| 121 | | 0 | CH | CH | $CH_2Ph$ | *o*-$SO_2NH_2$ | 554.3 |
| 122 | | 0 | CH | CH | $CH_2O$-i-Pr | *o*-$SO_2NH_2$ | 536.3 |
| 123 | (-) | 0 | CH | N | $CH_2OMe$ | *o*-$SO_2NH_2$ | 509.3 |
| 124 | (-) | 0 | CH | N | $CH_2OMe$ | *o*-$SO_2NH$-t-Bu | 565.4 |

| | | | | | | | |
|---|---|---|---|---|---|---|---|
| 125 | | 0 | CH | N | $CH_2$-tetrazol-1-yl | *o*-$SO_2NHMe$ | 561.6 |
| 126 | (-) | 0 | CH | N | $CH_2$-tetrazol-1-yl | *o*-$SO_2NHMe$ | 561.6 |
| 127 | (-) | 0 | CH | CH | $CH_2$-tetrazol-1-yl | *o*-$SO_2NH$-n-Pr | 588.6 |
| 128 | | 0 | CH | CH | $CH_2$-tetrazol-1-yl | *o*-$SO_2NH$-n-Pr | 588.4 |
| 129 | (-) | 0 | CH | CH | $CH_2$-tetrazol-1-yl | *o*-$SO_2NHMe$ | 560.4 |
| 130 | | 0 | CH | CH | $CH_2I$ | *o*-$SO_2NH_2$ | 604.3 |
| 131 | | 0 | CH | CH | $CH_2$-1-(4,5-dichloroimidazole) | *o*-$SO_2NHMe$ | 612.2 |
| 132 | | 0 | N | N | $CH_2$-tetrazol-1-yl | *o*-$SO_2NH_2$ | 548.4 |
| 133 | | 1 | CH | CH | $CO_2Me$ | *o*-$SO_2NH_2$ | 536.3 |
| 134 | | 1 | CH | CH | $CO_2Me$ | *o*-$SO_2NH$-t-Bu | 592.4 |
| 135 | | 1 | CH | N | $CO_2H$ | *o*-$SO_2NH_2$ | 523.4 |
| 136 | (-) | 1 | N | N | $CO_2H$ | *o*-$SO_2NH_2$ | 524.3 |
| 137 | (-) | 1 | CH | N | $CO_2H$ | *o*-$SO_2NH_2$ | 523.4 |
| 138 | | 1 | N | N | $CO_2H$ | *o*-$SO_2NH_2$ | 524.4 |
| 139 | | 0 | CH | CH | $CH_2CO_2Me$ | *o*-OMe | 487.3 |
| 140 | | 0 | CH | CH | $CH_2CO_2Me$ | *m*-OMe | 487.3 |
| 141 | | 0 | CH | CH | $CH_2CONH_2$ | *o*-OMe | 472.2 |
| 142 | | 0 | CH | CH | $CH_2CONH_2$ | *m*-OMe | 472.2 |
| 143 | | 0 | CH | CH | $CH_2CO_2Me$ | *m*-$CF_3$ | 525.2 |
| 144 | | 0 | CH | CH | $CH_2CONH_2$ | *m*-$CF_3$ | 510.2 |
| 145 | | 0 | CH | CH | $CH_2CONH_2$ | *m*-$SO_2Me$ | 535.3 |
| 146 | | 0 | CH | CH | $CH_2CONH_2$ | *o*-Me | 456.5 |
| 147 | | 0 | CH | CH | $CH_2CO_2Me$ | *o*-Me | 471.5 |
| 148 | | 0 | CH | CH | $CH_2CONH_2$ | *m*-Me | 456.5 |
| 149 | | 0 | CH | CH | $CH_2CO_2Me$ | *m*-Me | 471.5 |
| 150 | | 0 | CH | CH | $CH_2CO_2Me$ | *m*-$SO_2NH_2$ | 536.5 |

| | | | | | | | |
|---|---|---|---|---|---|---|---|
| 151 | | 0 | CH | CH | $CH_2CONH_2$ | $o$-$SO_2NMe_2$ | 549.4 |
| 152 | | 0 | CH | CH | $CH_2CONH_2$ | $o$-$SO_2NHMe$ | 535.4 |
| 153 | | 0 | CH | CH | $CH_2CO_2Me$ | $o$-SMe | 503.4 |
| 154 | | 0 | CH | CH | $CH_2CO_2Me$ | $o$-$SO_2Me$ | 535.4 |
| 155 | | 1 | CH | CH | $CO_2Me$ | $o$-$SO_2Me$ | 535.4 |
| 156 | | 0 | CH | CH | $CH_2CONH_2$ | $o$-$CO_2Me$ | 500.3 |
| 157 | | 0 | CH | CH | $CH_2CO_2Me$ | $o$-$CO_2Me$ | 515.4 |
| 158 | | 0 | CH | CH | $CH_2CONH_2$ | $o$-SOMe | 488.3 |
| 159 | | 0 | CH | CH | $CH_2OMe$ | $o$-$SO_2Me$ | 507.4 |
| 160 | | 0 | CH | CH | $CH_2OMe$ | $o$-$SO_2Et$ | 521.4 |
| 161 | | 0 | CH | CH | $CH_2OMe$ | $o$-$SO_2$-n-Pr | 535.4 |
| 162 | | 0 | CH | CH | $CH_2OMe$ | $o$-$SO_2$-i-Bu | 549.5 |
| 163 | | 0 | CH | CH | $CH_2$-tetrazol-1-yl | $o$-$SO_2Me$ | 545.2 |
| 164 | | 0 | CH | CH | $CH_2$-tetrazol-1-yl | $o$-$SO_2CF_3$ | |
| 165 | (-) | 0 | CH | CH | $CH_2$-tetrazol-1-yl | $o$-$CF_3$ | 535.4 |
| 166 | (-) | 0 | N | CH | $CH_2$-tetrazol-1-yl | $o$-$CF_3$ | 536.3 |
| 167 | | 0 | N | N | $CH_2$-tetrazol-1-yl | $o$-$CF_3$ | |
| 168 | | 0 | CCl | CH | $CH_2$-tetrazol-1-yl | $o$-$CF_3$ | |
| 169 | | 0 | CF | CH | $CH_2$-tetrazol-1-yl | $o$-$CF_3$ | |
| 170 | | 0 | CH | CH | $CH_2$-imidazol-1-yl | $o$-$CF_3$ | |
| 171 | | 0 | CH | CH | $CH_2$-imidazol-1-yl | $o$-$SO_2NH_2$ | |

**TABLE 3**

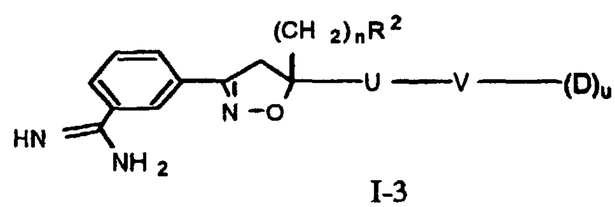

I-3

| EX# | U | $(CH_2)_nR^2$ | V-(D)u | MS $(M+H)^+$ |
|---|---|---|---|---|
| 172 | CONH | $CH_2OMe$ | 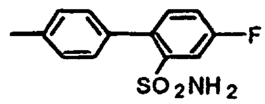 | 526.4 |
| 173 | CONH | $CH_3$ | 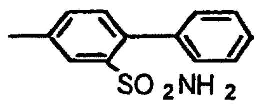 | 478.3 |
| 174 | CONH | $CH_3$ | 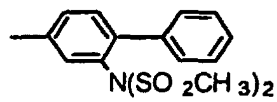 | 570.3 |
| 175 | CONH | $CH_3$ | 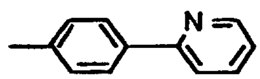 | 400.3 |
| 176 | CONH | $CH_3$ | 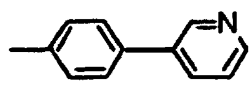 | 400.2 |
| 177 | CONH | $CH_3$ | 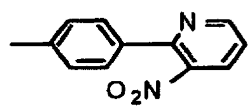 | 445.4 |
| 192 | CONH | $CH_2CO_2Me$ | 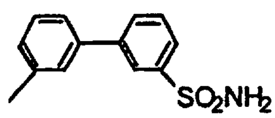 | 536.2 |
| 193 | CONH | $CH_2CO_2Me$ | 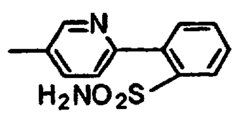 | 537.2 |

TABLE 4

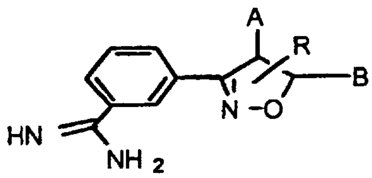

I-4

| EX# | R | A | B | MS (M+H)+ |
|---|---|---|---|---|
| 10 | H | 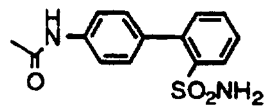 | $CH_3$ | 478.3 |
| 203 | H | 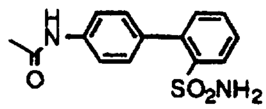 | $CH_3OCH_2$ | 508.4 |
| 204 | H | 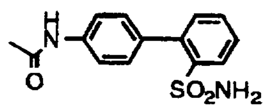 | tetrazole-1-yl-$CH_2$- | 546.4 |
| 205 | H | 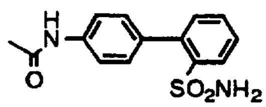 | $CF_3$ | 532.3 |
| 206 | H | 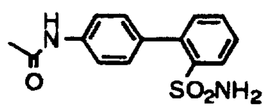 | $Si(Et)_2Me$ | 564.4 |
| 207 | 4-$CH_2OCH_3$ | 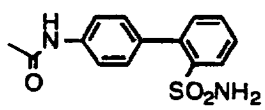 | $CH_3$ | 522.3 |
| 11 | H | $CH_3$ | 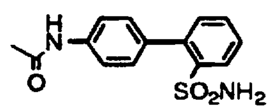 | 478.3 |
| 208 | H | $CH_3OCH_2$ | 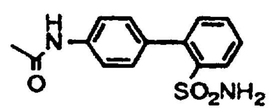 | 508.4 |
| 209 | H | $CF_3$ | 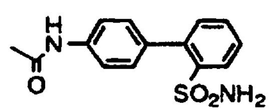 | 532.3 |
| 210 | 5-$CH_2OCH_3$ | $CH_3$ | 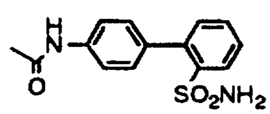 | 522.3 |

## TABLE 5

| EX# | Structures | MS (M+H)+ |
|---|---|---|

| | | |
|---|---|---|
| 211 | 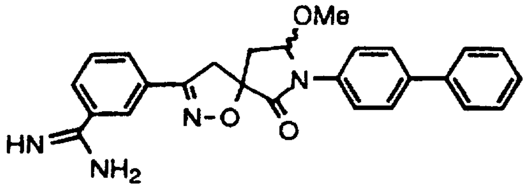 | 441.3 |
| 212 | 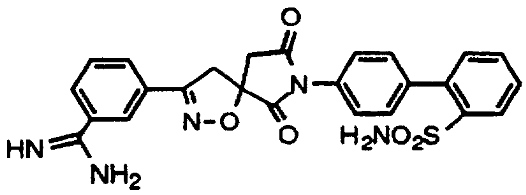 | 504.3 |
| 213 | 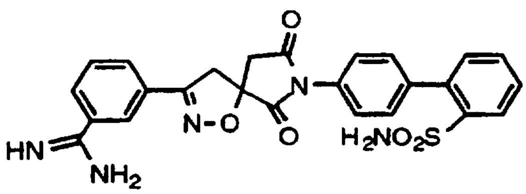 | 504.3 |
| 214 | 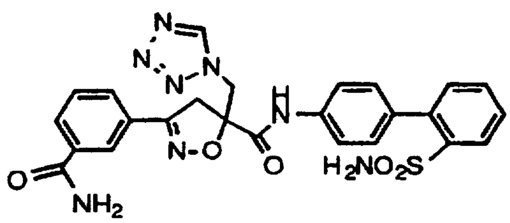 | |
| 215 | 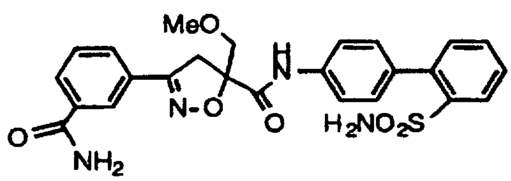 | |
| 216 | 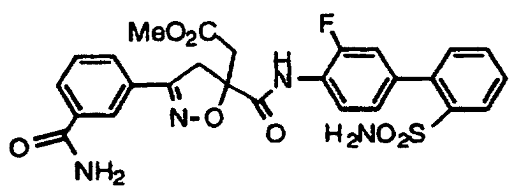 | |
| 217 | 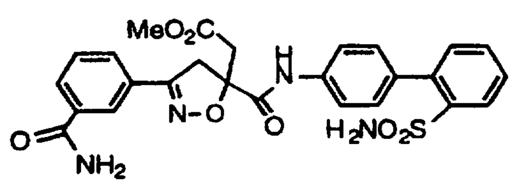 | |
| 218 | 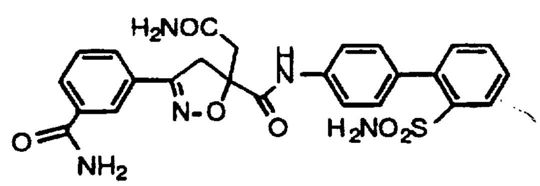 | |

## TABLE 6

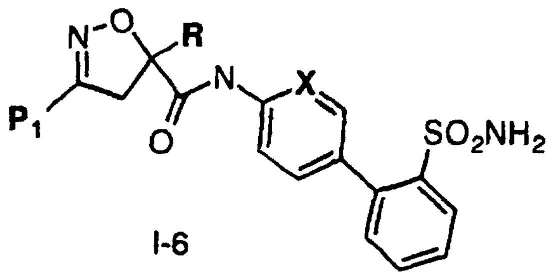

I-6

| EX# | P1 | R | X | MP(⁰C) | MS(M+H)+ |
|---|---|---|---|---|---|
| 219 | 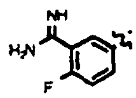 | $-CH_3$ | CH | 140 | 496.3 |
| 220 | 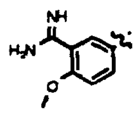 | $-CH_3$ | CH | 240 | 508.3 (69%) |
| 221 | 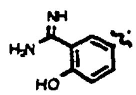 | $-CH_3$ | CH | 235 | 494.3 |
| 222 | 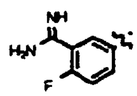 | $CH_2OCH_3$ | N | 81 | 528.4 |
| 223 | 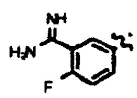 | $CH_2OCH_3$ | CH | 175 | 526.4 |
| 224 | 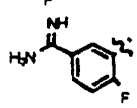 | $CH_2OCH_3$ | CH | 215 | 526.3 |
| 225 | 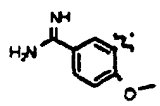 | $-CH_3$ | CH | 245 | 508.4 |
| 226 | 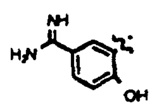 | $-CH_3$ | CH | 238 | 494.2 |
| 227 | 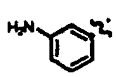 | $-CH_3$ | CH | 207 | 451.4 |

Tables 7-15 identify additional representative compounds of this invention which can be prepared by the methods described above.

The divalent radicals V in the compounds of Tables 7-11 have the following structures

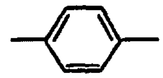

1,4-phenylene

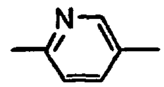

pyridin-2,5-diyl

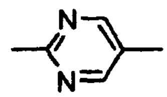

pyrimidin-2,5-diyl

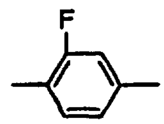

2-fluoro-1,4-phenylene

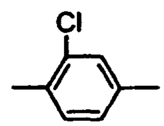

2-chloro-1,4-phenylene

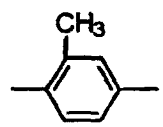

2-methyl-1,4-phenylene

The pyridin-2,5-diyl and pyrimidin-2,5-diyl radicals are bonded to the D moiety at the 5 position. The 2-substituted-1,4-phenylene radicals are bonded to the D moiety at the 4 position.

The compounds of Tables 7-11 have the structures indicated by the formula "a" under each table heading. The corresponding compounds having the structures of formula "b" under each table heading can be obtained by substituting the appropriate starting material, as illustrated in Examples 10 and 11.

**TABLE 7**

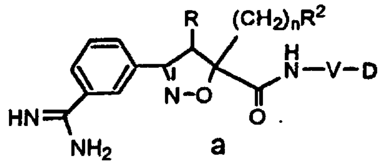

a

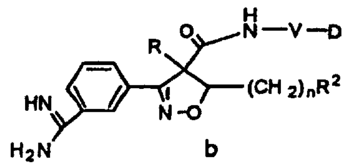

b

| Part | Cpd | R | $(CH_2)_nR^2$ | V | -D |
|---|---|---|---|---|---|
| A1 | 1 | $CH_2OCH_3$ | $CH_2OMe$ | 1-4-phenylene | 2-aminosulfonylphenyl |
| | 2 | $CH_2OCH_3$ | $CH_2OEt$ | 1-4-phenylene | 2-aminosulfonylphenyl |
| | 3 | $CH_2OCH_3$ | $CH_2O$-n-Pr | 1-4-phenylene | 2-aminosulfonylphenyl |
| | 4 | $CH_2OCH_3$ | $CH_2O$-i-Pr | 1-4-phenylene | 2-aminosulfonylphenyl |
| | 5 | $CH_2OCH_3$ | $CH_2O$-n-Bu | 1-4-phenylene | 2-aminosulfonylphenyl |
| | 6 | $CH_2OCH_3$ | $CH_2O$-i-Bu | 1-4-phenylene | 2-aminosulfonylphenyl |
| | 7 | $CH_2OCH_3$ | $CH_2Ph$ | 1-4-phenylene | 2-aminosulfonylphenyl |
| | 8 | $CH_2OCH_3$ | $CH_2$-pyrazol-1-yl | 1-4-phenylene | 2-aminosulfonylphenyl |
| | 9 | $CH_2OCH_3$ | $CH_2$-imidazol-1-yl | 1-4-phenylene | 2-aminosulfonylphenyl |
| | 10 | $CH_2OCH_3$ | $CH_2$-tetrazol-1-yl | 1-4-phenylene | 2-aminosulfonylphenyl |
| | 11 | $CH_2OCH_3$ | $CH_2$-tetrazol-2-yl | 1-4-phenylene | 2-aminosulfonylphenyl |
| | 12 | $CH_2OCH_3$ | $CH_2$-triazol-1-yl | 1-4-phenylene | 2-aminosulfonylphenyl |
| | 13 | $CH_2OCH_3$ | $CH_2SEt$ | 1-4-phenylene | 2-aminosulfonylphenyl |
| | 14 | $CH_2OCH_3$ | $CH_2SO_2Et$ | 1-4-phenylene | 2-aminosulfonylphenyl |
| | 15 | $CH_2OCH_3$ | $CF_3$ | 1-4-phenylene | 2-aminosulfonylphenyl |
| | 16 | $CH_2OCH_3$ | $CH_3$ | 1-4-phenylene | 2-aminosulfonylphenyl |
| | 17 | $CH_2OCH_3$ | H | 1-4-phenylene | 2-aminosulfonylphenyl |
| A2 | 1 | $CH_2OCH_3$ | $CH_2OMe$ | pyridin-2,5-diyl | 2-aminosulfonylphenyl |
| | 2 | $CH_2OCH_3$ | $CH_2OEt$ | pyridin-2,5-diyl | 2-aminosulfonylphenyl |
| | 3 | $CH_2OCH_3$ | $CH_2O$-n-Pr | pyridin-2,5-diyl | 2-aminosulfonylphenyl |

| | | | | | |
|---|---|---|---|---|---|
| | 4 | $CH_2OCH_3$ | $CH_2O$-i-Pr | pyridin-2,5-diyl | 2-aminosulfonylphenyl |
| | 5 | $CH_2OCH_3$ | $CH_2O$-n-Bu | pyridin-2,5-diyl | 2-aminosulfonylphenyl |
| | 6 | $CH_2OCH_3$ | $CH_2O$-i-Bu | pyridin-2,5-diyl | 2-aminosulfonylphenyl |
| | 7 | $CH_2OCH_3$ | $CH_2Ph$ | pyridin-2,5-diyl | 2-aminosulfonylphenyl |
| | 8 | $CH_2OCH_3$ | $CH_2$-pyrazol-1-yl | pyridin-2,5-diyl | 2-aminosulfonylphenyl |
| | 9 | $CH_2OCH_3$ | $CH_2$-imidazol-1-yl | pyridin-2,5-diyl | 2-aminosulfonylphenyl |
| | 10 | $CH_2OCH_3$ | $CH_2$-tetrazol-1-yl | pyridin-2,5-diyl | 2-aminosulfonylphenyl |
| | 11 | $CH_2OCH_3$ | $CH_2$-tetrazol-2-yl | pyridin-2,5-diyl | 2-aminosulfonylphenyl |
| | 12 | $CH_2OCH_3$ | $CH_2$-triazol-1-yl | pyridin-2,5-diyl | 2-aminosulfonylphenyl |
| | 13 | $CH_2OCH_3$ | $CH_2SEt$ | pyridin-2,5-diyl | 2-aminosulfonylphenyl |
| | 14 | $CH_2OCH_3$ | $CH_2SO_2Et$ | pyridin-2,5-diyl | 2-aminosulfonylphenyl |
| | 15 | $CH_2OCH_3$ | $CF_3$ | pyridin-2,5-diyl | 2-aminosulfonylphenyl |
| | 16 | $CH_2OCH_3$ | $CH_3$ | pyridin-2,5-diyl | 2-aminosulfonylphenyl |
| | 17 | $CH_2OCH_3$ | H | pyridin-2,5-diyl | 2-aminosulfonylphenyl |
| A3 | 1 | $CH_2OCH_3$ | $CH_2OMe$ | pyrimidin-2,5-diyl | 2-aminosulfonylphenyl |
| | 2 | $CH_2OCH_3$ | $CH_2OEt$ | pyrimidin-2,5-diyl | 2-aminosulfonylphenyl |
| | 3 | $CH_2OCH_3$ | $CH_2O$-n-Pr | pyrimidin-2,5-diyl | 2-aminosulfonylphenyl |
| | 4 | $CH_2OCH_3$ | $CH_2O$-i-Pr | pyrimidin-2,5-diyl | 2-aminosulfonylphenyl |

| | | | | | |
|---|---|---|---|---|---|
| | 5 | $CH_2OCH_3$ | $CH_2O$-n-Bu | pyrimidin-2,5-diyl | 2-aminosulfonylphenyl |
| | 6 | $CH_2OCH_3$ | $CH_2O$-i-Bu | pyrimidin-2,5-diyl | 2-aminosulfonylphenyl |
| | 7 | $CH_2OCH_3$ | $CH_2Ph$ | pyrimidin-2,5-diyl | 2-aminosulfonylphenyl |
| | 8 | $CH_2OCH_3$ | $CH_2$-pyrazol-1-yl | pyrimidin-2,5-diyl | 2-aminosulfonylphenyl |
| | 9 | $CH_2OCH_3$ | $CH_2$-imidazol-1-yl | pyrimidin-2,5-diyl | 2-aminosulfonylphenyl |
| | 10 | $CH_2OCH_3$ | $CH_2$-tetrazol-1-yl | pyrimidin-2,5-diyl | 2-aminosulfonylphenyl |
| | 11 | $CH_2OCH_3$ | $CH_2$-tetrazol-2-yl | pyrimidin-2,5-diyl | 2-aminosulfonylphenyl |
| | 12 | $CH_2OCH_3$ | $CH_2$-triazol-1-yl | pyrimidin-2,5-diyl | 2-aminosulfonylphenyl |
| | 13 | $CH_2OCH_3$ | $CH_2SEt$ | pyrimidin-2,5-diyl | 2-aminosulfonylphenyl |
| | 14 | $CH_2OCH_3$ | $CH_2SO_2Et$ | pyrimidin-2,5-diyl | 2-aminosulfonylphenyl |
| | 15 | $CH_2OCH_3$ | $CF_3$ | pyrimidin-2,5-diyl | 2-aminosulfonylphenyl |
| | 16 | $CH_2OCH_3$ | $CH_3$ | pyrimidin-2,5-diyl | 2-aminosulfonylphenyl |
| | 17 | $CH_2OCH_3$ | H | pyrimidin-2,5-diyl | 2-aminosulfonylphenyl |
| A4 | 1 | $CH_2OCH_3$ | $CH_2OMe$ | 2-fluoro-1,4-phenylene | 2-aminosulfonylphenyl |
| | 2 | $CH_2OCH_3$ | $CH_2OEt$ | 2-fluoro-1,4-phenylene | 2-aminosulfonylphenyl |
| | 3 | $CH_2OCH_3$ | $CH_2O$-n-Pr | 2-fluoro-1,4-phenylene | 2-aminosulfonylphenyl |
| | 4 | $CH_2OCH_3$ | $CH_2O$-i-Pr | 2-fluoro-1,4-phenylene | 2-aminosulfonylphenyl |
| | 5 | $CH_2OCH_3$ | $CH_2O$-n-Bu | 2-fluoro-1,4-phenylene | 2-aminosulfonylphenyl |

| | | | | | |
|---|---|---|---|---|---|
| | 6 | $CH_2OCH_3$ | $CH_2O$-i-Bu | 2-fluoro-1,4-phenylene | 2-aminosulfonylphenyl |
| | 7 | $CH_2OCH_3$ | $CH_2Ph$ | 2-fluoro-1,4-phenylene | 2-aminosulfonylphenyl |
| | 8 | $CH_2OCH_3$ | $CH_2$-pyrazol-1-yl | 2-fluoro-1,4-phenylene | 2-aminosulfonylphenyl |
| | 9 | $CH_2OCH_3$ | $CH_2$-imidazol-1-yl | 2-fluoro-1,4-phenylene | 2-aminosulfonylphenyl |
| | 10 | $CH_2OCH_3$ | $CH_2$-tetrazol-1-yl | 2-fluoro-1,4-phenylene | 2-aminosulfonylphenyl |
| | 11 | $CH_2OCH_3$ | $CH_2$-tetrazol-2-yl | 2-fluoro-1,4-phenylene | 2-aminosulfonylphenyl |
| | 12 | $CH_2OCH_3$ | $CH_2$-triazol-1-yl | 2-fluoro-1,4-phenylene | 2-aminosulfonylphenyl |
| | 13 | $CH_2OCH_3$ | $CH_2SEt$ | 2-fluoro-1,4-phenylene | 2-aminosulfonylphenyl |
| | 14 | $CH_2OCH_3$ | $CH_2SO_2Et$ | 2-fluoro-1,4-phenylene | 2-aminosulfonylphenyl |
| | 15 | $CH_2OCH_3$ | $CF_3$ | 2-fluoro-1,4-phenylene | 2-aminosulfonylphenyl |
| | 16 | $CH_2OCH_3$ | $CH_3$ | 2-fluoro-1,4-phenylene | 2-aminosulfonylphenyl |
| | 17 | $CH_2OCH_3$ | H | 2-fluoro-1,4-phenylene | 2-aminosulfonylphenyl |
| A5 | 1 | $CH_2OCH_3$ | $CH_2OMe$ | 2-chloro-1,4-phenylene | 2-aminosulfonylphenyl |
| | 2 | $CH_2OCH_3$ | $CH_2OEt$ | 2-chloro-1,4-phenylene | 2-aminosulfonylphenyl |
| | 3 | $CH_2OCH_3$ | $CH_2O$-n-Pr | 2-chloro-1,4-phenylene | 2-aminosulfonylphenyl |
| | 4 | $CH_2OCH_3$ | $CH_2O$-i-Pr | 2-chloro-1,4-phenylene | 2-aminosulfonylphenyl |
| | 5 | $CH_2OCH_3$ | $CH_2O$-n-Bu | 2-chloro-1,4-phenylene | 2-aminosulfonylphenyl |
| | 6 | $CH_2OCH_3$ | $CH_2O$-i-Bu | 2-chloro-1,4-phenylene | 2-aminosulfonylphenyl |

| | | | | | |
|---|---|---|---|---|---|
| | 7 | $CH_2OCH_3$ | $CH_2Ph$ | 2-chloro-1,4-phenylene | 2-aminosulfonylphenyl |
| | 8 | $CH_2OCH_3$ | $CH_2$-pyrazol-1-yl | 2-chloro-1,4-phenylene | 2-aminosulfonylphenyl |
| | 9 | $CH_2OCH_3$ | $CH_2$-imidazol-1-yl | 2-chloro-1,4-phenylenel | 2-aminosulfonylphenyl |
| | 10 | $CH_2OCH_3$ | $CH_2$-tetrazol-1-yl | 2-chloro-1,4-phenylene | 2-aminosulfonylphenyl |
| | 11 | $CH_2OCH_3$ | $CH_2$-tetrazol-2-yl | 2-chloro-1,4-phenylene | 2-aminosulfonylphenyl |
| | 12 | $CH_2OCH_3$ | $CH_2$-triazol-1-yl | 2-chloro-1,4-phenylene | 2-aminosulfonylphenyl |
| | 13 | $CH_2OCH_3$ | $CH_2SEt$ | 2-chloro-1,4-phenylene | 2-aminosulfonylphenyl |
| | 14 | $CH_2OCH_3$ | $CH_2SO_2Et$ | 2-chloro-1,4-phenylene | 2-aminosulfonylphenyl |
| | 15 | $CH_2OCH_3$ | $CF_3$ | 2-chloro-1,4-phenylene | 2-aminosulfonylphenyl |
| | 16 | $CH_2OCH_3$ | $CH_3$ | 2-chloro-1,4-phenylene | 2-aminosulfonylphenyl |
| | 17 | $CH_2OCH_3$ | H | 2-chloro-1,4-phenylene | 2-aminosulfonylphenyl |
| B1 | 1 | $CH_3$ | $CH_2OMe$ | 1,4-phenylene | 2-aminosulfonylphenyl |
| | 2 | $CH_3$ | $CH_2OEt$ | 1,4-phenylene | 2-aminosulfonylphenyl |
| | 3 | $CH_3$ | $CH_2O$-n-Pr | 1,4-phenylene | 2-aminosulfonylphenyl |
| | 4 | $CH_3$ | $CH_2O$-i-Pr | 1,4-phenylene | 2-aminosulfonylphenyl |
| | 5 | $CH_3$ | $CH_2O$-n-Bu | 1,4-phenylene | 2-aminosulfonylphenyl |
| | 6 | $CH_3$ | $CH_2O$-i-Bu | 1,4-phenylene | 2-aminosulfonylphenyl |
| | 7 | $CH_3$ | $CH_2Ph$ | 1,4-phenylene | 2-aminosulfonylphenyl |
| | 8 | $CH_3$ | $CH_2$-pyrazol-1-yl | 1,4-phenylene | 2-aminosulfonylphenyl |
| | 9 | $CH_3$ | $CH_2$-imidazol-1-yl | 1,4-phenylene | 2-aminosulfonylphenyl |
| | 10 | $CH_3$ | $CH_2$-tetrazol-1-yl | 1,4-phenylene | 2-aminosulfonylphenyl |

| | | | | | |
|---|---|---|---|---|---|
| | 11 | $CH_3$ | $CH_2$-tetrazol-2-yl | 1,4-phenylene | 2-aminosulfonylphenyl |
| | 12 | $CH_3$ | $CH_2$-triazol-1-yl | 1,4-phenylene | 2-aminosulfonylphenyl |
| | 13 | $CH_3$ | $CH_2SEt$ | 1,4-phenylene | 2-aminosulfonylphenyl |
| | 14 | $CH_3$ | $CH_2SO_2Et$ | 1,4-phenylene | 2-aminosulfonylphenyl |
| | 15 | $CH_3$ | $CF_3$ | 1,4-phenylene | 2-aminosulfonylphenyl |
| | 16 | $CH_3$ | $CH_3$ | 1,4-phenylene | 2-aminosulfonylphenyl |
| | 17 | $CH_3$ | H | 1,4-phenylene | 2-aminosulfonylphenyl |
| B2 | 1 | $CH_3$ | $CH_2OMe$ | pyridin-2,5-diyl | 2-aminosulfonylphenyl |
| | 2 | $CH_3$ | $CH_2OEt$ | pyridin-2,5-diyl | 2-aminosulfonylphenyl |
| | 3 | $CH_3$ | $CH_2O$-n-Pr | pyridin-2,5-diyl | 2-aminosulfonylphenyl |
| | 4 | $CH_3$ | $CH_2O$-i-Pr | pyridin-2,5-diyl | 2-aminosulfonylphenyl |
| | 5 | $CH_3$ | $CH_2O$-n-Bu | pyridin-2,5-diyl | 2-aminosulfonylphenyl |
| | 6 | $CH_3$ | $CH_2O$-i-Bu | pyridin-2,5-diyl | 2-aminosulfonylphenyl |
| | 7 | $CH_3$ | $CH_2Ph$ | pyridin-2,5-diyl | 2-aminosulfonylphenyl |
| | 8 | $CH_3$ | $CH_2$-pyrazol-1-yl | pyridin-2,5-diyl | 2-aminosulfonylphenyl |
| | 9 | $CH_3$ | $CH_2$-imidazol-1-yl | pyridin-2,5-diyl | 2-aminosulfonylphenyl |
| | 10 | $CH_3$ | $CH_2$-tetrazol-1-yl | pyridin-2,5-diyl | 2-aminosulfonylphenyl |
| | 11 | $CH_3$ | $CH_2$-tetrazol-2-yl | pyridin-2,5-diyl | 2-aminosulfonylphenyl |
| | 12 | $CH_3$ | $CH_2$-triazol-1-yl | pyridin-2,5-diyl | 2-aminosulfonylphenyl |
| | 13 | $CH_3$ | $CH_2SEt$ | pyridin-2,5-diyl | 2-aminosulfonylphenyl |

| | | | | | |
|---|---|---|---|---|---|
| | 14 | $CH_3$ | $CH_2SO_2Et$ | pyridin-2,5-diyl | 2-aminosulfonylphenyl |
| | 15 | $CH_3$ | $CF_3$ | pyridin-2,5-diyl | 2-aminosulfonylphenyl |
| | 16 | $CH_3$ | $CH_3$ | pyridin-2,5-diyl | 2-aminosulfonylphenyl |
| | 17 | $CH_3$ | H | pyridin-2,5-diyl | 2-aminosulfonylphenyl |
| B3 | 1 | $CH_3$ | $CH_2OMe$ | pyrimidin-2,5-diyl | 2-aminosulfonylphenyl |
| | 2 | $CH_3$ | $CH_2OEt$ | pyrimidin-2,5-diyl | 2-aminosulfonylphenyl |
| | 3 | $CH_3$ | $CH_2O$-n-Pr | pyrimidin-2,5-diyl | 2-aminosulfonylphenyl |
| | 4 | $CH_3$ | $CH_2O$-i-Pr | pyrimidin-2,5-diyl | 2-aminosulfonylphenyl |
| | 5 | $CH_3$ | $CH_2O$-n-Bu | pyrimidin-2,5-diyl | 2-aminosulfonylphenyl |
| | 6 | $CH_3$ | $CH_2O$-i-Bu | pyrimidin-2,5-diyl | 2-aminosulfonylphenyl |
| | 7 | $CH_3$ | $CH_2Ph$ | pyrimidin-2,5-diyl | 2-aminosulfonylphenyl |
| | 8 | $CH_3$ | $CH_2$-pyrazol-1-yl | pyrimidin-2,5-diyl | 2-aminosulfonylphenyl |
| | 9 | $CH_3$ | $CH_2$-imidazol-1-yl | pyrimidin-2,5-diyl | 2-aminosulfonylphenyl |
| | 10 | $CH_3$ | $CH_2$-tetrazol-1-yl | pyrimidin-2,5-diyl | 2-aminosulfonylphenyl |
| | 11 | $CH_3$ | $CH_2$-tetrazol-2-yl | pyrimidin-2,5-diyl | 2-aminosulfonylphenyl |
| | 12 | $CH_3$ | $CH_2$-triazol-1-yl | pyrimidin-2,5-diyl | 2-aminosulfonylphenyl |
| | 13 | $CH_3$ | $CH_2SEt$ | pyrimidin-2,5-diyl | 2-aminosulfonylphenyl |
| | 14 | $CH_3$ | $CH_2SO_2Et$ | pyrimidin-2,5-diyl | 2-aminosulfonylphenyl |

| | | | | | |
|---|---|---|---|---|---|
| | 15 | $CH_3$ | $CF_3$ | pyrimidin-2,5-diyl | 2-aminosulfonylphenyl |
| | 16 | $CH_3$ | $CH_3$ | pyrimidin-2,5-diyl | 2-aminosulfonylphenyl |
| | 17 | $CH_3$ | H | pyrimidin-2,5-diyl | 2-aminosulfonylphenyl |
| B4 | 1 | $CH_3$ | $CH_2OMe$ | 2-fluoro-1,4-phenylene | 2-aminosulfonylphenyl |
| | 2 | $CH_3$ | $CH_2OEt$ | 2-fluoro-1,4-phenylene | 2-aminosulfonylphenyl |
| | 3 | $CH_3$ | $CH_2O$-n-Pr | 2-fluoro-1,4-phenylene | 2-aminosulfonylphenyl |
| | 4 | $CH_3$ | $CH_2O$-i-Pr | 2-fluoro-1,4-phenylene | 2-aminosulfonylphenyl |
| | 5 | $CH_3$ | $CH_2O$-n-Bu | 2-fluoro-1,4-phenylene | 2-aminosulfonylphenyl |
| | 6 | $CH_3$ | $CH_2O$-i-Bu | 2-fluoro-1,4-phenylene | 2-aminosulfonylphenyl |
| | 7 | $CH_3$ | $CH_2Ph$ | 2-fluoro-1,4-phenylene | 2-aminosulfonylphenyl |
| | 8 | $CH_3$ | $CH_2$-pyrazol-1-yl | 2-fluoro-1,4-phenylene | 2-aminosulfonylphenyl |
| | 9 | $CH_3$ | $CH_2$-imidazol-1-yl | 2-fluoro-1,4-phenylene | 2-aminosulfonylphenyl |
| | 10 | $CH_3$ | $CH_2$-tetrazol-1-yl | 2-fluoro-1,4-phenylene | 2-aminosulfonylphenyl |
| | 11 | $CH_3$ | $CH_2$-tetrazol-2-yl | 2-fluoro-1,4-phenylene | 2-aminosulfonylphenyl |
| | 12 | $CH_3$ | $CH_2$-triazol-1-yl | 2-fluoro-1,4-phenylene | 2-aminosulfonylphenyl |
| | 13 | $CH_3$ | $CH_2SEt$ | 2-fluoro-1,4-phenylene | 2-aminosulfonylphenyl |
| | 14 | $CH_3$ | $CH_2SO_2Et$ | 2-fluoro-1,4-phenylene | 2-aminosulfonylphenyl |
| | 15 | $CH_3$ | $CF_3$ | 2-fluoro-1,4-phenylene | 2-aminosulfonylphenyl |

| | | | | | |
|---|---|---|---|---|---|
| | 16 | $CH_3$ | $CH_3$ | 2-fluoro-1,4-phenylene | 2-aminosulfonylphenyl |
| | 17 | $CH_3$ | H | 2-fluoro-1,4-phenylene | 2-aminosulfonylphenyl |
| B5 | 1 | $CH_3$ | $CH_2OMe$ | 2-chloro-1,4-phenylene | 2-aminosulfonylphenyl |
| | 2 | $CH_3$ | $CH_2OEt$ | 2-chloro-1,4-phenylene | 2-aminosulfonylphenyl |
| | 3 | $CH_3$ | $CH_2O$-n-Pr | 2-chloro-1,4-phenylene | 2-aminosulfonylphenyl |
| | 4 | $CH_3$ | $CH_2O$-i-Pr | 2-chloro-1,4-phenylene | 2-aminosulfonylphenyl |
| | 5 | $CH_3$ | $CH_2O$-n-Bu | 2-chloro-1,4-phenylene | 2-aminosulfonylphenyl |
| | 6 | $CH_3$ | $CH_2O$-i-Bu | 2-chloro-1,4-phenylene | 2-aminosulfonylphenyl |
| | 7 | $CH_3$ | $CH_2Ph$ | 2-chloro-1,4-phenylene | 2-aminosulfonylphenyl |
| | 8 | $CH_3$ | $CH_2$-pyrazol-1-yl | 2-chloro-1,4-phenylene | 2-aminosulfonylphenyl |
| | 9 | $CH_3$ | $CH_2$-imidazol-1-yl | 2-chloro-1,4-phenylene | 2-aminosulfonylphenyl |
| | 10 | $CH_3$ | $CH_2$-tetrazol-1-yl | 2-chloro-1,4-phenylene | 2-aminosulfonylphenyl |
| | 11 | $CH_3$ | $CH_2$-tetrazol-2-yl | 2-chloro-1,4-phenylene | 2-aminosulfonylphenyl |
| | 12 | $CH_3$ | $CH_2$-triazol-1-yl | 2-chloro-1,4-phenylene | 2-aminosulfonylphenyl |
| | 13 | $CH_3$ | $CH_2SEt$ | 2-chloro-1,4-phenylene | 2-aminosulfonylphenyl |
| | 14 | $CH_3$ | $CH_2SO_2Et$ | 2-chloro-1,4-phenylene | 2-aminosulfonylphenyl |
| | 15 | $CH_3$ | $CF_3$ | 2-chloro-1,4-phenylene | 2-aminosulfonylphenyl |
| | 16 | $CH_3$ | $CH_3$ | 2-chloro-1,4-phenylene | 2-aminosulfonylphenyl |

| | | | | | |
|---|---|---|---|---|---|
| | 17 | $CH_3$ | H | 2-chloro-1,4-phenylene | 2-aminosulfonylphenyl |
| C1 | 1 | H | $CH_2OMe$ | 1,4-phenylene | 2-aminosulfonylphenyl |
| | 2 | H | $CH_2OEt$ | 1,4-phenylene | 2-aminosulfonylphenyl |
| | 3 | H | $CH_2O$-n-Pr | 1,4-phenylene | 2-aminosulfonylphenyl |
| | 4 | H | $CH_2O$-i-Pr | 1,4-phenylene | 2-aminosulfonylphenyl |
| | 5 | H | $CH_2O$-n-Bu | 1,4-phenylene | 2-aminosulfonylphenyl |
| | 6 | H | $CH_2O$-i-Bu | 1,4-phenylene | 2-aminosulfonylphenyl |
| | 7 | H | $CH_2Ph$ | 1,4-phenylene | 2-aminosulfonylphenyl |
| | 8 | H | $CH_2$-pyrazol-1-yl | 1,4-phenylene | 2-aminosulfonylphenyl |
| | 9 | H | $CH_2$-imidazol-1-yl | 1,4-phenylene | 2-aminosulfonylphenyl |
| | 10 | H | $CH_2$-tetrazol-1-yl | 1,4-phenylene | 2-aminosulfonylphenyl |
| | 11 | H | $CH_2$-tetrazol-2-yl | 1,4-phenylene | 2-aminosulfonylphenyl |
| | 12 | H | $CH_2$-triazol-1-yl | 1,4-phenylene | 2-aminosulfonylphenyl |
| | 13 | H | $CH_2SEt$ | 1,4-phenylene | 2-aminosulfonylphenyl |
| | 14 | H | $CH_2SO_2Et$ | 1,4-phenylene | 2-aminosulfonylphenyl |
| | 15 | H | $CF_3$ | 1,4-phenylene | 2-aminosulfonylphenyl |
| | 16 | H | $CH_3$ | 1,4-phenylene | 2-aminosulfonylphenyl |
| | 17 | H | H | 1,4-phenylene | 2-aminosulfonylphenyl |
| C2 | 1 | H | $CH_2OMe$ | pyridin-2,5-diyl | 2-aminosulfonylphenyl |
| | 2 | H | $CH_2OEt$ | pyridin-2,5-diyl | 2-aminosulfonylphenyl |
| | 3 | H | $CH_2O$-n-Pr | pyridin-2,5-diyl | 2-aminosulfonylphenyl |
| | 4 | H | $CH_2O$-i-Pr | pyridin-2,5-diyl | 2-aminosulfonylphenyl |
| | 5 | H | $CH_2O$-n-Bu | pyridin-2,5-diyl | 2-aminosulfonylphenyl |
| | 6 | H | $CH_2O$-i-Bu | pyridin-2,5-diyl | 2-aminosulfonylphenyl |

| | | | | | |
|---|---|---|---|---|---|
| | 7 | H | $CH_2Ph$ | pyridin-2,5-diyl | 2-aminosulfonylphenyl |
| | 8 | H | $CH_2$-pyrazol-1-yl | pyridin-2,5-diyl | 2-aminosulfonylphenyl |
| | 9 | H | $CH_2$-imidazol-1-yl | pyridin-2,5-diyl | 2-aminosulfonylphenyl |
| | 10 | H | $CH_2$-tetrazol-1-yl | pyridin-2,5-diyl | 2-aminosulfonylphenyl |
| | 11 | H | $CH_2$-tetrazol-2-yl | pyridin-2,5-diyl | 2-aminosulfonylphenyl |
| | 12 | H | $CH_2$-triazol-1-yl | pyridin-2,5-diyl | 2-aminosulfonylphenyl |
| | 13 | H | $CH_2SEt$ | pyridin-2,5-diyl | 2-aminosulfonylphenyl |
| | 14 | H | $CH_2SO_2Et$ | pyridin-2,5-diyl | 2-aminosulfonylphenyl |
| | 15 | H | $CF_3$ | pyridin-2,5-diyl | 2-aminosulfonylphenyl |
| | 16 | H | $CH_3$ | pyridin-2,5-diyl | 2-aminosulfonylphenyl |
| | 17 | H | H | pyridin-2,5-diyl | 2-aminosulfonylphenyl |
| C3 | 1 | H | $CH_2OMe$ | pyrimidin-2,5-diyl | 2-aminosulfonylphenyl |
| | 2 | H | $CH_2OEt$ | pyrimidin-2,5-diyl | 2-aminosulfonylphenyl |
| | 3 | H | $CH_2O$-n-Pr | pyrimidin-2,5-diyl | 2-aminosulfonylphenyl |
| | 4 | H | $CH_2O$-i-Pr | pyrimidin-2,5-diyl | 2-aminosulfonylphenyl |
| | 5 | H | $CH_2O$-n-Bu | pyrimidin-2,5-diyl | 2-aminosulfonylphenyl |
| | 6 | H | $CH_2O$-i-Bu | pyrimidin-2,5-diyl | 2-aminosulfonylphenyl |
| | 7 | H | $CH_2Ph$ | pyrimidin-2,5-diyl | 2-aminosulfonylphenyl |

| | | | | | |
|---|---|---|---|---|---|
| | 8 | H | $CH_2$-pyrazol-1-yl | pyrimidin-2,5-diyl | 2-aminosulfonylphenyl |
| | 9 | H | $CH_2$-imidazol-1-yl | pyrimidin-2,5-diyl | 2-aminosulfonylphenyl |
| | 10 | H | $CH_2$-tetrazol-1-yl | pyrimidin-2,5-diyl | 2-aminosulfonylphenyl |
| | 11 | H | $CH_2$-tetrazol-2-yl | pyrimidin-2,5-diyl | 2-aminosulfonylphenyl |
| | 12 | H | $CH_2$-triazol-1-yl | pyrimidin-2,5-diyl | 2-aminosulfonylphenyl |
| | 13 | H | $CH_2SEt$ | pyrimidin-2,5-diyl | 2-aminosulfonylphenyl |
| | 14 | H | $CH_2SO_2Et$ | pyrimidin-2,5-diyl | 2-aminosulfonylphenyl |
| | 15 | H | $CF_3$ | pyrimidin-2,5-diyl | 2-aminosulfonylphenyl |
| | 16 | H | $CH_3$ | pyrimidin-2,5-diyl | 2-aminosulfonylphenyl |
| | 17 | H | H | pyrimidin-2,5-diyl | 2-aminosulfonylphenyl |
| C4 | 1 | H | $CH_2OMe$ | 2-fluoro-1,4-phenylene | 2-aminosulfonylphenyl |
| | 2 | H | $CH_2OEt$ | 2-fluoro-1,4-phenylene | 2-aminosulfonylphenyl |
| | 3 | H | $CH_2O$-n-Pr | 2-fluoro-1,4-phenylene | 2-aminosulfonylphenyl |
| | 4 | H | $CH_2O$-i-Pr | 2-fluoro-1,4-phenylene | 2-aminosulfonylphenyl |
| | 5 | H | $CH_2O$-n-Bu | 2-fluoro-1,4-phenylene | 2-aminosulfonylphenyl |
| | 6 | H | $CH_2O$-i-Bu | 2-fluoro-1,4-phenylene | 2-aminosulfonylphenyl |
| | 7 | H | $CH_2Ph$ | 2-fluoro-1,4-phenylene | 2-aminosulfonylphenyl |
| | 8 | H | $CH_2$-pyrazol-1-yl | 2-fluoro-1,4-phenylene | 2-aminosulfonylphenyl |

| | | | | | |
|---|---|---|---|---|---|
| | 9 | H | $CH_2$-imidazol-1-yl | 2-fluoro-1,4-phenylene | 2-aminosulfonylphenyl |
| | 10 | H | $CH_2$-tetrazol-1-yl | 2-fluoro-1,4-phenylene | 2-aminosulfonylphenyl |
| | 11 | H | $CH_2$-tetrazol-2-yl | 2-fluoro-1,4-phenylene | 2-aminosulfonylphenyl |
| | 12 | H | $CH_2$-triazol-1-yl | 2-fluoro-1,4-phenylene | 2-aminosulfonylphenyl |
| | 13 | H | $CH_2SEt$ | 2-fluoro-1,4-phenylene | 2-aminosulfonylphenyl |
| | 14 | H | $CH_2SO_2Et$ | 2-fluoro-1,4-phenylene | 2-aminosulfonylphenyl |
| | 15 | H | $CF_3$ | 2-fluoro-1,4-phenylene | 2-aminosulfonylphenyl |
| | 16 | H | $CH_3$ | 2-fluoro-1,4-phenylene | 2-aminosulfonylphenyl |
| | 17 | H | H | 2-fluoro-1,4-phenylene | 2-aminosulfonylphenyl |
| C5 | 1 | H | $CH_2OMe$ | 2-chloro-1,4-phenylene | 2-aminosulfonylphenyl |
| | 2 | H | $CH_2OEt$ | 2-chloro-1,4-phenylene | 2-aminosulfonylphenyl |
| | 3 | H | $CH_2O$-n-Pr | 2-chloro-1,4-phenylene | 2-aminosulfonylphenyl |
| | 4 | H | $CH_2O$-i-Pr | 2-chloro-1,4-phenylene | 2-aminosulfonylphenyl |
| | 5 | H | $CH_2O$-n-Bu | 2-chloro-1,4-phenylene | 2-aminosulfonylphenyl |
| | 6 | H | $CH_2O$-i-Bu | 2-chloro-1,4-phenylene | 2-aminosulfonylphenyl |
| | 7 | H | $CH_2Ph$ | 2-chloro-1,4-phenylene | 2-aminosulfonylphenyl |
| | 8 | H | $CH_2$-pyrazol-1-yl | 2-chloro-1,4-phenylene | 2-aminosulfonylphenyl |
| | 9 | H | $CH_2$-imidazol-1-yl | 2-chloro-1,4-phenylene | 2-aminosulfonylphenyl |

| | | | | | |
|---|---|---|---|---|---|
| | 10 | H | $CH_2$-tetrazol-1-yl | 2-chloro-1,4-phenylene | 2-aminosulfonylphenyl |
| | 11 | H | $CH_2$-tetrazol-2-yl | 2-chloro-1,4-phenylene | 2-aminosulfonylphenyl |
| | 12 | H | $CH_2$-triazol-1-yl | 2-chloro-1,4-phenylene | 2-aminosulfonylphenyl |
| | 13 | H | $CH_2SEt$ | 2-chloro-1,4-phenylene | 2-aminosulfonylphenyl |
| | 14 | H | $CH_2SO_2Et$ | 2-chloro-1,4-phenylene | 2-aminosulfonylphenyl |
| | 15 | H | $CF_3$ | 2-chloro-1,4-phenylene | 2-aminosulfonylphenyl |
| | 16 | H | $CH_3$ | 2-chloro-1,4-phenylene | 2-aminosulfonylphenyl |
| | 17 | H | H | 2-chloro-1,4-phenylene | 2-aminosulfonylphenyl |
| C6 | 1 | H | $CH_2OMe$ | 2-methyl-1,4-phenylene | 2-aminosulfonylphenyl |
| | 2 | H | $CH_2OEt$ | 2-methyl-1,4-phenylene | 2-aminosulfonylphenyl |
| | 3 | H | $CH_2O$-n-Pr | 2-methyl-1,4-phenylene | 2-aminosulfonylphenyl |
| | 4 | H | $CH_2O$-i-Pr | 2-methyl-1,4-phenylene | 2-aminosulfonylphenyl |
| | 5 | H | $CH_2O$-n-Bu | 2-methyl-1,4-phenylene | 2-aminosulfonylphenyl |
| | 6 | H | $CH_2O$-i-Bu | 2-methyl-1,4-phenylene | 2-aminosulfonylphenyl |
| | 7 | H | $CH_2Ph$ | 2-methyl-1,4-phenylene | 2-aminosulfonylphenyl |
| | 8 | H | $CH_2$-pyrazol-1-yl | 2-methyl-1,4-phenylene | 2-aminosulfonylphenyl |
| | 9 | H | $CH_2$-imidazol-1-yl | 2-methyl-1,4-phenylene | 2-aminosulfonylphenyl |
| | 10 | H | $CH_2$-tetrazol-1-yl | 2-methyl-1,4-phenylene | 2-aminosulfonylphenyl |

| | | | | | |
|---|---|---|---|---|---|
| | 11 | H | $CH_2$-tetrazol-2-yl | 2-methyl-1,4-phenylene | 2-aminosulfonylphenyl |
| | 12 | H | $CH_2$-triazol-1-yl | 2-methyl-1,4-phenylene | 2-aminosulfonylphenyl |
| | 13 | H | $CH_2SEt$ | 2-methyl-1,4-phenylene | 2-aminosulfonylphenyl |
| | 14 | H | $CH_2SO_2Et$ | 2-methyl-1,4-phenylene | 2-aminosulfonylphenyl |
| | 15 | H | $CF_3$ | 2-methyl-1,4-phenylene | 2-aminosulfonylphenyl |
| | 16 | H | $CH_3$ | 2-methyl-1,4-phenylene | 2-aminosulfonylphenyl |
| | 17 | H | H | 2-methyl-1,4-phenylene | 2-aminosulfonylphenyl |
| D1 | 1 | H | $CH_2OMe$ | 1,4-phenylene | 2-trifluoromethylphenyl |
| | 2 | H | $CH_2OEt$ | 1,4-phenylene | 2-trifluoromethylphenyl |
| | 3 | H | $CH_2O$-n-Pr | 1,4-phenylene | 2-trifluoromethylphenyl |
| | 4 | H | $CH_2O$-i-Pr | 1,4-phenylene | 2-trifluoromethylphenyl |
| | 5 | H | $CH_2O$-n-Bu | 1,4-phenylene | 2-trifluoromethylphenyl |
| | 6 | H | $CH_2O$-i-Bu | 1,4-phenylene | 2-trifluoromethylphenyl |
| | 7 | H | $CH_2Ph$ | 1,4-phenylene | 2-trifluoromethylphenyl |
| | 8 | H | $CH_2$-pyrazol-1-yl | 1,4-phenylene | 2-trifluoromethylphenyl |
| | 9 | H | $CH_2$-imidazol-1-yl | 1,4-phenylene | 2-trifluoromethylphenyl |
| | 10 | H | $CH_2$-tetrazol-1-yl | 1,4-phenylene | 2-trifluoromethylphenyl |
| | 11 | H | $CH_2$-tetrazol-2-yl | 1,4-phenylene | 2-trifluoromethylphenyl |

| | | | | | |
|---|---|---|---|---|---|
| | 12 | H | $CH_2$-triazol-1-yl | 1,4-phenylene | 2-trifluoromethylphenyl |
| | 13 | H | $CH_2SEt$ | 1,4-phenylene | 2-trifluoromethylphenyl |
| | 14 | H | $CH_2SO_2Et$ | 1,4-phenylene | 2-trifluoromethylphenyl |
| | 15 | H | $CF_3$ | 1,4-phenylene | 2-trifluoromethylphenyl |
| | 16 | H | $CH_3$ | 1,4-phenylene | 2-trifluoromethylphenyl |
| | 17 | H | H | 1,4-phenylene | 2-trifluoromethylphenyl |
| D2 | 1 | H | $CH_2OMe$ | pyridin-2,5-diyl | 2-trifluoromethylphenyl |
| | 2 | H | $CH_2OEt$ | pyridin-2,5-diyl | 2-trifluoromethylphenyl |
| | 3 | H | $CH_2O$-n-Pr | pyridin-2,5-diyl | 2-trifluoromethylphenyl |
| | 4 | H | $CH_2O$-i-Pr | pyridin-2,5-diyl | 2-trifluoromethylphenyl |
| | 5 | H | $CH_2O$-n-Bu | pyridin-2,5-diyl | 2-trifluoromethylphenyl |
| | 6 | H | $CH_2O$-i-Bu | pyridin-2,5-diyl | 2-trifluoromethylphenyl |
| | 7 | H | $CH_2Ph$ | pyridin-2,5-diyl | 2-trifluoromethylphenyl |
| | 8 | H | $CH_2$-pyrazol-1-yl | pyridin-2,5-diyl | 2-trifluoromethylphenyl |
| | 9 | H | $CH_2$-imidazol-1-yl | pyridin-2,5-diyl | 2-trifluoromethylphenyl |
| | 10 | H | $CH_2$-tetrazol-1-yl | pyridin-2,5-diyl | 2-trifluoromethylphenyl |
| | 11 | H | $CH_2$-tetrazol-2-yl | pyridin-2,5-diyl | 2-trifluoromethylphenyl |
| | 12 | H | $CH_2$-triazol-1-yl | pyridin-2,5-diyl | 2-trifluoromethylphenyl |

| | | | | | |
|---|---|---|---|---|---|
| | 13 | H | $CH_2SEt$ | pyridin-2,5-diyl | 2-trifluoromethylphenyl |
| | 14 | H | $CH_2SO_2Et$ | pyridin-2,5-diyl | 2-trifluoromethylphenyl |
| | 15 | H | $CF_3$ | pyridin-2,5-diyl | 2-trifluoromethylphenyl |
| | 16 | H | $CH_3$ | pyridin-2,5-diyl | 2-trifluoromethylphenyl |
| | 17 | H | H | pyridin-2,5-diyl | 2-trifluoromethylphenyl |
| D3 | 1 | H | $CH_2OMe$ | pyrimidin-2,5-diyl | 2-trifluoromethylphenyl |
| | 2 | H | $CH_2OEt$ | pyrimidin-2,5-diyl | 2-trifluoromethylphenyl |
| | 3 | H | $CH_2O$-n-Pr | pyrimidin-2,5-diyl | 2-trifluoromethylphenyl |
| | 4 | H | $CH_2O$-i-Pr | pyrimidin-2,5-diyl | 2-trifluoromethylphenyl |
| | 5 | H | $CH_2O$-n-Bu | pyrimidin-2,5-diyl | 2-trifluoromethylphenyl |
| | 6 | H | $CH_2O$-i-Bu | pyrimidin-2,5-diyl | 2-trifluoromethylphenyl |
| | 7 | H | $CH_2Ph$ | pyrimidin-2,5-diyl | 2-trifluoromethylphenyl |
| | 8 | H | $CH_2$-pyrazol-1-yl | pyrimidin-2,5-diyl | 2-trifluoromethylphenyl |
| | 9 | H | $CH_2$-imidazol-1-yl | pyrimidin-2,5-diyl | 2-trifluoromethylphenyl |
| | 10 | H | $CH_2$-tetrazol-1-yl | pyrimidin-2,5-diyl | 2-trifluoromethylphenyl |
| | 11 | H | $CH_2$-tetrazol-2-yl | pyrimidin-2,5-diyl | 2-trifluoromethylphenyl |
| | 12 | H | $CH_2$-triazol-1-yl | pyrimidin-2,5-diyl | 2-trifluoromethylphenyl |
| | 13 | H | $CH_2SEt$ | pyrimidin-2,5-diyl | 2-trifluoromethylphenyl |

| | | | | | |
|---|---|---|---|---|---|
| | 14 | H | $CH_2SO_2Et$ | pyrimidin-2,5-diyl | 2-trifluoromethylphenyl |
| | 15 | H | $CF_3$ | pyrimidin-2,5-diyl | 2-trifluoromethylphenyl |
| | 16 | H | $CH_3$ | pyrimidin-2,5-diyl | 2-trifluoromethylphenyl |
| | 17 | H | H | pyrimidin-2,5-diyl | 2-trifluoromethylphenyl |
| D4 | 1 | H | $CH_2OMe$ | 2-fluoro-1,4-phenylene | 2-trifluoromethylphenyl |
| | 2 | H | $CH_2OEt$ | 2-fluoro-1,4-phenylene | 2-trifluoromethylphenyl |
| | 3 | H | $CH_2O$-n-Pr | 2-fluoro-1,4-phenylene | 2-trifluoromethylphenyl |
| | 4 | H | $CH_2O$-i-Pr | 2-fluoro-1,4-phenylene | 2-trifluoromethylphenyl |
| | 5 | H | $CH_2O$-n-Bu | 2-fluoro-1,4-phenylene | 2-trifluoromethylphenyl |
| | 6 | H | $CH_2O$-i-Bu | 2-fluoro-1,4-phenylene | 2-trifluoromethylphenyl |
| | 7 | H | $CH_2Ph$ | 2-fluoro-1,4-phenylene | 2-trifluoromethylphenyl |
| | 8 | H | $CH_2$-pyrazol-1-yl | 2-fluoro-1,4-phenylene | 2-trifluoromethylphenyl |
| | 9 | H | $CH_2$-imidazol-1-yl | 2-fluoro-1,4-phenylene | 2-trifluoromethylphenyl |
| | 10 | H | $CH_2$-tetrazol-1-yl | 2-fluoro-1,4-phenylene | 2-trifluoromethylphenyl |
| | 11 | H | $CH_2$-tetrazol-2-yl | 2-fluoro-1,4-phenylene | 2-trifluoromethylphenyl |
| | 12 | H | $CH_2$-triazol-1-yl | 2-fluoro-1,4-phenylene | 2-trifluoromethylphenyl |
| | 13 | H | $CH_2SEt$ | 2-fluoro-1,4-phenylene | 2-trifluoromethylphenyl |
| | 14 | H | $CH_2SO_2Et$ | 2-fluoro-1,4-phenylene | 2-trifluoromethylphenyl |

| | | | | | |
|---|---|---|---|---|---|
| | 15 | H | $CF_3$ | 2-fluoro-1,4-phenylene | 2-trifluoromethylphenyl |
| | 16 | H | $CH_3$ | 2-fluoro-1,4-phenylene | 2-trifluoromethylphenyl |
| | 17 | H | H | 2-fluoro-1,4-phenylene | 2-trifluoromethylphenyl |
| D5 | 1 | H | $CH_2OMe$ | 2-chloro-1,4-phenylene | 2-trifluoromethylphenyl |
| | 2 | H | $CH_2OEt$ | 2-chloro-1,4-phenylene | 2-trifluoromethylphenyl |
| | 3 | H | $CH_2O$-n-Pr | 2-chloro-1,4-phenylene | 2-trifluoromethylphenyl |
| | 4 | H | $CH_2O$-i-Pr | 2-chloro-1,4-phenylene | 2-trifluoromethylphenyl |
| | 5 | H | $CH_2O$-n-Bu | 2-chloro-1,4-phenylene | 2-trifluoromethylphenyl |
| | 6 | H | $CH_2O$-i-Bu | 2-chloro-1,4-phenylene | 2-trifluoromethylphenyl |
| | 7 | H | $CH_2Ph$ | 2-chloro-1,4-phenylene | 2-trifluoromethylphenyl |
| | 8 | H | $CH_2$-pyrazol-1-yl | 2-chloro-1,4-phenylene | 2-trifluoromethylphenyl |
| | 9 | H | $CH_2$-imidazol-1-yl | 2-chloro-1,4-phenylene | 2-trifluoromethylphenyl |
| | 10 | H | $CH_2$-tetrazol-1-yl | 2-chloro-1,4-phenylene | 2-trifluoromethylphenyl |
| | 11 | H | $CH_2$-tetrazol-2-yl | 2-chloro-1,4-phenylene | 2-trifluoromethylphenyl |
| | 12 | H | $CH_2$-triazol-1-yl | 2-chloro-1,4-phenylene | 2-trifluoromethylphenyl |
| | 13 | H | $CH_2SEt$ | 2-chloro-1,4-phenylene | 2-trifluoromethylphenyl |
| | 14 | H | $CH_2SO_2Et$ | 2-chloro-1,4-phenylene | 2-trifluoromethylphenyl |
| | 15 | H | $CF_3$ | 2-chloro-1,4-phenylene | 2-trifluoromethylphenyl |

| | | | | | |
|---|---|---|---|---|---|
| | 16 | H | $CH_3$ | 2-chloro-1,4-phenylene | 2-trifluoromethylphenyl |
| | 17 | H | H | 2-chloro-1,4-phenylene | 2-trifluoromethylphenyl |
| D6 | 1 | H | $CH_2OMe$ | 2-methyl-1,4-phenylene | 2-trifluoromethylphenyl |
| | 2 | H | $CH_2OEt$ | 2-methyl-1,4-phenylene | 2-trifluoromethylphenyl |
| | 3 | H | $CH_2O$-n-Pr | 2-methyl-1,4-phenylene | 2-trifluoromethylphenyl |
| | 4 | H | $CH_2O$-i-Pr | 2-methyl-1,4-phenylene | 2-trifluoromethylphenyl |
| | 5 | H | $CH_2O$-n-Bu | 2-methyl-1,4-phenylene | 2-trifluoromethylphenyl |
| | 6 | H | $CH_2O$-i-Bu | 2-methyl-1,4-phenylene | 2-trifluoromethylphenyl |
| | 7 | H | $CH_2Ph$ | 2-methyl-1,4-phenylene | 2-trifluoromethylphenyl |
| | 8 | H | $CH_2$-pyrazol-1-yl | 2-methyl-1,4-phenylene | 2-trifluoromethylphenyl |
| | 9 | H | $CH_2$-imidazol-1-yl | 2-methyl-1,4-phenylene | 2-trifluoromethylphenyl |
| | 10 | H | $CH_2$-tetrazol-1-yl | 2-methyl-1,4-phenylene | 2-trifluoromethylphenyl |
| | 11 | H | $CH_2$-tetrazol-2-yl | 2-methyl-1,4-phenylene | 2-trifluoromethylphenyl |
| | 12 | H | $CH_2$-triazol-1-yl | 2-methyl-1,4-phenylenel | 2-trifluoromethylphenyl |
| | 13 | H | $CH_2SEt$ | 2-methyl-1,4-phenylene | 2-trifluoromethylphenyl |
| | 14 | H | $CH_2SO_2Et$ | 2-methyl-1,4-phenylene | 2-trifluoromethylphenyl |
| | 15 | H | $CF_3$ | 2-methyl-1,4-phenylene | 2-trifluoromethylphenyl |
| | 16 | H | $CH_3$ | 2-methyl-1,4-phenylene | 2-trifluoromethylphenyl |

| | | | | | |
|---|---|---|---|---|---|
| | 17 | H | H | 2-methyl-1,4-phenylene | 2-trifluoromethylphenyl |
| E1 | 1 | H | $CH_2OMe$ | 1,4-phenylene | 2-trifluoromethoxyphenyl |
| | 2 | H | $CH_2OEt$ | 1,4-phenylene | 2-trifluoromethoxyphenyl |
| | 3 | H | $CH_2O$-n-Pr | 1,4-phenylene | 2-trifluoromethoxyphenyl |
| | 4 | H | $CH_2O$-i-Pr | 1,4-phenylene | 2-trifluoromethoxyphenyl |
| | 5 | H | $CH_2O$-n-Bu | 1,4-phenylene | 2-trifluoromethoxyphenyl |
| | 6 | H | $CH_2O$-i-Bu | 1,4-phenylene | 2-trifluoromethoxyphenyl |
| | 7 | H | $CH_2Ph$ | 1,4-phenylene | 2-trifluoromethoxyphenyl |
| | 8 | H | $CH_2$-pyrazol-1-yl | 1,4-phenylene | 2-trifluoromethoxyphenyl |
| | 9 | H | $CH_2$-imidazol-1-yl | 1,4-phenylene | 2-trifluoromethoxyphenyl |
| | 10 | H | $CH_2$-tetrazol-1-yl | 1,4-phenylene | 2-trifluoromethoxyphenyl |
| | 11 | H | $CH_2$-tetrazol-2-yl | 1,4-phenylene | 2-trifluoromethoxyphenyl |
| | 12 | H | $CH_2$-triazol-1-yl | 1,4-phenylene | 2-trifluoromethoxyphenyl |
| | 13 | H | $CH_2SEt$ | 1,4-phenylene | 2-trifluoromethoxyphenyl |
| | 14 | H | $CH_2SO_2Et$ | 1,4-phenylene | 2-trifluoromethoxyphenyl |
| | 15 | H | $CF_3$ | 1,4-phenylene | 2-trifluoromethoxyphenyl |
| | 16 | H | $CH_3$ | 1,4-phenylene | 2-trifluoromethoxyphenyl |
| | 17 | H | H | 1,4-phenylene | 2-trifluoromethoxyphenyl |

| | | | | | |
|---|---|---|---|---|---|
| E2 | 1 | H | $CH_2OMe$ | pyridin-2,5-diyl | 2-trifluoromethoxyphenyl |
| | 2 | H | $CH_2OEt$ | pyridin-2,5-diyl | 2-trifluoromethoxyphenyl |
| | 3 | H | $CH_2O$-n-Pr | pyridin-2,5-diyl | 2-trifluoromethoxyphenyl |
| | 4 | H | $CH_2O$-i-Pr | pyridin-2,5-diyl | 2-trifluoromethoxyphenyl |
| | 5 | H | $CH_2O$-n-Bu | pyridin-2,5-diyl | 2-trifluoromethoxyphenyl |
| | 6 | H | $CH_2O$-i-Bu | pyridin-2,5-diyl | 2-trifluoromethoxyphenyl |
| | 7 | H | $CH_2Ph$ | pyridin-2,5-diyl | 2-trifluoromethoxyphenyl |
| | 8 | H | $CH_2$-pyrazol-1-yl | pyridin-2,5-diyl | 2-trifluoromethoxyphenyl |
| | 9 | H | $CH_2$-imidazol-1-yl | pyridin-2,5-diyl | 2-trifluoromethoxyphenyl |
| | 10 | H | $CH_2$-tetrazol-1-yl | pyridin-2,5-diyl | 2-trifluoromethoxyphenyl |
| | 11 | H | $CH_2$-tetrazol-2-yl | pyridin-2,5-diyl | 2-trifluoromethoxyphenyl |
| | 12 | H | $CH_2$-triazol-1-yl | pyridin-2,5-diyl | 2-trifluoromethoxyphenyl |
| | 13 | H | $CH_2SEt$ | pyridin-2,5-diyl | 2-trifluoromethoxyphenyl |
| | 14 | H | $CH_2SO_2Et$ | pyridin-2,5-diyl | 2-trifluoromethoxyphenyl |
| | 15 | H | $CF_3$ | pyridin-2,5-diyl | 2-trifluoromethoxyphenyl |
| | 16 | H | $CH_3$ | pyridin-2,5-diyl | 2-trifluoromethoxyphenyl |
| | 17 | H | H | pyridin-2,5-diyl | 2-trifluoromethoxyphenyl |
| E3 | 1 | H | $CH_2OMe$ | pyrimidin-2,5-diyl | 2-trifluoromethoxyphenyl |

| | | | | | |
|---|---|---|---|---|---|
| | 2 | H | $CH_2OEt$ | pyrimidin-2,5-diyl | 2-trifluoromethoxyphenyl |
| | 3 | H | $CH_2O$-n-Pr | pyrimidin-2,5-diyl | 2-trifluoromethoxyphenyl |
| | 4 | H | $CH_2O$-i-Pr | pyrimidin-2,5-diyl | 2-trifluoromethoxyphenyl |
| | 5 | H | $CH_2O$-n-Bu | pyrimidin-2,5-diyl | 2-trifluoromethoxyphenyl |
| | 6 | H | $CH_2O$-i-Bu | pyrimidin-2,5-diyl | 2-trifluoromethoxyphenyl |
| | 7 | H | $CH_2Ph$ | pyrimidin-2,5-diyl | 2-trifluoromethoxyphenyl |
| | 8 | H | $CH_2$-pyrazol-1-yl | pyrimidin-2,5-diyl | 2-trifluoromethoxyphenyl |
| | 9 | H | $CH_2$-imidazol-1-yl | pyrimidin-2,5-diyl | 2-trifluoromethoxyphenyl |
| | 10 | H | $CH_2$-tetrazol-1-yl | pyrimidin-2,5-diyl | 2-trifluoromethoxyphenyl |
| | 11 | H | $CH_2$-tetrazol-2-yl | pyrimidin-2,5-diyl | 2-trifluoromethoxyphenyl |
| | 12 | H | $CH_2$-triazol-1-yl | pyrimidin-2,5-diyl | 2-trifluoromethoxyphenyl |
| | 13 | H | $CH_2SEt$ | pyrimidin-2,5-diyl | 2-trifluoromethoxyphenyl |
| | 14 | H | $CH_2SO_2Et$ | pyrimidin-2,5-diyl | 2-trifluoromethoxyphenyl |
| | 15 | H | $CF_3$ | pyrimidin-2,5-diyl | 2-trifluoromethoxyphenyl |
| | 16 | H | $CH_3$ | pyrimidin-2,5-diyl | 2-trifluoromethoxyphenyl |
| | 17 | H | H | pyrimidin-2,5-diyl | 2-trifluoromethoxyphenyl |
| E4 | 1 | H | $CH_2OMe$ | 2-fluoro-1,4-phenylene | 2-trifluoromethoxyphenyl |
| | 2 | H | $CH_2OEt$ | 2-fluoro-1,4-phenylene | 2-trifluoromethoxyphenyl |

| | | | | | |
|---|---|---|---|---|---|
| | 3 | H | $CH_2O$-n-Pr | 2-fluoro-1,4-phenylene | 2-trifluoromethoxyphenyl |
| | 4 | H | $CH_2O$-i-Pr | 2-fluoro-1,4-phenylene | 2-trifluoromethoxyphenyl |
| | 5 | H | $CH_2O$-n-Bu | 2-fluoro-1,4-phenylene | 2-trifluoromethoxyphenyl |
| | 6 | H | $CH_2O$-i-Bu | 2-fluoro-1,4-phenylene | 2-trifluoromethoxyphenyl |
| | 7 | H | $CH_2Ph$ | 2-fluoro-1,4-phenylene | 2-trifluoromethoxyphenyl |
| | 8 | H | $CH_2$-pyrazol-1-yl | 2-fluoro-1,4-phenylene | 2-trifluoromethoxyphenyl |
| | 9 | H | $CH_2$-imidazol-1-yl | 2-fluoro-1,4-phenylene | 2-trifluoromethoxyphenyl |
| | 10 | H | $CH_2$-tetrazol-1-yl | 2-fluoro-1,4-phenylene | 2-trifluoromethoxyphenyl |
| | 11 | H | $CH_2$-tetrazol-2-yl | 2-fluoro-1,4-phenylene | 2-trifluoromethoxyphenyl |
| | 12 | H | $CH_2$-triazol-1-yl | 2-fluoro-1,4-phenylene | 2-trifluoromethoxyphenyl |
| | 13 | H | $CH_2SEt$ | 2-fluoro-1,4-phenylene | 2-trifluoromethoxyphenyl |
| | 14 | H | $CH_2SO_2Et$ | 2-fluoro-1,4-phenylene | 2-trifluoromethoxyphenyl |
| | 15 | H | $CF_3$ | 2-fluoro-1,4-phenylene | 2-trifluoromethoxyphenyl |
| | 16 | H | $CH_3$ | 2-fluoro-1,4-phenylene | 2-trifluoromethoxyphenyl |
| | 17 | H | H | 2-fluoro-1,4-phenylene | 2-trifluoromethoxyphenyl |
| E5 | 1 | H | $CH_2OMe$ | 2-chloro-1,4-phenylene | 2-trifluoromethoxyphenyl |
| | 2 | H | $CH_2OEt$ | 2-chloro-1,4-phenylene | 2-trifluoromethoxyphenyl |
| | 3 | H | $CH_2O$-n-Pr | 2-chloro-1,4-phenylene | 2-trifluoromethoxyphenyl |

| | | | | | |
|---|---|---|---|---|---|
| | 4 | H | $CH_2O$-i-Pr | 2-chloro-1,4-phenylene | 2-trifluoromethoxyphenyl |
| | 5 | H | $CH_2O$-n-Bu | 2-chloro-1,4-phenylene | 2-trifluoromethoxyphenyl |
| | 6 | H | $CH_2O$-i-Bu | 2-chloro-1,4-phenylene | 2-trifluoromethoxyphenyl |
| | 7 | H | $CH_2Ph$ | 2-chloro-1,4-phenylene | 2-trifluoromethoxyphenyl |
| | 8 | H | $CH_2$-pyrazol-1-yl | 2-chloro-1,4-phenylene | 2-trifluoromethoxyphenyl |
| | 9 | H | $CH_2$-imidazol-1-yl | 2-chloro-1,4-phenylene | 2-trifluoromethoxyphenyl |
| | 10 | H | $CH_2$-tetrazol-1-yl | 2-chloro-1,4-phenylene | 2-trifluoromethoxyphenyl |
| | 11 | H | $CH_2$-tetrazol-2-yl | 2-chloro-1,4-phenylene | 2-trifluoromethoxyphenyl |
| | 12 | H | $CH_2$-triazol-1-yl | 2-chloro-1,4-phenylene | 2-trifluoromethoxyphenyl |
| | 13 | H | $CH_2SEt$ | 2-chloro-1,4-phenylene | 2-trifluoromethoxyphenyl |
| | 14 | H | $CH_2SO_2Et$ | 2-chloro-1,4-phenylene | 2-trifluoromethoxyphenyl |
| | 15 | H | $CF_3$ | 2-chloro-1,4-phenylene | 2-trifluoromethoxyphenyl |
| | 16 | H | $CH_3$ | 2-chloro-1,4-phenylene | 2-trifluoromethoxyphenyl |
| | 17 | H | H | 2-chloro-1,4-phenylene | 2-trifluoromethoxyphenyl |
| E6 | 1 | H | $CH_2OMe$ | 2-methyl-1,4-phenylene | 2-trifluoromethoxyphenyl |
| | 2 | H | $CH_2OEt$ | 2-methyl-1,4-phenylene | 2-trifluoromethoxyphenyl |
| | 3 | H | $CH_2O$-n-Pr | 2-methyl-1,4-phenylene | 2-trifluoromethoxyphenyl |
| | 4 | H | $CH_2O$-i-Pr | 2-methyl-1,4-phenylene | 2-trifluoromethoxyphenyl |

| | | | | | |
|---|---|---|---|---|---|
| | 5 | H | $CH_2O$-n-Bu | 2-methyl-1,4-phenylene | 2-trifluoromethoxyphenyl |
| | 6 | H | $CH_2O$-i-Bu | 2-methyl-1,4-phenylene | 2-trifluoromethoxyphenyl |
| | 7 | H | $CH_2Ph$ | 2-methyl-1,4-phenylene | 2-trifluoromethoxyphenyl |
| | 8 | H | $CH_2$-pyrazol-1-yl | 2-methyl-1,4-phenylene | 2-trifluoromethoxyphenyl |
| | 9 | H | $CH_2$-imidazol-1-yl | 2-methyl-1,4-phenylene | 2-trifluoromethoxyphenyl |
| | 10 | H | $CH_2$-tetrazol-1-yl | 2-methyl-1,4-phenylene | 2-trifluoromethoxyphenyl |
| | 11 | H | $CH_2$-tetrazol-2-yl | 2-methyl-1,4-phenylene | 2-trifluoromethoxyphenyl |
| | 12 | H | $CH_2$-triazol-1-yl | 2-methyl-1,4-phenylene | 2-trifluoromethoxyphenyl |
| | 13 | H | $CH_2SEt$ | 2-methyl-1,4-phenylene | 2-trifluoromethoxyphenyl |
| | 14 | H | $CH_2SO_2Et$ | 2-methyl-1,4-phenylene | 2-trifluoromethoxyphenyl |
| | 15 | H | $CF_3$ | 2-methyl-1,4-phenylene | 2-trifluoromethoxyphenyl |
| | 16 | H | $CH_3$ | 2-methyl-1,4-phenylene | 2-trifluoromethoxyphenyl |
| | 17 | H | H | 2-methyl-1,4-phenylene | 2-trifluoromethoxyphenyl |
| F1 | 1 | H | $CH_2OMe$ | 1,4-phenylene | 2-trifluoromethyl-sulfonyl-phenyl |
| | 2 | H | $CH_2OEt$ | 1,4-phenylene | 2-trifluoromethyl-sulfonyl-phenyl |
| | 3 | H | $CH_2O$-n-Pr | 1,4-phenylene | 2-trifluoromethyl-sulfonyl-phenyl |
| | 4 | H | $CH_2O$-i-Pr | 1,4-phenylene | 2-trifluoromethyl-sulfonyl-phenyl |
| | 5 | H | $CH_2O$-n-Bu | 1,4-phenylene | 2-trifluoromethyl-sulfonyl-phenyl |

| | | | | | |
|---|---|---|---|---|---|
| | 6 | H | $CH_2O$-i-Bu | 1,4-phenylene | 2-trifluoromethyl-sulfonyl-phenyl |
| | 7 | H | $CH_2Ph$ | 1,4-phenylene | 2-trifluoromethyl-sulfonyl-phenyl |
| | 8 | H | $CH_2$-pyrazol-1-yl | 1,4-phenylene | 2-trifluoromethyl-sulfonyl-phenyl |
| | 9 | H | $CH_2$-imidazol-1-yl | 1,4-phenylene | 2-trifluoromethyl-sulfonyl-phenyl |
| | 10 | H | $CH_2$-tetrazol-1-yl | 1,4-phenylene | 2-trifluoromethyl-sulfonyl-phenyl |
| | 11 | H | $CH_2$-tetrazol-2-yl | 1,4-phenylene | 2-trifluoromethyl-sulfonyl-phenyl |
| | 12 | H | $CH_2$-triazol-1-yl | 1,4-phenylene | 2-trifluoromethyl-sulfonyl-phenyl |
| | 13 | H | $CH_2SEt$ | 1,4-phenylene | 2-trifluoromethyl-sulfonyl-phenyl |
| | 14 | H | $CH_2SO_2Et$ | 1,4-phenylene | 2-trifluoromethyl-sulfonyl-phenyl |
| | 15 | H | $CF_3$ | 1,4-phenylene | 2-trifluoromethyl-sulfonyl-phenyl |
| | 16 | H | $CH_3$ | 1,4-phenylene | 2-trifluoromethyl-sulfonyl-phenyl |
| | 17 | H | H | 1,4-phenylene | 2-trifluoromethyl-sulfonyl-phenyl |
| F2 | 1 | H | $CH_2OMe$ | pyridin-2,5-diyl | 2-trifluoromethyl-sulfonyl-phenyl |
| | 2 | H | $CH_2OEt$ | pyridin-2,5-diyl | 2-trifluoromethyl-sulfonyl-phenyl |
| | 3 | H | $CH_2O$-n-Pr | pyridin-2,5-diyl | 2-trifluoromethyl-sulfonyl-phenyl |
| | 4 | H | $CH_2O$-i-Pr | pyridin-2,5-diyl | 2-trifluoromethyl-sulfonyl-phenyl |
| | 5 | H | $CH_2O$-n-Bu | pyridin-2,5-diyl | 2-trifluoromethyl-sulfonyl-phenyl |
| | 6 | H | $CH_2O$-i-Bu | pyridin-2,5-diyl | 2-trifluoromethyl-sulfonyl-phenyl |

| | | | | | |
|---|---|---|---|---|---|
| | 7 | H | $CH_2Ph$ | pyridin-2,5-diyl | 2-trifluoromethyl-sulfonyl-phenyl |
| | 8 | H | $CH_2$-pyrazol-1-yl | pyridin-2,5-diyl | 2-trifluoromethyl-sulfonyl-phenyl |
| | 9 | H | $CH_2$-imidazol-1-yl | pyridin-2,5-diyl | 2-trifluoromethyl-sulfonyl-phenyl |
| | 10 | H | $CH_2$-tetrazol-1-yl | pyridin-2,5-diyl | 2-trifluoromethyl-sulfonyl-phenyl |
| | 11 | H | $CH_2$-tetrazol-2-yl | pyridin-2,5-diyl | 2-trifluoromethyl-sulfonyl-phenyl |
| | 12 | H | $CH_2$-triazol-1-yl | pyridin-2,5-diyl | 2-trifluoromethyl-sulfonyl-phenyl |
| | 13 | H | $CH_2SEt$ | pyridin-2,5-diyl | 2-trifluoromethyl-sulfonyl-phenyl |
| | 14 | H | $CH_2SO_2Et$ | pyridin-2,5-diyl | 2-trifluoromethyl-sulfonyl-phenyl |
| | 15 | H | $CF_3$ | pyridin-2,5-diyl | 2-trifluoromethyl-sulfonyl-phenyl |
| | 16 | H | $CH_3$ | pyridin-2,5-diyl | 2-trifluoromethyl-sulfonyl-phenyl |
| | 17 | H | H | pyridin-2,5-diyl | 2-trifluoromethyl-sulfonyl-phenyl |
| F3 | 1 | H | $CH_2OMe$ | pyrimidin-2,5-diyl | 2-trifluoromethyl-sulfonyl-phenyl |
| | 2 | H | $CH_2OEt$ | pyrimidin-2,5-diyl | 2-trifluoromethyl-sulfonyl-phenyl |
| | 3 | H | $CH_2O$-n-Pr | pyrimidin-2,5-diyl | 2-trifluoromethyl-sulfonyl-phenyl |
| | 4 | H | $CH_2O$-i-Pr | pyrimidin-2,5-diyl | 2-trifluoromethyl-sulfonyl-phenyl |
| | 5 | H | $CH_2O$-n-Bu | pyrimidin-2,5-diyl | 2-trifluoromethyl-sulfonyl-phenyl |
| | 6 | H | $CH_2O$-i-Bu | pyrimidin-2,5-diyl | 2-trifluoromethyl-sulfonyl-phenyl |
| | 7 | H | $CH_2Ph$ | pyrimidin-2,5-diyl | 2-trifluoromethyl-sulfonyl-phenyl |

| | | | | | |
|---|---|---|---|---|---|
| | 8 | H | $CH_2$-pyrazol-1-yl | pyrimidin-2,5-diyl | 2-trifluoromethyl-sulfonyl-phenyl |
| | 9 | H | $CH_2$-imidazol-1-yl | pyrimidin-2,5-diyl | 2-trifluoromethyl-sulfonyl-phenyl |
| | 10 | H | $CH_2$-tetrazol-1-yl | pyrimidin-2,5-diyl | 2-trifluoromethyl-sulfonyl-phenyl |
| | 11 | H | $CH_2$-tetrazol-2-yl | pyrimidin-2,5-diyl | 2-trifluoromethyl-sulfonyl-phenyl |
| | 12 | H | $CH_2$-triazol-1-yl | pyrimidin-2,5-diyl | 2-trifluoromethyl-sulfonyl-phenyl |
| | 13 | H | $CH_2SEt$ | pyrimidin-2,5-diyl | 2-trifluoromethyl-sulfonyl-phenyl |
| | 14 | H | $CH_2SO_2Et$ | pyrimidin-2,5-diyl | 2-trifluoromethyl-sulfonyl-phenyl |
| | 15 | H | $CF_3$ | pyrimidin-2,5-diyl | 2-trifluoromethyl-sulfonyl-phenyl |
| | 16 | H | $CH_3$ | pyrimidin-2,5-diyl | 2-trifluoromethyl-sulfonyl-phenyl |
| | 17 | H | H | pyrimidin-2,5-diyl | 2-trifluoromethyl-sulfonyl-phenyl |
| F4 | 1 | H | $CH_2OMe$ | 2-fluoro-1,4-phenylene | 2-trifluoromethyl-sulfonyl-phenyl |
| | 2 | H | $CH_2OEt$ | 2-fluoro-1,4-phenylene | 2-trifluoromethyl-sulfonyl-phenyl |
| | 3 | H | $CH_2O$-n-Pr | 2-fluoro-1,4-phenylene | 2-trifluoromethyl-sulfonyl-phenyl |
| | 4 | H | $CH_2O$-i-Pr | 2-fluoro-1,4-phenylene | 2-trifluoromethyl-sulfonyl-phenyl |
| | 5 | H | $CH_2O$-n-Bu | 2-fluoro-1,4-phenylene | 2-trifluoromethyl-sulfonyl-phenyl |
| | 6 | H | $CH_2O$-i-Bu | 2-fluoro-1,4-phenylene | 2-trifluoromethyl-sulfonyl-phenyl |
| | 7 | H | $CH_2Ph$ | 2-fluoro-1,4-phenylene | 2-trifluoromethyl-sulfonyl-phenyl |
| | 8 | H | $CH_2$-pyrazol-1-yl | 2-fluoro-1,4-phenylene | 2-trifluoromethyl-sulfonyl-phenyl |

| | | | | | |
|---|---|---|---|---|---|
| | 9 | H | $CH_2$-imidazol-1-yl | 2-fluoro-1,4-phenylene | 2-trifluoromethyl-sulfonyl-phenyl |
| | 10 | H | $CH_2$-tetrazol-1-yl | 2-fluoro-1,4-phenylene | 2-trifluoromethyl-sulfonyl-phenyl |
| | 11 | H | $CH_2$-tetrazol-2-yl | 2-fluoro-1,4-phenylene | 2-trifluoromethyl-sulfonyl-phenyl |
| | 12 | H | $CH_2$-triazol-1-yl | 2-fluoro-1,4-phenylene | 2-trifluoromethyl-sulfonyl-phenyl |
| | 13 | H | $CH_2SEt$ | 2-fluoro-1,4-phenylene | 2-trifluoromethyl-sulfonyl-phenyl |
| | 14 | H | $CH_2SO_2Et$ | 2-fluoro-1,4-phenylene | 2-trifluoromethyl-sulfonyl-phenyl |
| | 15 | H | $CF_3$ | 2-fluoro-1,4-phenylene | 2-trifluoromethyl-sulfonyl-phenyl |
| | 16 | H | $CH_3$ | 2-fluoro-1,4-phenylene | 2-trifluoromethyl-sulfonyl-phenyl |
| | 17 | H | H | 2-fluoro-1,4-phenylene | 2-trifluoromethyl-sulfonyl-phenyl |
| F5 | 1 | H | $CH_2OMe$ | 2-chloro-1,4-phenylene | 2-trifluoromethyl-sulfonyl-phenyl |
| | 2 | H | $CH_2OEt$ | 2-chloro-1,4-phenylene | 2-trifluoromethyl-sulfonyl-phenyl |
| | 3 | H | $CH_2O$-n-Pr | 2-chloro-1,4-phenylene | 2-trifluoromethyl-sulfonyl-phenyl |
| | 4 | H | $CH_2O$-i-Pr | 2-chloro-1,4-phenylene | 2-trifluoromethyl-sulfonyl-phenyl |
| | 5 | H | $CH_2O$-n-Bu | 2-chloro-1,4-phenylene | 2-trifluoromethyl-sulfonyl-phenyl |
| | 6 | H | $CH_2O$-i-Bu | 2-chloro-1,4-phenylene | 2-trifluoromethyl-sulfonyl-phenyl |
| | 7 | H | $CH_2Ph$ | 2-chloro-1,4-phenylene | 2-trifluoromethyl-sulfonyl-phenyl |
| | 8 | H | $CH_2$-pyrazol-1-yl | 2-chloro-1,4-phenylene | 2-trifluoromethyl-sulfonyl-phenyl |
| | 9 | H | $CH_2$-imidazol-1-yl | 2-chloro-1,4-phenylene | 2-trifluoromethyl-sulfonyl-phenyl |

| | | | | | |
|---|---|---|---|---|---|
| | 10 | H | $CH_2$-tetrazol-1-yl | 2-chloro-1,4-phenylene | 2-trifluoromethyl-sulfonyl-phenyl |
| | 11 | H | $CH_2$-tetrazol-2-yl | 2-chloro-1,4-phenylene | 2-trifluoromethyl-sulfonyl-phenyl |
| | 12 | H | $CH_2$-triazol-1-yl | 2-chloro-1,4-phenylene | 2-trifluoromethyl-sulfonyl-phenyl |
| | 13 | H | $CH_2SEt$ | 2-chloro-1,4-phenylene | 2-trifluoromethyl-sulfonyl-phenyl |
| | 14 | H | $CH_2SO_2Et$ | 2-chloro-1,4-phenylene | 2-trifluoromethyl-sulfonyl-phenyl |
| | 15 | H | $CF_3$ | 2-chloro-1,4-phenylene | 2-trifluoromethyl-sulfonyl-phenyl |
| | 16 | H | $CH_3$ | 2-chloro-1,4-phenylene | 2-trifluoromethyl-sulfonyl-phenyl |
| | 17 | H | H | 2-chloro-1,4-phenylene | 2-trifluoromethyl-sulfonyl-phenyl |
| F6 | 1 | H | $CH_2OMe$ | 2-methyl-1,4-phenylene | 2-trifluoromethyl-sulfonyl-phenyl |
| | 2 | H | $CH_2OEt$ | 2-methyl-1,4-phenylene | 2-trifluoromethyl-sulfonyl-phenyl |
| | 3 | H | $CH_2O$-n-Pr | 2-methyl-1,4-phenylene | 2-trifluoromethyl-sulfonyl-phenyl |
| | 4 | H | $CH_2O$-i-Pr | 2-methyl-1,4-phenylene | 2-trifluoromethyl-sulfonyl-phenyl |
| | 5 | H | $CH_2O$-n-Bu | 2-methyl-1,4-phenylene | 2-trifluoromethyl-sulfonyl-phenyl |
| | 6 | H | $CH_2O$-i-Bu | 2-methyl-1,4-phenylene | 2-trifluoromethyl-sulfonyl-phenyl |
| | 7 | H | $CH_2Ph$ | 2-methyl-1,4-phenylene | 2-trifluoromethyl-sulfonyl-phenyl |
| | 8 | H | $CH_2$-pyrazol-1-yl | 2-methyl-1,4-phenylene | 2-trifluoromethyl-sulfonyl-phenyl |
| | 9 | H | $CH_2$-imidazol-1-yl | 2-methyl-1,4-phenylene | 2-trifluoromethyl-sulfonyl-phenyl |
| | 10 | H | $CH_2$-tetrazol-1-yl | 2-methyl-1,4-phenylene | 2-trifluoromethyl-sulfonyl-phenyl |

| | | | | | |
|---|---|---|---|---|---|
| | 11 | H | $CH_2$-tetrazol-2-yl | 2-methyl-1,4-phenylene | 2-trifluoromethyl-sulfonyl-phenyl |
| | 12 | H | $CH_2$-triazol-1-yl | 2-methyl-1,4-phenylene | 2-trifluoromethyl-sulfonyl-phenyl |
| | 13 | H | $CH_2SEt$ | 2-methyl-1,4-phenylene | 2-trifluoromethyl-sulfonyl-phenyl |
| | 14 | H | $CH_2SO_2Et$ | 2-methyl-1,4-phenylene | 2-trifluoromethyl-sulfonyl-phenyl |
| | 15 | H | $CF_3$ | 2-methyl-1,4-phenylene | 2-trifluoromethyl-sulfonyl-phenyl |
| | 16 | H | $CH_3$ | 2-methyl-1,4-phenylene | 2-trifluoromethyl-sulfonyl-phenyl |
| | 17 | H | H | 2-methyl-1,4-phenylene | 2-trifluoromethyl-sulfonyl-phenyl |
| G1 | 1 | H | $CH_2OMe$ | phenyl | 2-methoxyphenyl |
| | 2 | H | $CH_2OEt$ | phenyl | 2-methoxyphenyl |
| | 3 | H | $CH_2O$-n-Pr | phenyl | 2-methoxyphenyl |
| | 4 | H | $CH_2O$-i-Pr | phenyl | 2-methoxyphenyl |
| | 5 | H | $CH_2O$-n-Bu | phenyl | 2-methoxyphenyl |
| | 6 | H | $CH_2O$-i-Bu | phenyl | 2-methoxyphenyl |
| | 7 | H | $CH_2Ph$ | phenyl | 2-methoxyphenyl |
| | 8 | H | $CH_2$-pyrazol-1-yl | phenyl | 2-methoxyphenyl |
| | 9 | H | $CH_2$-imidazol-1-yl | phenyl | 2-methoxyphenyl |
| | 10 | H | $CH_2$-tetrazol-1-yl | phenyl | 2-methoxyphenyl |
| | 11 | H | $CH_2$-tetrazol-2-yl | phenyl | 2-methoxyphenyl |
| | 12 | H | $CH_2$-triazol-1-yl | phenyl | 2-methoxyphenyl |
| | 13 | H | $CH_2SEt$ | phenyl | 2-methoxyphenyl |
| | 14 | H | $CH_2SO_2Et$ | phenyl | 2-methoxyphenyl |
| | 15 | H | $CF_3$ | phenyl | 2-methoxyphenyl |
| | 16 | H | $CH_3$ | phenyl | 2-methoxyphenyl |
| | 17 | H | H | phenyl | 2-methoxyphenyl |

| | | | | | |
|---|---|---|---|---|---|
| G2 | 1 | H | $CH_2OMe$ | pyridin-2,5-diyl | 2-methoxyphenyl |
| | 2 | H | $CH_2OEt$ | pyridin-2,5-diyl | 2-methoxyphenyl |
| | 3 | H | $CH_2O$-n-Pr | pyridin-2,5-diyl | 2-methoxyphenyl |
| | 4 | H | $CH_2O$-i-Pr | pyridin-2,5-diyl | 2-methoxyphenyl |
| | 5 | H | $CH_2O$-n-Bu | pyridin-2,5-diyl | 2-methoxyphenyl |
| | 6 | H | $CH_2O$-i-Bu | pyridin-2,5-diyl | 2-methoxyphenyl |
| | 7 | H | $CH_2Ph$ | pyridin-2,5-diyl | 2-methoxyphenyl |
| | 8 | H | $CH_2$-pyrazol-1-yl | pyridin-2,5-diyl | 2-methoxyphenyl |
| | 9 | H | $CH_2$-imidazol-1-yl | pyridin-2,5-diyl | 2-methoxyphenyl |
| | 10 | H | $CH_2$-tetrazol-1-yl | pyridin-2,5-diyl | 2-methoxyphenyl |
| | 11 | H | $CH_2$-tetrazol-2-yl | pyridin-2,5-diyl | 2-methoxyphenyl |
| | 12 | H | $CH_2$-triazol-1-yl | pyridin-2,5-diyl | 2-methoxyphenyl |
| | 13 | H | $CH_2SEt$ | pyridin-2,5-diyl | 2-methoxyphenyl |
| | 14 | H | $CH_2SO_2Et$ | pyridin-2,5-diyl | 2-methoxyphenyl |
| | 15 | H | $CF_3$ | pyridin-2,5-diyl | 2-methoxyphenyl |
| | 16 | H | $CH_3$ | pyridin-2,5-diyl | 2-methoxyphenyl |
| | 17 | H | H | pyridin-2,5-diyl | 2-methoxyphenyl |
| G3 | 1 | H | $CH_2OMe$ | pyrimidin-2,5-diyl | 2-methoxyphenyl |

| | | | | | |
|---|---|---|---|---|---|
| | 2 | H | $CH_2OEt$ | pyrimidin-2,5-diyl | 2-methoxyphenyl |
| | 3 | H | $CH_2O$-n-Pr | pyrimidin-2,5-diyl | 2-methoxyphenyl |
| | 4 | H | $CH_2O$-i-Pr | pyrimidin-2,5-diyl | 2-methoxyphenyl |
| | 5 | H | $CH_2O$-n-Bu | pyrimidin-2,5-diyl | 2-methoxyphenyl |
| | 6 | H | $CH_2O$-i-Bu | pyrimidin-2,5-diyl | 2-methoxyphenyl |
| | 7 | H | $CH_2Ph$ | pyrimidin-2,5-diyl | 2-methoxyphenyl |
| | 8 | H | $CH_2$-pyrazol-1-yl | pyrimidin-2,5-diyl | 2-methoxyphenyl |
| | 9 | H | $CH_2$-imidazol-1-yl | pyrimidin-2,5-diyl | 2-methoxyphenyl |
| | 10 | H | $CH_2$-tetrazol-1-yl | pyrimidin-2,5-diyl | 2-methoxyphenyl |
| | 11 | H | $CH_2$-tetrazol-2-yl | pyrimidin-2,5-diyl | 2-methoxyphenyl |
| | 12 | H | $CH_2$-triazol-1-yl | pyrimidin-2,5-diyl | 2-methoxyphenyl |
| | 13 | H | $CH_2SEt$ | pyrimidin-2,5-diyl | 2-methoxyphenyl |
| | 14 | H | $CH_2SO_2Et$ | pyrimidin-2,5-diyl | 2-methoxyphenyl |
| | 15 | H | $CF_3$ | pyrimidin-2,5-diyl | 2-methoxyphenyl |
| | 16 | H | $CH_3$ | pyrimidin-2,5-diyl | 2-methoxyphenyl |
| | 17 | H | H | pyrimidin-2,5-diyl | 2-methoxyphenyl |
| G4 | 1 | H | $CH_2OMe$ | 2-fluoro-1,4-phenylene | 2-methoxyphenyl |
| | 2 | H | $CH_2OEt$ | 2-fluoro-1,4-phenylene | 2-methoxyphenyl |

| | | | | | |
|---|---|---|---|---|---|
| | 3 | H | $CH_2O$-n-Pr | 2-fluoro-1,4-phenylene | 2-methoxyphenyl |
| | 4 | H | $CH_2O$-i-Pr | 2-fluoro-1,4-phenylene | 2-methoxyphenyl |
| | 5 | H | $CH_2O$-n-Bu | 2-fluoro-1,4-phenylene | 2-methoxyphenyl |
| | 6 | H | $CH_2O$-i-Bu | 2-fluoro-1,4-phenylene | 2-methoxyphenyl |
| | 7 | H | $CH_2Ph$ | 2-fluoro-1,4-phenylene | 2-methoxyphenyl |
| | 8 | H | $CH_2$-pyrazol-1-yl | 2-fluoro-1,4-phenylene | 2-methoxyphenyl |
| | 9 | H | $CH_2$-imidazol-1-yl | 2-fluoro-1,4-phenylene | 2-methoxyphenyl. |
| | 10 | H | $CH_2$-tetrazol-1-yl | 2-fluoro-1,4-phenylene | 2-methoxyphenyl |
| | 11 | H | $CH_2$-tetrazol-2-yl | 2-fluoro-1,4-phenylene | 2-methoxyphenyl |
| | 12 | H | $CH_2$-triazol-1-yl | 2-fluoro-1,4-phenylene | 2-methoxyphenyl |
| | 13 | H | $CH_2SEt$ | 2-fluoro-1,4-phenylene | 2-methoxyphenyl |
| | 14 | H | $CH_2SO_2Et$ | 2-fluoro-1,4-phenylene | 2-methoxyphenyl |
| | 15 | H | $CF_3$ | 2-fluoro-1,4-phenylene | 2-methoxyphenyl |
| | 16 | H | $CH_3$ | 2-fluoro-1,4-phenylene | 2-methoxyphenyl |
| | 17 | H | H | 2-fluoro-1,4-phenylene | 2-methoxyphenyl |
| G5 | 1 | H | $CH_2OMe$ | 2-chloro-1,4-phenylene | 2-methoxyphenyl |
| | 2 | H | $CH_2OEt$ | 2-chloro-1,4-phenylene | 2-methoxyphenyl |
| | 3 | H | $CH_2O$-n-Pr | 2-chloro-1,4-phenylene | 2-methoxyphenyl |

| | | | | | |
|---|---|---|---|---|---|
| | 4 | H | $CH_2O$-i-Pr | 2-chloro-1,4-phenylene | 2-methoxyphenyl |
| | 5 | H | $CH_2O$-n-Bu | 2-chloro-1,4-phenylene | 2-methoxyphenyl |
| | 6 | H | $CH_2O$-i-Bu | 2-chloro-1,4-phenylene | 2-methoxyphenyl |
| | 7 | H | $CH_2Ph$ | 2-chloro-1,4-phenylene | 2-methoxyphenyl |
| | 8 | H | $CH_2$-pyrazol-1-yl | 2-chloro-1,4-phenylene | 2-methoxyphenyl |
| | 9 | H | $CH_2$-imidazol-1-yl | 2-chloro-1,4-phenylene | 2-methoxyphenyl |
| | 10 | H | $CH_2$-tetrazol-1-yl | 2-chloro-1,4-phenylene | 2-methoxyphenyl |
| | 11 | H | $CH_2$-tetrazol-2-yl | 2-chloro-1,4-phenylene | 2-methoxyphenyl |
| | 12 | H | $CH_2$-triazol-1-yl | 2-chloro-1,4-phenylene | 2-methoxyphenyl |
| | 13 | H | $CH_2SEt$ | 2-chloro-1,4-phenylene | 2-methoxyphenyl |
| | 14 | H | $CH_2SO_2Et$ | 2-chloro-1,4-phenylene | 2-methoxyphenyl |
| | 15 | H | $CF_3$ | 2-chloro-1,4-phenylene | 2-methoxyphenyl |
| | 16 | H | $CH_3$ | 2-chloro-1,4-phenylene | 2-methoxyphenyl |
| | 17 | H | H | 2-chloro-1,4-phenylene | 2-methoxyphenyl |
| G6 | 1 | H | $CH_2OMe$ | 2-methyl-1,4-phenylene | 2-methoxyphenyl |
| | 2 | H | $CH_2OEt$ | 2-methyl-1,4-phenylene | 2-methoxyphenyl |
| | 3 | H | $CH_2O$-n-Pr | 2-methyl-1,4-phenylene | 2-methoxyphenyl |
| | 4 | H | $CH_2O$-i-Pr | 2-methyl-1,4-phenylene | 2-methoxyphenyl |

| | | | | | |
|---|---|---|---|---|---|
| | 5 | H | $CH_2O$-n-Bu | 2-methyl-1,4-phenylene | 2-methoxyphenyl |
| | 6 | H | $CH_2O$-i-Bu | 2-methyl-1,4-phenylene | 2-methoxyphenyl |
| | 7 | H | $CH_2Ph$ | 2-methyl-1,4-phenylene | 2-methoxyphenyl |
| | 8 | H | $CH_2$-pyrazol-1-yl | 2-methyl-1,4-phenylene | 2-methoxyphenyl |
| | 9 | H | $CH_2$-imidazol-1-yl | 2-methyl-1,4-phenylene | 2-methoxyphenyl |
| | 10 | H | $CH_2$-tetrazol-1-yl | 2-methyl-1,4-phenylene | 2-methoxyphenyl |
| | 11 | H | $CH_2$-tetrazol-2-yl | 2-methyl-1,4-phenylene | 2-methoxyphenyl |
| | 12 | H | $CH_2$-triazol-1-yl | 2-methyl-1,4-phenylene | 2-methoxyphenyl |
| | 13 | H | $CH_2SEt$ | 2-methyl-1,4-phenylene | 2-methoxyphenyl |
| | 14 | H | $CH_2SO_2Et$ | 2-methyl-1,4-phenylene | 2-methoxyphenyl |
| | 15 | H | $CF_3$ | 2-methyl-1,4-phenylene | 2-methoxyphenyl |
| | 16 | H | $CH_3$ | 2-methyl-1,4-phenylene | 2-methoxyphenyl |
| | 17 | H | H | 2-methyl-1,4-phenylene | 2-methoxyphenyl |
| H1 | 1 | H | $CH_2OMe$ | phenyl | 2-methylsulfonylphenyl |
| | 2 | H | $CH_2OEt$ | phenyl | 2-methylsulfonylphenyl |
| | 3 | H | $CH_2O$-n-Pr | phenyl | 2-methylsulfonylphenyl |
| | 4 | H | $CH_2O$-i-Pr | phenyl | 2-methylsulfonylphenyl |
| | 5 | H | $CH_2O$-n-Bu | phenyl | 2-methylsulfonylphenyl |

| | | | | | |
|---|---|---|---|---|---|
| | 6 | H | $CH_2O$-i-Bu | phenyl | 2-methylsulfonylphenyl |
| | 7 | H | $CH_2Ph$ | phenyl | 2-methylsulfonylphenyl |
| | 8 | H | $CH_2$-pyrazol-1-yl | phenyl | 2-methylsulfonylphenyl |
| | 9 | H | $CH_2$-imidazol-1-yl | phenyl | 2-methylsulfonylphenyl |
| | 10 | H | $CH_2$-tetrazol-1-yl | phenyl | 2-methylsulfonylphenyl |
| | 11 | H | $CH_2$-tetrazol-2-yl | phenyl | 2-methylsulfonylphenyl |
| | 12 | H | $CH_2$-triazol-1-yl | phenyl | 2-methylsulfonylphenyl |
| | 13 | H | $CH_2SEt$ | phenyl | 2-methylsulfonylphenyl |
| | 14 | H | $CH_2SO_2Et$ | phenyl | 2-methylsulfonylphenyl |
| | 15 | H | $CF_3$ | phenyl | 2-methylsulfonylphenyl |
| | 16 | H | $CH_3$ | phenyl | 2-methylsulfonylphenyl |
| | 17 | H | H | phenyl | 2-methylsulfonylphenyl |
| H2 | 1 | H | $CH_2OMe$ | pyridin-2,5-diyl | 2-methylsulfonylphenyl |
| | 2 | H | $CH_2OEt$ | pyridin-2,5-diyl | 2-methylsulfonylphenyl |
| | 3 | H | $CH_2O$-n-Pr | pyridin-2,5-diyl | 2-methylsulfonylphenyl |
| | 4 | H | $CH_2O$-i-Pr | pyridin-2,5-diyl | 2-methylsulfonylphenyl |
| | 5 | H | $CH_2O$-n-Bu | pyridin-2,5-diyl | 2-methylsulfonylphenyl |
| | 6 | H | $CH_2O$-i-Bu | pyridin-2,5-diyl | 2-methylsulfonylphenyl |

| | | | | | |
|---|---|---|---|---|---|
| | 7 | H | $CH_2Ph$ | pyridin-2,5-diyl | 2-methylsulfonylphenyl |
| | 8 | H | $CH_2$-pyrazol-1-yl | pyridin-2,5-diyl | 2-methylsulfonylphenyl |
| | 9 | H | $CH_2$-imidazol-1-yl | pyridin-2,5-diyl | 2-methylsulfonylphenyl |
| | 10 | H | $CH_2$-tetrazol-1-yl | pyridin-2,5-diyl | 2-methylsulfonylphenyl |
| | 11 | H | $CH_2$-tetrazol-2-yl | pyridin-2,5-diyl | 2-methylsulfonylphenyl |
| | 12 | H | $CH_2$-triazol-1-yl | pyridin-2,5-diyl | 2-methylsulfonylphenyl |
| | 13 | H | $CH_2SEt$ | pyridin-2,5-diyl | 2-methylsulfonylphenyl |
| | 14 | H | $CH_2SO_2Et$ | pyridin-2,5-diyl | 2-methylsulfonylphenyl |
| | 15 | H | $CF_3$ | pyridin-2,5-diyl | 2-methylsulfonylphenyl |
| | 16 | H | $CH_3$ | pyridin-2,5-diyl | 2-methylsulfonylphenyl |
| | 17 | H | H | pyridin-2,5-diyl | 2-methylsulfonylphenyl |
| H3 | 1 | H | $CH_2OMe$ | pyrimidin-2,5-diyl | 2-methylsulfonylphenyl |
| | 2 | H | $CH_2OEt$ | pyrimidin-2,5-diyl | 2-methylsulfonylphenyl |
| | 3 | H | $CH_2O$-n-Pr | pyrimidin-2,5-diyl | 2-methylsulfonylphenyl |
| | 4 | H | $CH_2O$-i-Pr | pyrimidin-2,5-diyl | 2-methylsulfonylphenyl |
| | 5 | H | $CH_2O$-n-Bu | pyrimidin-2,5-diyl | 2-methylsulfonylphenyl |
| | 6 | H | $CH_2O$-i-Bu | pyrimidin-2,5-diyl | 2-methylsulfonylphenyl |
| | 7 | H | $CH_2Ph$ | pyrimidin-2,5-diyl | 2-methylsulfonylphenyl |

| | | | | | |
|---|---|---|---|---|---|
| | 8 | H | $CH_2$-pyrazol-1-yl | pyrimidin-2,5-diyl | 2-methylsulfonylphenyl |
| | 9 | H | $CH_2$-imidazol-1-yl | pyrimidin-2,5-diyl | 2-methylsulfonylphenyl |
| | 10 | H | $CH_2$-tetrazol-1-yl | pyrimidin-2,5-diyl | 2-methylsulfonylphenyl |
| | 11 | H | $CH_2$-tetrazol-2-yl | pyrimidin-2,5-diyl | 2-methylsulfonylphenyl |
| | 12 | H | $CH_2$-triazol-1-yl | pyrimidin-2,5-diyl | 2-methylsulfonylphenyl |
| | 13 | H | $CH_2SEt$ | pyrimidin-2,5-diyl | 2-methylsulfonylphenyl |
| | 14 | H | $CH_2SO_2Et$ | pyrimidin-2,5-diyl | 2-methylsulfonylphenyl |
| | 15 | H | $CF_3$ | pyrimidin-2,5-diyl | 2-methylsulfonylphenyl |
| | 16 | H | $CH_3$ | pyrimidin-2,5-diyl | 2-methylsulfonylphenyl |
| | 17 | H | H | pyrimidin-2,5-diyl | 2-methylsulfonylphenyl |
| H4 | 1 | H | $CH_2OMe$ | 2-fluoro-1,4-phenylene | 2-methylsulfonylphenyl |
| | 2 | H | $CH_2OEt$ | 2-fluoro-1,4-phenylene | 2-methylsulfonylphenyl |
| | 3 | H | $CH_2O$-n-Pr | 2-fluoro-1,4-phenylene | 2-methylsulfonylphenyl |
| | 4 | H | $CH_2O$-i-Pr | 2-fluoro-1,4-phenylene | 2-methylsulfonylphenyl |
| | 5 | H | $CH_2O$-n-Bu | 2-fluoro-1,4-phenylene | 2-methylsulfonylphenyl |
| | 6 | H | $CH_2O$-i-Bu | 2-fluoro-1,4-phenylene | 2-methylsulfonylphenyl |
| | 7 | H | $CH_2Ph$ | 2-fluoro-1,4-phenylene | 2-methylsulfonylphenyl |
| | 8 | H | $CH_2$-pyrazol-1-yl | 2-fluoro-1,4-phenylene | 2-methylsulfonylphenyl |

| | | | | | |
|---|---|---|---|---|---|
| | 9 | H | $CH_2$-imidazol-1-yl | 2-fluoro-1,4-phenylene | 2-methylsulfonylphenyl |
| | 10 | H | $CH_2$-tetrazol-1-yl | 2-fluoro-1,4-phenylene | 2-methylsulfonylphenyl |
| | 11 | H | $CH_2$-tetrazol-2-yl | 2-fluoro-1,4-phenylene | 2-methylsulfonylphenyl |
| | 12 | H | $CH_2$-triazol-1-yl | 2-fluoro-1,4-phenylene | 2-methylsulfonylphenyl |
| | 13 | H | $CH_2SEt$ | 2-fluoro-1,4-phenylene | 2-methylsulfonylphenyl |
| | 14 | H | $CH_2SO_2Et$ | 2-fluoro-1,4-phenylene | 2-methylsulfonylphenyl |
| | 15 | H | $CF_3$ | 2-fluoro-1,4-phenylene | 2-methylsulfonylphenyl |
| | 16 | H | $CH_3$ | 2-fluoro-1,4-phenylene | 2-methylsulfonylphenyl |
| | 17 | H | H | 2-fluoro-1,4-phenylene | 2-methylsulfonylphenyl |
| H5 | 1 | H | $CH_2OMe$ | 2-chloro-1,4-phenylene | 2-methylsulfonylphenyl |
| | 2 | H | $CH_2OEt$ | 2-chloro-1,4-phenylene | 2-methylsulfonylphenyl |
| | 3 | H | $CH_2O$-n-Pr | 2-chloro-1,4-phenylene | 2-methylsulfonylphenyl |
| | 4 | H | $CH_2O$-i-Pr | 2-chloro-1,4-phenylene | 2-methylsulfonylphenyl |
| | 5 | H | $CH_2O$-n-Bu | 2-chloro-1,4-phenylene | 2-methylsulfonylphenyl |
| | 6 | H | $CH_2O$-i-Bu | 2-chloro-1,4-phenylene | 2-methylsulfonylphenyl |
| | 7 | H | $CH_2Ph$ | 2-chloro-1,4-phenylene | 2-methylsulfonylphenyl |
| | 8 | H | $CH_2$-pyrazol-1-yl | 2-chloro-1,4-phenylene | 2-methylsulfonylphenyl |
| | 9 | H | $CH_2$-imidazol-1-yl | 2-chloro-1,4-phenylene | 2-methylsulfonylphenyl |

| | | | | | |
|---|---|---|---|---|---|
| | 10 | H | $CH_2$-tetrazol-1-yl | 2-chloro-1,4-phenylene | 2-methylsulfonylphenyl |
| | 11 | H | $CH_2$-tetrazol-2-yl | 2-chloro-1,4-phenylene | 2-methylsulfonylphenyl |
| | 12 | H | $CH_2$-triazol-1-yl | 2-chloro-1,4-phenylene | 2-methylsulfonylphenyl |
| | 13 | H | $CH_2SEt$ | 2-chloro-1,4-phenylene | 2-methylsulfonylphenyl |
| | 14 | H | $CH_2SO_2Et$ | 2-chloro-1,4-phenylene | 2-methylsulfonylphenyl |
| | 15 | H | $CF_3$ | 2-chloro-1,4-phenylene | 2-methylsulfonylphenyl |
| | 16 | H | $CH_3$ | 2-chloro-1,4-phenylene | 2-methylsulfonylphenyl |
| | 17 | H | H | 2-chloro-1,4-phenylene | 2-methylsulfonylphenyl |
| H6 | 1 | H | $CH_2OMe$ | 2-methyl-1,4-phenylene | 2-methylsulfonylphenyl |
| | 2 | H | $CH_2OEt$ | 2-methyl-1,4-phenylene | 2-methylsulfonylphenyl |
| | 3 | H | $CH_2O$-n-Pr | 2-methyl-1,4-phenylene | 2-methylsulfonylphenyl |
| | 4 | H | $CH_2O$-i-Pr | 2-methyl-1,4-phenylene | 2-methylsulfonylphenyl |
| | 5 | H | $CH_2O$-n-Bu | 2-methyl-1,4-phenylene | 2-methylsulfonylphenyl |
| | 6 | H | $CH_2O$-i-Bu | 2-methyl-1,4-phenylene | 2-methylsulfonylphenyl |
| | 7 | H | $CH_2Ph$ | 2-methyl-1,4-phenylene | 2-methylsulfonylphenyl |
| | 8 | H | $CH_2$-pyrazol-1-yl | 2-methyl-1,4-phenylene | 2-methylsulfonylphenyl |
| | 9 | H | $CH_2$-imidazol-1-yl | 2-methyl-1,4-phenylene | 2-methylsulfonylphenyl |
| | 10 | H | $CH_2$-tetrazol-1-yl | 2-methyl-1,4-phenylene | 2-methylsulfonylphenyl |

| | | | | |
|---|---|---|---|---|
| 11 | H | $CH_2$-tetrazol-2-yl | 2-methyl-1,4-phenylene | 2-methylsulfonylphenyl |
| 12 | H | $CH_2$-triazol-1-yl | 2-methyl-1,4-phenylene | 2-methylsulfonylphenyl |
| 13 | H | $CH_2SEt$ | 2-methyl-1,4-phenylene | 2-methylsulfonylphenyl |
| 14 | H | $CH_2SO_2Et$ | 2-methyl-1,4-phenylene | 2-methylsulfonylphenyl |
| 15 | H | $CF_3$ | 2-methyl-1,4-phenylene | 2-methylsulfonylphenyl |
| 16 | H | $CH_3$ | 2-methyl-1,4-phenylene | 2-methylsulfonylphenyl |
| 17 | H | H | 2-methyl-1,4-phenylene | 2-methylsulfonylphenyl |

**TABLE 8**

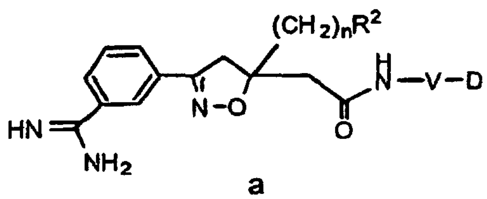

a

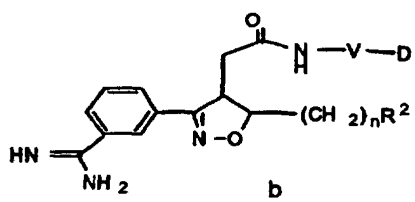

b

| Part | Cpd | $(CH_2)_nR^2$ | V | D |
|---|---|---|---|---|
| A | 1 | $CH_2OMe$ | phenyl | 2-aminosulfonylphenyl |
| | 2 | $CH_2OEt$ | phenyl | 2-aminosulfonylphenyl |

| | | | | |
|---|---|---|---|---|
| | 3 | $CH_2O$-n-Pr | phenyl | 2-aminosulfonylphenyl |
| | 4 | $CH_2O$-i-Pr | phenyl | 2-aminosulfonylphenyl |
| | 5 | $CH_2O$-n-Bu | phenyl | 2-aminosulfonylphenyl |
| | 6 | $CH_2O$-i-Bu | phenyl | 2-aminosulfonylphenyl |
| | 7 | $CH_2Ph$ | phenyl | 2-aminosulfonylphenyl |
| | 8 | $CH_2$-pyrazol-1-yl | phenyl | 2-aminosulfonylphenyl |
| | 9 | $CH_2$-imidazol-1-yl | phenyl | 2-aminosulfonylphenyl |
| | 10 | $CH_2$-tetrazol-1-yl | phenyl | 2-aminosulfonylphenyl |
| | 11 | $CH_2$-tetrazol-2-yl | phenyl | 2-aminosulfonylphenyl |
| | 12 | $CH_2$-triazol-1-yl | phenyl | 2-aminosulfonylphenyl |
| | 13 | $CH_2SEt$ | phenyl | 2-aminosulfonylphenyl |
| | 14 | $CH_2SO_2Et$ | phenyl | 2-aminosulfonylphenyl |
| | 15 | $CF_3$ | phenyl | 2-aminosulfonylphenyl |
| | 16 | $CH_3$ | phenyl | 2-aminosulfonylphenyl |
| | 17 | H | phenyl | 2-aminosulfonylphenyl |
| B | 1 | $CH_2OMe$ | pyridin-2,5-diyl | 2-aminosulfonylphenyl |
| | 2 | $CH_2OEt$ | pyridin-2,5-diyl | 2-aminosulfonylphenyl |
| | 3 | $CH_2O$-n-Pr | pyridin-2,5-diyl | 2-aminosulfonylphenyl |
| | 4 | $CH_2O$-i-Pr | pyridin-2,5-diyl | 2-aminosulfonylphenyl |
| | 5 | $CH_2O$-n-Bu | pyridin-2,5-diyl | 2-aminosulfonylphenyl |
| | 6 | $CH_2O$-i-Bu | pyridin-2,5-diyl | 2-aminosulfonylphenyl |
| | 7 | $CH_2Ph$ | pyridin-2,5-diyl | 2-aminosulfonylphenyl |
| | 8 | $CH_2$-pyrazol-1-yl | pyridin-2,5-diyl | 2-aminosulfonylphenyl |
| | 9 | $CH_2$-imidazol-1-yl | pyridin-2,5-diyl | 2-aminosulfonylphenyl |
| | 10 | $CH_2$-tetrazol-1-yl | pyridin-2,5-diyl | 2-aminosulfonylphenyl |
| | 11 | $CH_2$-tetrazol-2-yl | pyridin-2,5-diyl | 2-aminosulfonylphenyl |
| | 12 | $CH_2$-triazol-1-yl | pyridin-2,5-diyl | 2-aminosulfonylphenyl |
| | 13 | $CH_2SEt$ | pyridin-2,5-diyl | 2-aminosulfonylphenyl |
| | 14 | $CH_2SO_2Et$ | pyridin-2,5-diyl | 2-aminosulfonylphenyl |
| | 15 | $CF_3$ | pyridin-2,5-diyl | 2-aminosulfonylphenyl |

| | | | | |
|---|---|---|---|---|
| | 16 | $CH_3$ | pyridin-2,5-diyl | 2-aminosulfonylphenyl |
| | 17 | H | pyridin-2,5-diyl | 2-aminosulfonylphenyl |
| C | 1 | $CH_2OMe$ | pyrimidin-2,5-diyl | 2-aminosulfonylphenyl |
| | 2 | $CH_2OEt$ | pyrimidin-2,5-diyl | 2-aminosulfonylphenyl |
| | 3 | $CH_2O$-n-Pr | pyrimidin-2,5-diyl | 2-aminosulfonylphenyl |
| | 4 | $CH_2O$-i-Pr | pyrimidin-2,5-diyl | 2-aminosulfonylphenyl |
| | 5 | $CH_2O$-n-Bu | pyrimidin-2,5-diyl | 2-aminosulfonylphenyl |
| | 6 | $CH_2O$-i-Bu | pyrimidin-2,5-diyl | 2-aminosulfonylphenyl |
| | 7 | $CH_2Ph$ | pyrimidin-2,5-diyl | 2-aminosulfonylphenyl |
| | 8 | $CH_2$-pyrazol-1-yl | pyrimidin-2,5-diyl | 2-aminosulfonylphenyl |
| | 9 | $CH_2$-imidazol-1-yl | pyrimidin-2,5-diyl | 2-aminosulfonylphenyl |
| | 10 | $CH_2$-tetrazol-1-yl | pyrimidin-2,5-diyl | 2-aminosulfonylphenyl |
| | 11 | $CH_2$-tetrazol-2-yl | pyrimidin-2,5-diyl | 2-aminosulfonylphenyl |
| | 12 | $CH_2$-triazol-1-yl | pyrimidin-2,5-diyl | 2-aminosulfonylphenyl |
| | 13 | $CH_2SEt$ | pyrimidin-2,5-diyl | 2-aminosulfonylphenyl |
| | 14 | $CH_2SO_2Et$ | pyrimidin-2,5-diyl | 2-aminosulfonylphenyl |
| | 15 | $CF_3$ | pyrimidin-2,5-diyl | 2-aminosulfonylphenyl |
| | 16 | $CH_3$ | pyrimidin-2,5-diyl | 2-aminosulfonylphenyl |
| | 17 | H | pyrimidin-2,5-diyl | 2-aminosulfonylphenyl |

## TABLE 12

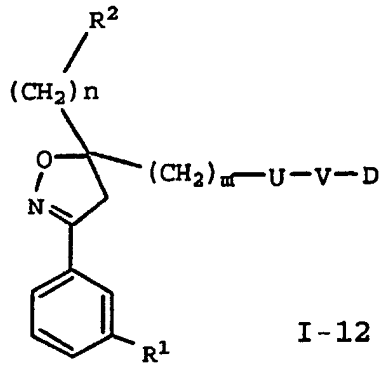

I-12

| Cpd.# | $R^1$ | n | m | $R^2$ | -U-V-D |
|---|---|---|---|---|---|
| 1 | 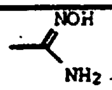 | 1 | 0 | $CO_2Me$ | 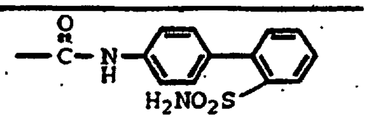 |
| 2 | 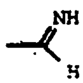 | 1 | 0 | $CO_2Me$ | 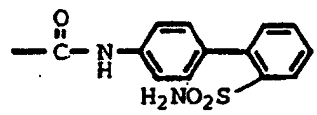 |
| 3 | 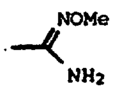 | 1 | 0 | $CO_2Me$ | 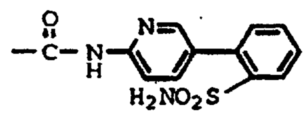 |
| 4 | 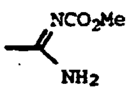 | 1 | 0 | $CO_2Me$ | 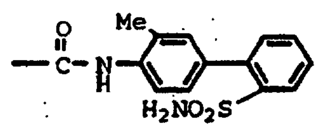 |
| 5 | 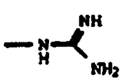 | 1 | 0 | $CO_2Me$ | 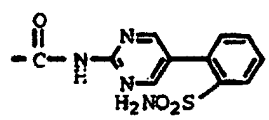 |
| 6 | 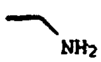 | 1 | 0 | $CO_2Me$ | 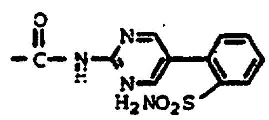 |

TABLE 13

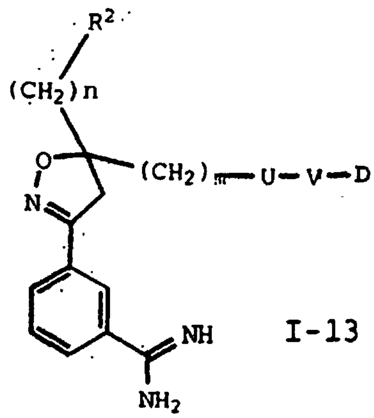

I-13

| Cpd. # | n | m | $R^2$ | -U-V-D |
|---|---|---|---|---|
| 1 | 1 | 0 | CO2Me | 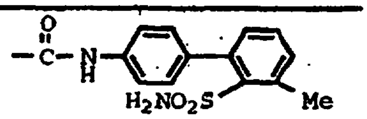 |
| 2 | 1 | 0 | CO2Me | 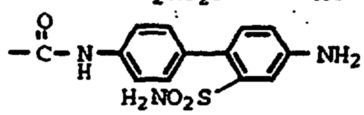 |
| 3 | 1 | 0 | CO2Me | 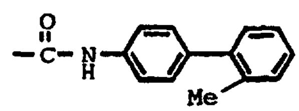 |
| 4 | 1 | 0 | CO2Me | 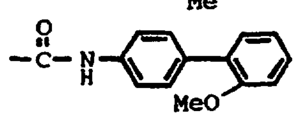 |
| 5 | 1 | 0 | CO2Me | 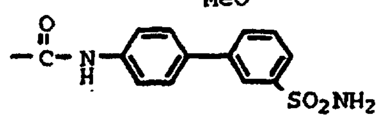 |
| 6 | 1 | 0 | CO2Me | 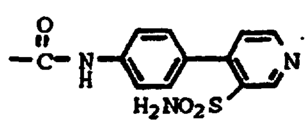 |
| 7 | 1 | 0 | CO2Me | 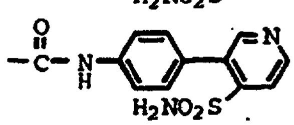 |
| 8 | 1 | 0 | CO2Me | 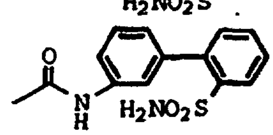 |
| 9 | 1 | 0 | CO2Me | 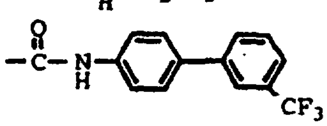 |

| | | | | |
|---|---|---|---|---|
| 14 | 1 | 0 | $CO_2Me$ | 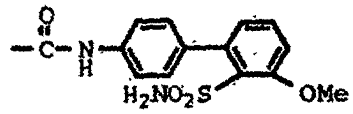 |
| 15 | 1 | 0 | $CO_2Me$ | 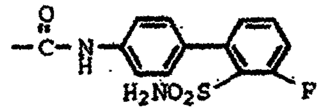 |
| 16 | 1 | 0 | $CO_2Me$ | 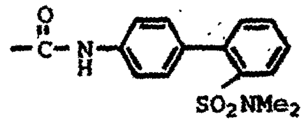 |
| 17 | 1 | 0 | $CO_2Me$ | 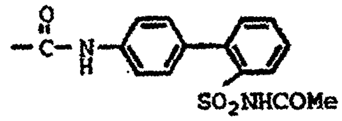 |
| 18 | 1 | 0 | $CO_2Me$ | 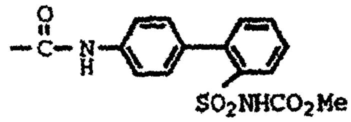 |
| 19 | 1 | 0 | $CO_2Me$ | 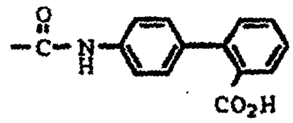 |
| 20 | 1 | 0 | $CO_2Me$ | 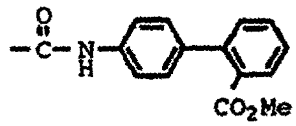 |
| 21 | 1 | 0 | $CO_2Me$ | 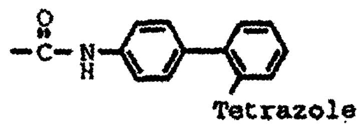 |
| 22 | 1 | 0 | $CO_2Me$ | 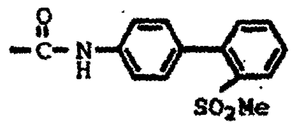 |
| 23 | 1 | 0 | $CO_2Me$ | 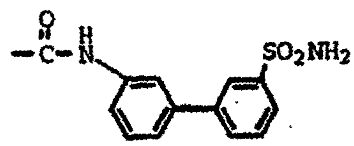 |

| | | | | |
|---|---|---|---|---|
| 24 | 1 | 0 | $CO_2Me$ | 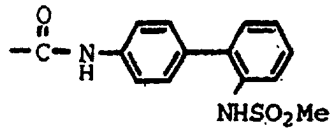 |
| 25 | 1 | 0 | $CO_2Me$ | 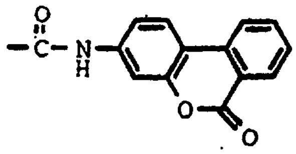 |
| 26 | 1 | 0 | $CO_2Me$ | 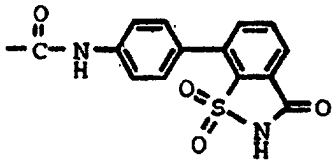 |
| 27 | 1 | 0 | $CO_2Me$ | 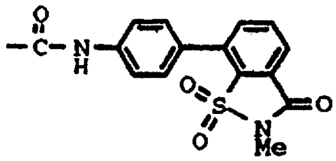 |

TABLE 14

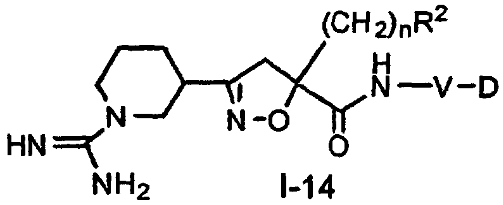

I-14

| Cpd | $(CH_2)_nR^2$ | V | -D |
|---|---|---|---|
| 1 | $CH_2OMe$ | phenyl | 2-aminosulfonylphenyl |
| 2 | $CH_2OEt$ | phenyl | 2-aminosulfonylphenyl |
| 3 | $CH_2O$-n-Pr | phenyl | 2-aminosulfonylphenyl |
| 4 | $CH_2O$-i-Pr | phenyl | 2-aminosulfonylphenyl |
| 5 | $CH_2O$-n-Bu | phenyl | 2-aminosulfonylphenyl |
| 6 | $CH_2O$-i-Bu | phenyl | 2-aminosulfonylphenyl |
| 7 | $CH_2Ph$ | phenyl | 2-aminosulfonylphenyl |
| 8 | $CH_2$-pyrazol-1-yl | phenyl | 2-aminosulfonylphenyl |
| 9 | $CH_2$-imidazol-1-yl | phenyl | 2-aminosulfonylphenyl |
| 10 | $CH_2$-tetrazol-1-yl | phenyl | 2-aminosulfonylphenyl |
| 11 | $CH_2$-tetrazol-2-yl | phenyl | 2-aminosulfonylphenyl |
| 12 | $CH_2$-triazol-1-yl | phenyl | 2-aminosulfonylphenyl |
| 13 | $CH_2SEt$ | phenyl | 2-aminosulfonylphenyl |
| 14 | $CH_2SO_2Et$ | phenyl | 2-aminosulfonylphenyl |
| 15 | $CF_3$ | phenyl | 2-aminosulfonylphenyl |
| 16 | $CH_3$ | phenyl | 2-aminosulfonylphenyl |
| 17 | H | phenyl | 2-aminosulfonylphenyl |

**TABLE 15**

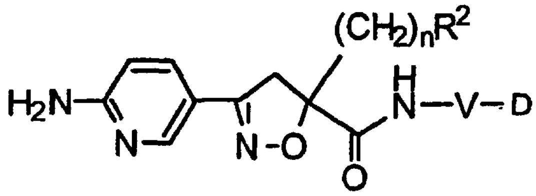

I-15

| Cpd | $(CH_2)_nR^2$ | V | -D |
|---|---|---|---|
| 1 | $CH_2OMe$ | phenyl | 2-aminosulfonylphenyl |
| 2 | $CH_2OEt$ | phenyl | 2-aminosulfonylphenyl |
| 3 | $CH_2O$-n-Pr | phenyl | 2-aminosulfonylphenyl |
| 4 | $CH_2O$-i-Pr | phenyl | 2-aminosulfonylphenyl |
| 5 | $CH_2O$-n-Bu | phenyl | 2-aminosulfonylphenyl |
| 6 | $CH_2O$-i-Bu | phenyl | 2-aminosulfonylphenyl |
| 7 | $CH_2Ph$ | phenyl | 2-aminosulfonylphenyl |
| 8 | $CH_2$-pyrazol-1-yl | phenyl | 2-aminosulfonylphenyl |
| 9 | $CH_2$-imidazol-1-yl | phenyl | 2-aminosulfonylphenyl |
| 10 | $CH_2$-tetrazol-1-yl | phenyl | 2-aminosulfonylphenyl |
| 11 | $CH_2$-tetrazol-2-yl | phenyl | 2-aminosulfonylphenyl |
| 12 | $CH_2$-triazol-1-yl | phenyl | 2-aminosulfonylphenyl |
| 13 | $CH_2SEt$ | phenyl | 2-aminosulfonylphenyl |
| 14 | $CH_2SO_2Et$ | phenyl | 2-aminosulfonylphenyl |
| 15 | $CF_3$ | phenyl | 2-aminosulfonylphenyl |
| 16 | $CH_3$ | phenyl | 2-aminosulfonylphenyl |
| 17 | H | phenyl | 2-aminosulfonylphenyl |

## TABLE 16

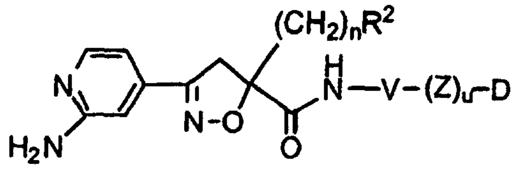

I-16

| Cpd | $(CH_2)_nR^2$ | V | -D |
|---|---|---|---|
| 1 | $CH_2OMe$ | phenyl | 2-aminosulfonylphenyl |
| 2 | $CH_2OEt$ | phenyl | 2-aminosulfonylphenyl |
| 3 | $CH_2O$-n-Pr | phenyl | 2-aminosulfonylphenyl |
| 4 | $CH_2O$-i-Pr | phenyl | 2-aminosulfonylphenyl |
| 5 | $CH_2O$-n-Bu | phenyl | 2-aminosulfonylphenyl |
| 6 | $CH_2O$-i-Bu | phenyl | 2-aminosulfonylphenyl |
| 7 | $CH_2Ph$ | phenyl | 2-aminosulfonylphenyl |
| 8 | $CH_2$-pyrazol-1-yl | phenyl | 2-aminosulfonylphenyl |
| 9 | $CH_2$-imidazol-1-yl | phenyl | 2-aminosulfonylphenyl |
| 10 | $CH_2$-tetrazol-1-yl | phenyl | 2-aminosulfonylphenyl |
| 11 | $CH_2$-tetrazol-2-yl | phenyl | 2-aminosulfonylphenyl |
| 12 | $CH_2$-triazol-1-yl | phenyl | 2-aminosulfonylphenyl |
| 13 | $CH_2SEt$ | phenyl | 2-aminosulfonylphenyl |
| 14 | $CH_2SO_2Et$ | phenyl | 2-aminosulfonylphenyl |
| 15 | $CF_3$ | phenyl | 2-aminosulfonylphenyl |
| 16 | $CH_3$ | phenyl | 2-aminosulfonylphenyl |
| 17 | H | phenyl | 2-aminosulfonylphenyl |

**TABLE 17**

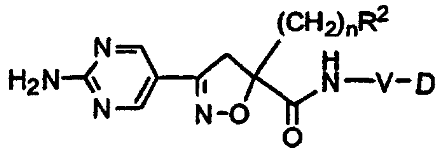

I-17

| Cpd | $(CH_2)_nR^2$ | V | -D |
|---|---|---|---|
| 1 | $CH_2OMe$ | phenyl | 2-aminosulfonylphenyl |
| 2 | $CH_2OEt$ | phenyl | 2-aminosulfonylphenyl |
| 3 | $CH_2O$-n-Pr | phenyl | 2-aminosulfonylphenyl |
| 4 | $CH_2O$-i-Pr | phenyl | 2-aminosulfonylphenyl |
| 5 | $CH_2O$-n-Bu | phenyl | 2-aminosulfonylphenyl |
| 6 | $CH_2O$-i-Bu | phenyl | 2-aminosulfonylphenyl |
| 7 | $CH_2Ph$ | phenyl | 2-aminosulfonylphenyl |
| 8 | $CH_2$-pyrazol-1-yl | phenyl | 2-aminosulfonylphenyl |
| 9 | $CH_2$-imidazol-1-yl | phenyl | 2-aminosulfonylphenyl |
| 10 | $CH_2$-tetrazol-1-yl | phenyl | 2-aminosulfonylphenyl |
| 11 | $CH_2$-tetrazol-2-yl | phenyl | 2-aminosulfonylphenyl |
| 12 | $CH_2$-triazol-1-yl | phenyl | 2-aminosulfonylphenyl |
| 13 | $CH_2SEt$ | phenyl | 2-aminosulfonylphenyl |
| 14 | $CH_2SO_2Et$ | phenyl | 2-aminosulfonylphenyl |
| 15 | $CF_3$ | phenyl | 2-aminosulfonylphenyl |
| 16 | $CH_3$ | phenyl | 2-aminosulfonylphenyl |
| 17 | H | phenyl | 2-aminosulfonylphenyl |

**TABLE 18**

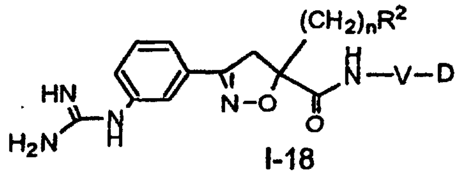

I-18

| Cpd | $(CH_2)_nR^2$ | V | -D |
|---|---|---|---|
| 1 | $CH_2OMe$ | phenyl | 2-aminosulfonylphenyl |
| 2 | $CH_2OEt$ | phenyl | 2-aminosulfonylphenyl |
| 3 | $CH_2O$-n-Pr | phenyl | 2-aminosulfonylphenyl |
| 4 | $CH_2O$-i-Pr | phenyl | 2-aminosulfonylphenyl |
| 5 | $CH_2O$-n-Bu | phenyl | 2-aminosulfonylphenyl |
| 6 | $CH_2O$-i-Bu | phenyl | 2-aminosulfonylphenyl |
| 7 | $CH_2Ph$ | phenyl | 2-aminosulfonylphenyl |
| 8 | $CH_2$-pyrazol-1-yl | phenyl | 2-aminosulfonylphenyl |
| 9 | $CH_2$-imidazol-1-yl | phenyl | 2-aminosulfonylphenyl |
| 10 | $CH_2$-tetrazol-1-yl | phenyl | 2-aminosulfonylphenyl |
| 11 | $CH_2$-tetrazol-2-yl | phenyl | 2-aminosulfonylphenyl |
| 12 | $CH_2$-triazol-1-yl | phenyl | 2-aminosulfonylphenyl |
| 13 | $CH_2SEt$ | phenyl | 2-aminosulfonylphenyl |
| 14 | $CH_2SO_2Et$ | phenyl | 2-aminosulfonylphenyl |
| 15 | $CF_3$ | phenyl | 2-aminosulfonylphenyl |
| 16 | $CH_3$ | phenyl | 2-aminosulfonylphenyl |
| 17 | H | phenyl | 2-aminosulfonylphenyl |

Utility

The compounds of this invention are useful as anticoagulants for the treatment or prevention of thromboembolic disorders in mammals. The term "thromboembolic disorders" as used herein includes arterial or venous cardiovascular or cerebrovascular thromboembolic disorders, including, for example, unstable angina, first or recurrent myocardial infarction, ischemic sudden death, transient ischemic attack, stroke, atherosclerosis, venous thrombosis, deep vein thrombosis, thrombophlebitis, arterial embolism, coronary and cerebral arterial thrombosis, cerebral embolism, kidney embolisms, pulmonary embolisms.

The anticoagulant effect of compounds of this invention is due to inhibition of Factor Xa. The activated factor Xa, whose major practical role is the generation of thrombin by the limited proteolysis of prothrombin, holds a central position that links the intrinsic and extrinsic activation mechanisms in the final common pathway of blood coagulation. The generation of thrombin, the final serine protease in the pathway to generate a fibrin clot, from its precursor is amplified by formation of prothrombinase complex (Factor Xa, Factor V, $Ca^{2+}$ and phospholipid). Since it is calculated

that one molecule of Factor Xa can generate 138 molecules of thrombin (*Elodi, S., Varadi, K.: Optimization of conditions for the catalytic effect of the factor IXa-factor VIII C0omplex: Probable role of the complex in the amplification of blood coagulation. Thromb. Res. 1979, 15, 617-629*), inhibition of factor Xa may be more efficient that inactivation of thrombin in interrupting the blood coagulation system.

The effectiveness of the compounds of the invention as inhibitors of Factor Xa was determined using purified human Factor Xa and synthetic substrate. The rate of Factor Xa hydrolysis of chromogenic substrate S2222 (Kabi Pharmacia, Franklin, OH) was measured both in the absence and presence of compounds of the present invention. Hydrolysis of the substrate resulted in the release of pNA, which was monitored spectrophotometrically by measuring the increase in absorbance at 405 nM. A decrease in the rate of absorbance change at 405 nm in the presence of inhibitor is indicative of enzyme inhibition. The results of this assay are expressed as inhibitory constant, $K_i$.

Factor Xa determinations were made in 0.10 M sodium phosphate buffer, pH 7.5, containing 0.20 M NaCl, and 0.5 % PEG 8000. The Michaelis constant, $K_m$, for substrate hydrolysis was determined at 25 °C using the method of Lineweaver and Burk.

Values of $K_i$ were determined by allowing 0.2 - 0.5 nM human Factor Xa (Enzyme Research Laboratories, South Bend, IN) to react with the substrate (0.20 mM - 1 mM) in the presence of inhibitor. Reactions were allowed to go for 30 minutes and the velocities (rate of absorbance change vs time) were measured in the time frame of 25-30 minutes. The following relationship was used to calculate $K_i$ values.

$$\frac{v_o - v_s}{v_s} = \frac{I}{K_i\,(1 + S/K_m)}$$

where:

- $v_o$ is the velocity of the control in the absence of inhibitor;
- $v_s$ is the velocity in the presence of inhibitor;
- I is the concentration of inhibitor;
- $K_i$ is the dissociation constant of the enzyme: inhibitor complex;
- S is the concentration of substrate;
- $K_m$ is the Michaelis constant.

The antithrombotic effect of the compounds of this invention can be demonstrated in a rat vena cava thrombosis model. In this model Male Sprague-Dawley rats weighing 350-450 grams anesthetized with a mixture of xylazine (10 mg/kg i.m.) and ketamine (110 mg/kg i.m.) are used. A carotid artery, a jugular vein and a femoral vein are cannulated for blood sampling, drug infusion and hypotonic saline injection, respectively. The abdominal vena cava is isolated and all its side-branches are ligated beneath the left renal vein. Thrombus formation is induced by rapid injection of 1 ml hypotonic saline (0.225%) into the vena cava. This is followed 15 seconds later by a 15-minute stasis of an isolated segment (about 1 cm) of the vena cava. The formed thrombus in the vena cava is removed and immediately weighed.

Test compounds or vehicle are given as continuous intravenous infusions or orally starting 1 hour before the injection of hypotonic saline. Arterial blood samples (1.5 ml) for the determination of clotting times are collected before and 1 hour after the infusion or oral dosing of test compounds or vehicle. The percentage inhibition of thrombus formation is determined for each treatment group. The ID50 values (dose which produces 50% inhibition of thrombus formation) are estimated by linear regression.

The compounds of this invention can be administered alone or in combination with one or more additional therapeutic agents. These include other anti-coagulant or coagulation inhibitory agents, anti-platelet or platelet inhibitory agents, thrombin inhibitors, or thrombolytic or fibrinolytic agents.

The compounds are administered to a mammal in a therapeutically effective amount. By "therapeutically effective amount" is meant an amount of a compound of Formula I that, when administered alone or in combination with an additional therapeutic agent to a mammal, is effective to prevent or ameliorate the thromboembolic disease condition or the progression of the disease.

By "administered in combination" or "combination therapy" is meant that the compound of Formula I and one or more additional therapeutic agents are administered concurrently to the mammal being treated. When administered in combination each component may be administered at the same time or sequentially in any order at different points in time. Thus, each component may be administered separately but sufficiently closely in time so as to provide the desired therapeutic effect.

Other anticoagulant agents (or coagulation inhibitory agents) that may be used in combination with the compounds of this invention include warfarin and heparin, as well as other Factor Xa inhibitors such as those described in the publications identified above under Background of the Invention.

The term anti-platelet agents (or platelet inhibitory agents), as used herein, denotes agents that inhibit platelet function such as by inhibiting the aggregation, adhesion or granular secretion of platelets. Such agents include the various known non-steroidal antiinflammatory drugs (NSAIDS) such as aspirin, ibuprofen, naproxen, sulindac, indomethacin, mefenamate, droxicam, diclofenac, sulfinpyrazone, and piroxicam, including pharmaceutically acceptable salts or prodrugs thereof. Of the NSAIDS, aspirin (acetylsalicyclic acid or ASA), and piroxicam. Other suitable antiplatelet agents include ticlopidine, including pharmaceutically acceptable salts or prodrugs thereof. Ticlopidine is also a preferred compound since it is known to be gentle on the gastro-intestinal tract in use. Still other suitable platelet inhibitory agents include thromboxane-A2-receptor antagonists and thromboxane-A2-synthetase inhibitors, as well as pharmaceutically acceptable salts or prodrugs thereof.

The term thrombin inhibitors (or anti-thrombin agents), as used herein, denotes inhibitors of the serine protease thrombin. By inhibiting thrombin, various thrombin-mediated processes, such as thrombin-mediated platelet activation (that is, for example, the aggregation of platelets, and/or the granular secretion of plasminogen activator inhibitor-1 and/or serotonin) and/or fibrin formation are disrupted. Such inhibitors include boroarginine derivatives and boropeptides, hirudin and argatroban, including pharmaceutically acceptable salts and prodrugs thereof. Boroarginine derivatives and boropeptides include N-acetyl and peptide derivatives of boronic acid, such as C-terminal a-aminoboronic acid derivatives of lysine, ornithine, arginine, homoarginine and corresponding isothiouronium analogs thereof. The term hirudin, as used herein, includes suitable derivatives or analogs of hirudin, referred to herein as hirulogs, such as disulfatohirudin. Boropeptide thrombin inhibitors include compounds described in Kettner et al., U.S. Patent No. 5,187,157 and European Patent Application Publication Number 293 881 A2. Other suitable boroarginine derivatives and boropeptide thrombin inhibitors include those disclosed in PCT Application Publication Number 92/07869 and European Patent Application Publication Number 471 651 A2.

The term thrombolytics (or fibrinolytic) agents (or thrombolytics or fibrinolytics), as used herein, denotes agents that lyse blood clots (thrombi). Such agents include tissue plasminogen activator, anistreplase, urokinase or streptokinase, including pharmaceutically acceptable salts or prodrugs thereof. The term anistreplase, as used herein, refers to anisoylated plasminogen streptokinase activator complex, as described, for example, in European Patent Application No. 028,489. The term urokinase, as used herein, is intended to denote both dual and single chain urokinase, the latter also being referred to herein as prourokinase.

Administration of the compounds of Formula I of the invention in combination with such.additional therapeutic agent, may afford an efficacy advantage over the compounds and agents alone, and may do so while permitting the use of lower doses of each. A lower dosage minimizes the potential of side effects, thereby providing an increased margin of safety.

The compounds of the present invention are also useful as standard or reference compounds, for example as a quality standard or control, in tests or assays involving the inhibition of Factor Xa. Such compounds may be provided in a commercial kit, for example, for use in pharmaceutical research involving Factor Xa. The compounds of the present invention may also be used in diagnostic assays involving Factor Xa.

Dosage and Formulation

The compounds of this invention can be administered in such oral dosage forms as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular form, all using dosage forms well known to those of ordinary skill in the pharmaceutical arts. They can be administered alone, but generally will be administered with a pharmaceutical carrier selected on the basis of the chosen route of administration and standard pharmaceutical practice.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind pf concurrent treatment; the frequency of treatment; the route of administration, the renal and hepatic function of the patient,and the effect desired. A physician or veterinarian can determine and prescribe the effective amount of the drug required to prevent, counter, or arrest the progress of the thromboembolic disorder.

By way of general guidance, the daily oral dosage of each active ingredient, when used for the indicated effects, will range between about 0.001 to 1000 mg/kg of body weight, preferably between about 0.01 to 100 mg/kg of body weight per day, and most preferably between about 1.0 to 20 mg/kg/day. Intravenously, the most preferred doses will range from about 1 to about 10 mg/kg/minute during a constant rate infusion. Compounds of this invention may be administered in a single daily dose, or the total daily dosage may be administered in divided doses of two, three, or four times daily.

Compounds of this invention can be administered in intranasal form via topical use of suitable intranasal vehicles, or via transdermal routes, using transdermal skin patches. When administered in the form of a transdermal

delivery system, the dosage administration will, of course, be continuous rather than intermittent throughout the dosage regimen.

The compounds are typically administered in admixture with suitable pharmaceutical diluents, excipients, or carriers (collectively referred to herein as pharmaceutical carriers) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl callulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

The compounds of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

Compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxide-polylysine substituted with palmitoyl residues. Furthermore, the compounds of the present invention may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacylates, and crosslinked or amphipathic block copolymers of hydrogels.

Dosage forms (pharmaceutical compositions) suitable for administration may contain from about 1 milligram to about 100 milligrams of active ingredient per dosage unit. In these pharmaceutical compositions the active ingredient will ordinarily be present in an amount of about 0.5-95% by weight based on the total weight of the composition.

Gelatin capsules may contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol.

Suitable pharmaceutical carriers are described in Remington's Pharmaceutical Sciences, Mack Publishing Company, a standard reference text in this field.

Representative useful pharmaceutical dosage-forms for administration of the compounds of this invention can be illustrated as follows:

Capsules

A large number of unit capsules are prepared by filling standard two-piece hard gelatin capsules each with 100 milligrams of powdered active ingredient, 150 milligrams of lactose, 50 milligrams of cellulose, and 6 milligrams magnesium stearate.

Soft Gelatin Capsules

A mixture of active ingredient in a digestable oil such as soybean oil, cottonseed oil or olive oil is prepared and injected by means of a positive displacement pump into gelatin to form soft gelatin capsules containing 100 milligrams of the active ingredient. The capsules are washed and dried.

Tablets

A large number of tablets are prepared by conventional procedures so that the dosage unit is 100 milligrams of active ingredient, 0.2 milligrams of colloidal silicon dioxide, 5 milligrams of magnesium stearate, 275 milligrams of microcrystalline cellulose, 11 milligrams of starch and 98.8 milligrams of lactose. Appropriate coatings may be applied to increase palatability or delay absorption.

Injectable

A parenteral composition suitable for administration by injection is prepared by stirring 1.5% by weight of active ingredient in 10% by volume propylene glycol and water. The solution is made isotonic with sodium chloride and sterilized.

Suspension

An aqueous suspension is prepared for oral administration so that each 5 mL contain 100 mg of finely divided active ingredient, 200 mg of sodium carboxymethyl cellulose, 5 mg of sodium benzoate, 1.0 g of sorbitol solution, U.S.P., and 0.025 mL of vanillin.

Where the compounds of this invention are combined with other anticoagulant agents, for example, a daily dosage may be about 0.1 to 100 milligrams of the compound of Formula I and about 1 to 7.5 milligrams of the second anticoagulant, per kilogram of patient body weight. For a tablet dosage form, the compounds of this invention generally may be present in an amount of about 5 to 10 milligrams per dosage unit, and the second anti-coagulant in an amount of about 1 to 5 milligrams per dosage unit.

Where the compounds of Formula I are administered in combination with an anti-platelet agent, by way of general guidance, typically a daily dosage may be about 0.01 to 25 milligrams of the compound of Formula I and about 50 to 150 milligrams of the anti-platelet agent, preferably about 0.1 to 1 milligrams of the compound of Formula I and about 1 to 3 milligrams of antiplatelet agents, per kilogram of patient body weight.

Where the compounds of Formula I are adminstered in combination with thrombolytic agent, typically a daily dosage may be about 0.1 to 1 milligrams of the compound of Formula I, per kilogram of patient body weight and, in the case of the thrombolytic agents, the usual dosage of the thrombolyic agent when administered alone may be reduced by about 70-80% when administered with a compound of Formula I.

Where two or more of the foregoing second therapeutic agents are administered with the compound of Formula I, generally the amount of each component in a typical daily dosage and typical dosage form may be reduced relative to the usual dosage of the agent when administered alone, in view of the additive or synergistic effect of the therapeutic agents when administered in combination.

Particularly when provided as a single dosage unit, the potential exists for a chemical interaction between the combined active ingredients. For this reason, when the compound of Formula I and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material which effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a lowviscosity grade of hydroxypropyl methylcellulose (HPMC) or other appropriate materials as known in the art, in order to further separate the active components. The polymer coating serves to form an additional barrier to interaction with the other component.

## Claims

**1.** Compounds of Formula (V) and (VI):

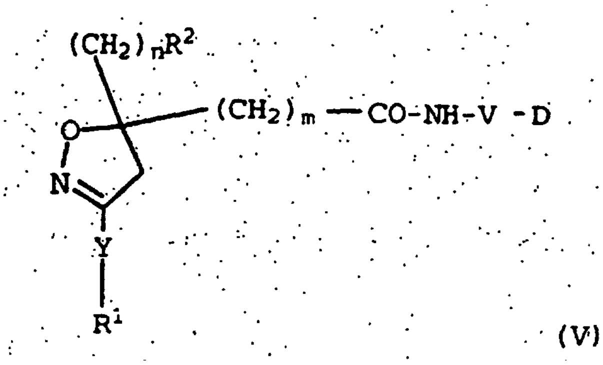

(V)

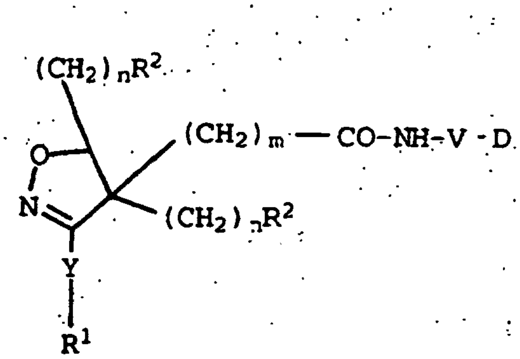

(VI)

including pharmaceutically acceptable salts
thereof, and all stereoisomeric forms thereof and mixtures of such stereoisomeric forms, wherein:

$R^1$ is C $(NR^{14})NR^5R^6$ and

D is selected from

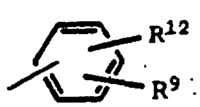

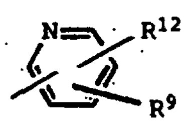

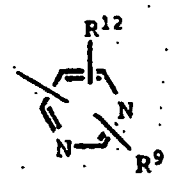

V is selected from the following:

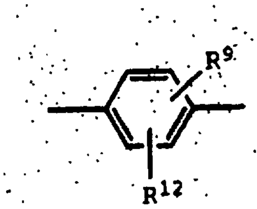

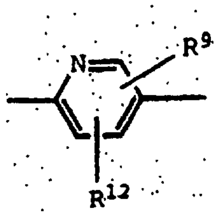

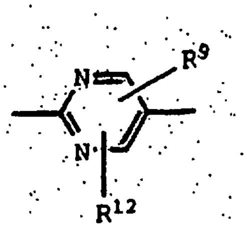

Y is selected from

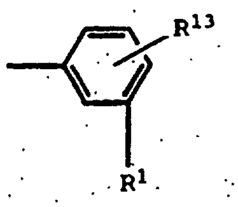
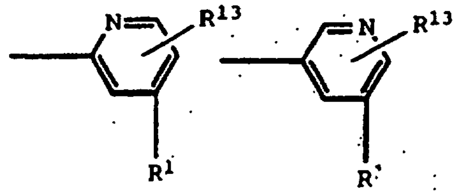
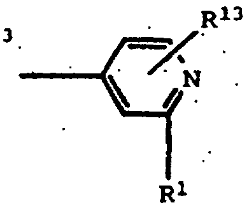

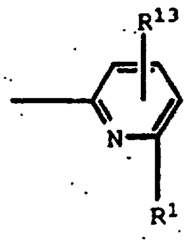
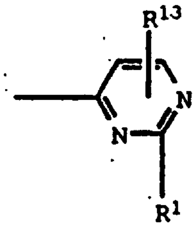
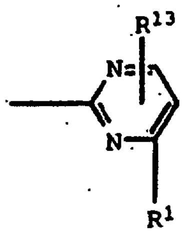

$R^2$ is selected from
- H
- $C_1$-$C_6$ alkyl
- $C_1$-$C_6$ alkoxy
- $CO_2R^5$
- $CONHR^5$
- $CONHCH_2CO_2R^5$
- $CONH(CH_2)_q$-$R^{10}$
- $R^{10}$
- $CO$-$R^5$
- $COCO_2R^5$
- $COCONHR^5$
- $SO_nR^5$
- $SO_2NHR^5$
- $NHR^7$
- $CH{=}CHCO_2R^5$
- $CH{=}CHCONHR^5$
- $O$-$(CH_2)_n$-$R^{10}$
- $SO_n$-$(CH_2)_n$-$R^{10}$
- $NH$-$(CH_2)_n$-$R^{10}$

$R^5$ and $R^6$ at each appearance are independently
- H
- $C_1$-$C_6$ alkyl
- $(CH_2)_n$-phenyl

$R^7$ is selected from
- H
- $C_1$-$C_6$ alkyl
- $SO_2R^5$
- $COR^5$
- $(CH_2)_r$-$R^{10}$
- $(CH_2)_n$-phenyl

$R^8$ is selected from
- H
- $C_1$-$C_6$ alkyl
- halogen

$NO_2$
$CF_3$
$OR^5$

R9 is selected from
- H
- $C_1$-$C_6$ alkyl
- halogen
- $NO_2$
- $NHR^7$
- $SO_2NHR^{11}$
- $CF_3$
- $OR^5$
- $CO_2R^5$
- $CONR^5R^7$
- CN
- $(CH_2)_pNR^5R^6$
- $C(NR^{14})NR^5R^6$
- $NHC(NR^{14})NR^5R^6$
- $NHC(NR^{14})H$
- $SO_n$-$R^5$
- $SO_n$-$CF_3$

imidazole, pyrazole, 1,2,3-triazole, 1,2,4-triazole
and tetrazole, each optionally substituted with $CF_3$, halogen, $NO_2$, $C_1$-$C_5$ alkyl, or $C_1$-$C_5$ alkoxy;

$R^{10}$ is selected from

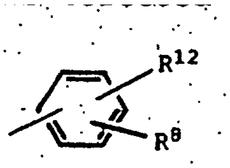 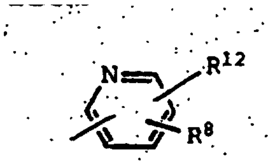 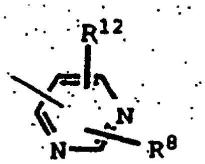 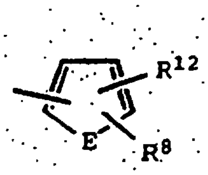

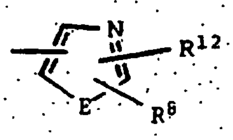 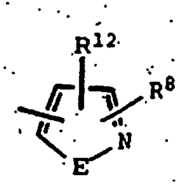 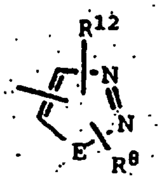 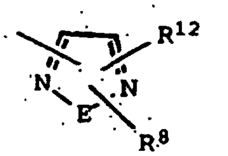

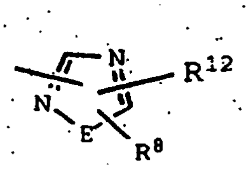 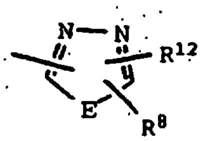 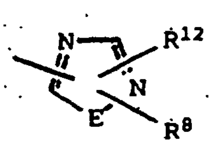 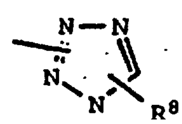

$R^{11}$ is selected from
- H
- $C_1$-$C_6$ alkyl
- $(CH_2)_n$-phenyl
- $COR^5$
- $CO_2R^5$

$R^{12}$ is selected from

H
$C_1$-$C_6$ alkyl
$C_1$-$C_6$ alkoxy
halogen
$NO_2$
$NHR^7$
CN
$CF_3$
$SONHR^{11}$

$R^{13}$ is selected from
H
OH
$C_1$-$C_{10}$ alkyl
$C_1$-$C_{10}$ alkoxy
nitro
halo
$CF_3$

$R^{14}$ is selected from
H
OH
$C_1$-$C_{10}$ alkyl
$C_1$-$C_{10}$ alkoxy
$CO_2$-$C_1$-$C_{10}$ alkyl
CO-$C_1$-$C_{10}$ alkyl
CONH-$C_1$-$C_{10}$ alkyl
CONH-phenyl
$CO_2(CH_2)_n$-phenyl;
at each appearance each of the following are independently:
m = 0-2
n = 0-4, except that in $-SO_n-$, n = 0-2;
p = 0-1
q = 0-4
r = 1-2.

**2.** A compound of claim 1 selected from the group consisting of the following compounds, including pharmaceutically acceptable salt forms thereof, and all stereoisomeric forms thereof and mixtures of such stereoisomeric forms:

3-(3-amidinophenyl)-5-[[(2'-aminosulfonyl-[1,1']-biphenyl-4-yl)-methyl]aminocarbonyl]-5-(carbomethoxymethyl)isoxazoline

3-(3-amidinophenyl)-5-[[(2'-aminosulfonyl-[1,1']-biphenyl-4-yl)methyl]aminocarbonyl]-5-(aminocarbonylmethyl)isoxazoline

3-(3-amidinophenyl)-5-[([1,1']-biphenyl-4-yl)aminocarbonyl]-5-(carbomethoxymethyl)isoxazoline

3-(3-amidinophenyl)-5-[([1,1']-biphenyl-4-yl)aminocarbonyl]-5-(carboxymethyl)isoxazoline

3-(3-amidinophenyl)-5-[([1,1']-biphenyl-4-yl)aminocarbonyl]-5-(aminocarbonylmethyl)isoxazoline

3-(3-amidinophenyl)-5-[([1,1']-biphenyl-4-yl)aminocarbonyl]-5-(hydroxymethyl)isoxazoline

3-(3-amidinophenyl)-5-[(2'-aminosulfonyl-3'-n-propyl-[1,1']-biphenyl-4-yl)aminocarbonyl]-5-(carbomethoxymethyl)isoxazoline

3-(3-amidinophenyl)-5-[(2'-t-butylaminosulfonyl-[1,1']-biphenyl-4-yl)aminocarbonyl]-5-(carbomethoxymethyl) isoxazoline

3-(3-amidinophenyl)-5-[(2'-t-butylaminosulfonyl-[1,1']-biphenyl-4-yl)aminocarbonyl]-5-(aminocarbonylmethyl) isoxazoline

3-(3-amidinophenyl)-5-[(4'-amino-[1,1']-biphenyl-4-yl)aminocarbonyl]-5-(carbomethoxymethyl)isoxazoline

3- (3-amidinophenyl)-5-[(2'-trifluoromethyl-[1,1']-biphenyl-4-yl)aminocarbonyl]-5-(carbomethoxymethyl)isoxazoline

3-(3-amidinophenyl)-5-[(2'-aminosulfonyl-3-methyl-[1,1']-biphenyl-4-yl)aminocarbonyl)]-5-(carbomethoxyethyl)isoxazoline

3-(3-amidinophenyl)-5-[(2'-aminosulfonyl-3-methyl-[1,1']-biphenyl-4-yl)aminocarbonyl]-5-(carboxyethyl)isoxazoline

3-(3-amidinophenyl)-5-[(2-aminosulfonyl-3-methyl-[1,1']-biphenyl-4-yl)aminocarbonyl]-5-(carbomethoxyethylene)isoxazoline

3-(3-amidinophenyl)-5-[[5-(2'-aminosulfonylphenyl-1-yl)pyrimidin-2-yl]aminocarbonyl]-5-(aminocarbonylmethyl)isoxazoline

3-(3-amidinophenyl)-5-[[5-(2'-aminosulfonylphenyl-1-yl)pyrimidin-2-yl]aminocarbonyl]-5-(hydroxyethyl)isoxazoline

3-(3-amidinophenyl)-5-[[5-(2'-aminosulfonylphenyl-1-yl)pyrimidin-2-yl]aminocarbonyl]-5-(methoxyethyl)isoxazoline

3-(3-amidinophenyl)-5-[[5-(2'-aminosulfonylphenyl-1-yl)pyrimidin-2-yl]aminocarbonyl]-5-(methyl)isoxazoline

3-(3-amidinophenyl)-5-[(2'-aminosulfonyl-[1,1']-biphenyl-4-yl)aminocarbonyl]-5-(carbomethoxymethyl)isoxazoline

3-(3-amidinophenyl)-5-[(2'-aminosulfonyl-[1,1']-biphenyl-4-yl)aminocarbonyl]-5-(carboxymethyl)isoxazoline

3-(3-amidinophenyl)-5- [(2'-aminosulfonyl-[1,1']-biphenyl-4-yl)aminocarbonyl]-5-(aminocarbonylmethyl)isoxazoline

3-(3-amidinophenyl)-5-[(2'-aminosulfonyl-[1,1']-biphenyl-4-yl)aminocarbonyl)-5-(hydroxyethyl)isoxazoline

3-(3-amidinophenyl)-5-[(2'-aminosulfonyl-[1,1']-biphenyl-4-yl)aminocarbonyl]-5-(carbomethoxymethylaminocarbonylmethyl)isoxazoline

3-(3-amidinophenyl)-5-[(2'-aminosulfonyl-[1,1']-biphenyl-4-yl)aminocarbonyl]-5-[(imidazole-4-yl)ethylaminocarbonylmethyl]isoxazoline

3-(3-amidinophenyl)-5-[(2'-aminosulfonyl-[1,1']-biphenyl-4-yl)aminocarbonyl]-5-(methoxyethyl)isoxazoline

3-(3-amidinophenyl)-5-[(2'-aminosulfonyl-[1,1']-biphenyl-4-yl)aminocarbonyl]-5-(methyl)isoxazoline

3-(3-amidinophenyl)-5-[(2'-aminosulfonyl-3-methyl-[1,1']-biphenyl-4-yl)aminocarbonyl]-5-(carbomethoxymethyl)isoxazoline

3-(3-amidinophenyl)-5-[(2'-aminosulfonyl-3-methyl-[1,1']-biphenyl-4-yl)aminocarbonyl]-5-(carboxymethyl)isoxazoline

3-(3-amidinophenyl)-5-[(2'-aminosulfonyl-3-methyl-[1,1']-biphenyl-4-yl)aminocarbonyl]-5-(aminocarbonylmethyl)isoxazoline

3-(3-amidinophenyl)-5-[(2'-aminosulfonyl-3-methyl[1,1']-biphenyl-4-yl)aminocarbonyl]-5-(carbomethoxymethylaminocarbonylmethyl)isoxazoline

3-(3-amidinophenyl)-5-[(2'-aminosulfonyl-3-methyl-[1,1']-biphenyl-4-yl)aminocarbonyl]-5-(hydroxyethyl)isoxazoline

3-(3-amidinophenyl)-5-[(2'-aminosulfonyl-3-methyl-[1,1']-biphenyl-4-yl)aminocarbonyl]-5-(methoxyethyl)isoxazoline

3-(3-amidinophenyl)-5-[(2'-aminosulfonyl-3-methyl-[1,1']-biphenyl-4-yl)aminocarbonyl]-5-(methyl)isoxazoline

3-(3-amidinophenyl)-5-[(2'-aminosulfonyl-3-fluoro-[1,1']-biphenyl-4-yl)aminocarbonyl]-5-(carbomethoxymethyl)isoxazoline

3-(3-amidinophenyl)-5-[(2'-aminosulfonyl-3-fluoro-[1,1']-biphenyl-4-yl)aminocarbonyl]-5-(carboxymethyl)isoxazoline

3-(3-amidinophenyl)-5-[(2'-aminosulfonyl-3-fluoro-[1,1']-biphenyl-4-yl)aminocarbonyl]-5-(aminocarbonylmethyl) isoxazoline

3-(3-amidinophenyl)-5-[(2'-aminosulfonyl-3-fluoro-[1,1'] biphenyl-4-yl)aminocarbonyl]-5-(hydroxyethyl)isoxazoline

3-(3-amidinophenyl)-5-[(2'-aminosulfonyl-3-fluoro-[1,1'] biphenyl-4-yl)aminocarbonyl]-5-(methoxyethyl)isoxazoline

3-(3-amidinophenyl)-5-[(2'-aminosulfonyl-3-fluoro-[1,1']-biphenyl-4-yl)aminocarbonyl]-5-(methyl)isoxazoline

3-(3-amidinophenyl)-5-[[2-(2'-aminosulfonylphenyl-1-yl)pyridin-5-yl]aminocarbonyl]-5-(carbomethoxymethyl) isoxazoline

3-(3-amidinophenyl)-5-[[2-(2'-aminosulfonylphenyl-1-yl)pyridin-5-yl]aminocarbonyl]-5-(carboxymethyl)isoxazoline

3-(3-amidinophenyl)-5-[[2-(2'-aminosulfonylphenyl-1-yl)pyridin-5-yl]aminocarbonyl]-5-(aminocarbonylmethyl) isoxazoline

3-(3-amidinophenyl)-5-[[2-(2'-aminosulfonylphenyl-1-yl)pyridin-5-yl]aminocarbonyl]-5-(hydroxyethyl)isoxazoline

3-(3-amidinophenyl)-5-[[2-(2'-aminosulfonylphenyl-1-yl)pyridin-5-yl]aminocarbonyl]-5-(methoxyethyl)isoxazoline

3-(3-amidinophenyl)-5-[[2-(2'-aminosulfonylphenyl-1-yl)pyridin-5-yl]aminocarbonyl]-5-(methyl) isoxazoline

3-(3-amidinophenyl)-5-[[5-(2'-aminosulfonylphenyl-1-yl)pyridin-2-yl]aminocarbonyl]-5-(carbomethoxymethyl) isoxazoline

3-(3-amidinophenyl)-5-[[5-(2'-aminosulfonylphenyl-1-yl)pyridin-2-yl]aminocarbonyl]-5-(carboxymethyl)isoxazoline

3-(3-amidinophenyl)-5-[[5-(2'-aminosulfonylphenyl-1-yl)pyridin-2-yl]aminocarbonyl]-5-(aminocarbonylmethyl) isoxazoline

3-(3-amidinophenyl)-5-[[5-(2'-aminosulfonylphenyl-1-yl)pyridin-2-yl]aminocarbonyl]-5-(hydroxyethyl)isoxazoline

3-(3-amidinophenyl)-5-[[5-(2'-aminosulfonylphenyl-1-yl)pyridin-2-yl]aminocarbonyl]-5-(methoxyethyl)isoxazoline

3-(3-amidinophenyl)-5-[[5-(2'-aminosulfonylphenyl-1-yl)pyridin-2-yl]aminocarbonyl]-5-(methyl)isoxazoline

3-(3-amidinophenyl)-5-[[5-(2'-aminosulfonylphenyl-1-yl)pyrimidin-2-yl]aminocarbonyl]-5-(carbomethoxymethyl)isoxazoline

3-(3-amidinophenyl)-5-[[5-(2'-aminosulfonylphenyl-1-yl)pyrimidin-2-yl]aminocarbonyl]-5-(carboxymethyl)isoxazoline

3-(3-amidinophenyl)-5-[5-(2'-aminosulfonylphenyl-1-yl)pyrimidin-2-yl]aminocarbonyl-5-carbomethoxymethyl-isoxazoline

3-(3-amidinophenyl)-5-[5-(2'-aminosulfonylphenyl-1-yl)pyridin-2-yl]aminocarbonyl-5-carbomethoxymethyl-isoxazoline

3-(3-amidinophenyl)-5-[2'-aminosulfonyl-[1,1']-biphenyl-4-yl]aminocarbonyl-5-(tetrazol-1-yl)methyl-isoxazoline

3-(3-amidinophenyl)-5-[5-(2'-aminosulfonylphenyl-1-yl)pyridin-2-yl]aminocarbonyl-5-(tetrazol-1-yl)methyl-isoxazoline

3-(3-amidinophenyl)-5-[5-(2'-aminosulfonylphenyl-1-yl)pyrimidin-2-yl]aminocarbonyl-5-(tetrazol-1-yl)methyl-isoxazoline

3-(3-amidinophenyl)-5-[2'-trifluoromethyl-[1,1']-biphenyl-4-yl]aminocarbonyl-5-(tetrazol-1-yl)methyl-isoxazoline

3-(3-amidinophenyl)-5-[5-(2'-trifluoromethylphenyl-1-yl)pyridin-2-yl]aminocarbonyl-5-(tetrazol-1-yl)methyl-isoxazoline

3-(3-amidinophenyl)-5-[5-(2'-trifluoromethylphenyl-1-yl)pyrimidin-2-yl]aminocarbonyl-5-(tetrazol-1-yl)methyl-isoxazoline

3-(3-amidinophenyl)-5-[5-(2'-trifluoromethylsulfonylphenyl-1-yl)pyridin-2-yl]aminocarbonyl-5-(tetrazol-1-yl) methyl-isoxazoline

3-(3-amidinophenyl)-5-[5-(2'-trifluoromethylsulfonylphenyl-1-yl)pyrimidin-2-yl]aminocarbonyl-5-(tetrazol-1-yl) methyl-isoxazoline

3-(3-amidinophenyl)-5-[2-aminosulfonyl-3-flouro-[1,1']-biphenyl-4-yl]aminocarbonyl-5-(tetrazol-1-yl)methyl-isoxazoline

3- (3-amidinophenyl)-5-[2'-aminosulfonyl-3-chloro-[1,1']-biphenyl-4-yl]aminocarbonyl-5-(tetrazol-1-yl)methyl-isoxazoline

3-(3-amidinophenyl)-5-[2'-trifluoromethyl-3-flouro-[1,1']-biphenyl-4-yl]aminocarbonyl-5-(tetrazol-1-yl)methyl-isoxazoline

3-(3-amidinophenyl)-5-[2'-trifluoromethyl-3-chloro-[1,1']-biphenyl-4-yl]aminocarbonyl-5-(tetrazol-1-yl)methyl-isoxazoline

3-(3-amidinophenyl)-5-[5-(2'-aminosulfonylphenyl-1-yl)pyridin-2-yl]aminocarbonyl-5-methoxymethyl-isoxazoline

3-(3-amidinophenyl)-5-[2'-methylaminosulfonyl-[1,1']-biphenyl-4-yl]aminocarbonyl-5-(tetrazol-1-yl)methyl-isoxazoline

3-(3-amidinophenyl)-5-[5-(2'-methylaminosulfonylphenyl-1-yl)pyridin-2-yl]aminocarbonyl-5-(tetrazol-1-yl)methyl-isoxazoline

3-(3-amidinophenyl)-5-[2'-methylsulfonyl-[1,1']-biphenyl-4-yl]aminocarbonyl-5-(tetrazol-1-yl)methyl-isoxazoline

3-(3-amidinophenyl)-5-[2'-methylsulfonyl-fluoro-[1,1']-biphenyl-4-yl]aminocarbonyl-5-(tetrazol-1-yl)methyl-isoxazoline

3-(3-amidinophenyl)-5-[2'-methylsulfonyl-chloro-[1,1']-biphenyl-4-yl]aminocarbonyl-5-(tetrazol-1-yl)methyl-isoxazoline

3-(3-amidinophenyl)-5-[2'-trifluoromethylsulfonyl-[1,1']-biphenyl-4-yl]aminocarbonyl-5-(tetrazol-1-yl)methyl-isoxazoline

3-(3-amidinophenyl)-5-[2'-aminosulfonyl-[1,1']-biphenyl-4-yl)aminocarbonyl-5-(imidazol-1-yl)methyl-isoxazoline

3-(3-amidinophenyl)-5-[2'-trifluoromethylsulfonyl-3-fluoro-[1,1']-biphenyl-4-yl]aminocarbonyl-5-(tetrazol-1-yl)methyl-isoxazoline

3-(3-amidinophenyl)-5-[2'-trifluoromethylsulfonyl-3-chloro-[1,1']-biphenyl-4-yl]aminocarbonyl-5-(tetrazol-1-yl)methyl-isoxazoline

3-(3-amidinophenyl)-5-[2'-aminosulfonyl-[1,1']-biphenyl-4-yl]aminocarbonyl-5-(imidazol-1-yl)methyl-isoxazoline

3-(3-amidinophenyl)-4-(2'-aminosulfonyl-[1,1']-biphenyl-4-yl)aminocarbonyl-5-methoxymethyl-isoxazoline

3-(3-amidinophenyl)-4-(2'-aminosulfonyl-[1,1']-biphenyl-4-yl)aminocarbonyl-5-trifluoromethyl-isoxazoline

3-(3-amidinophenyl)-5-(2'-aminosulfonyl-[1,1']-biphenyl-4-yl)aminocarbonyl-4-methoxymethyl-isoxazoline

**3.** Pharmaceutical composition comprising a therapeutically effective amount of a compound of either claim. 1 or claim 2 and a pharmaceutically acceptable carrier.

**4.** A compound of either claim 1 or claim 2 for use in treating or preventing a thromboembolic disorder in a mammal.

**5.** Use of a compound of either claim 1 or claim 2. in the manufacture of a medicament for treating or preventing 4 thromboembolic disorder in a mammal.

## Patentansprüche

**1.** Verbindungen der Formel (V) und (VI):

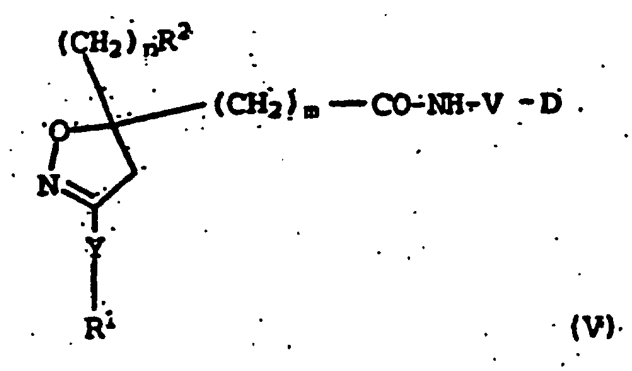

(V).

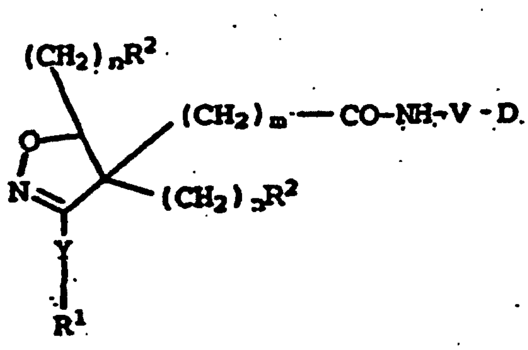

(VI)

einschließlich pharmazeutisch akzeptabler Salze davon und aller stereoisomeren Formen davon und Mischungen solcher stereoisomerer Formen, wobei:

$R^1$ $C(NR^{14})NR^5R^6$ ist und

D ausgewählt ist unter

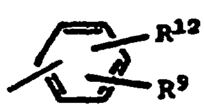 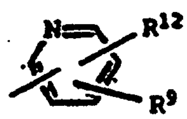 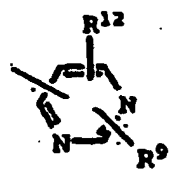

V ausgewählt ist unter den folgenden Gruppen:

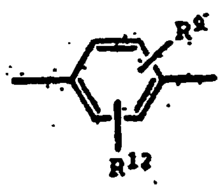 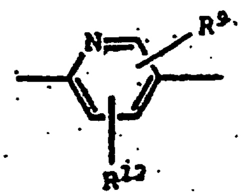 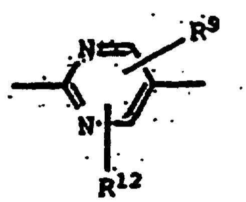

Y ausgewählt ist unter

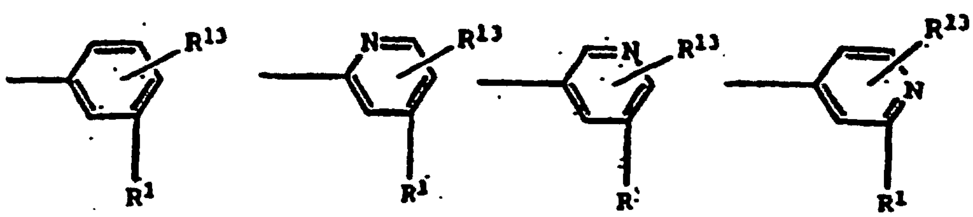

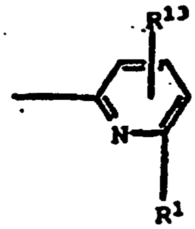

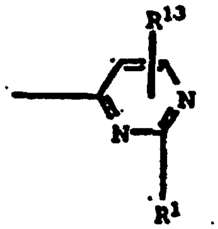

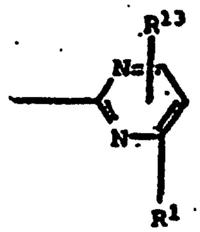

$R^2$ ausgewählt ist unter
- H
- $C_1$-$C_6$-Alkyl
- $C_1$-$C_6$-Alkoxy
- $CO_2R^5$
- $CONHR^5$
- $CONHCH_2CO_2R^5$
- $CONH(CH_2)_q$-$R^{10}$
- $R^{10}$
- CO-$R^5$
- $COCO_2R^5$
- $COCONHR^5$
- $SO_nR^5$
- $SO_2NHR^5$
- $NHR^7$
- $CH{=}CHCO_2R^5$
- $CH{=}CHCONHR^5$
- O-$(CH_2)_n$-$R^{10}$
- $SO_n(CH_2)_n$-$R^{10}$
- NH-$(CH_2)_n$-$R^{10}$

$R^5$ und $R^6$ bei jeder Erwähnung unabhängig voneinander
- H
- $C_1$-$C_6$-Alkyl
- $(CH_2)_n$-Phenyl

sind,

$R^7$ ausgewählt ist unter
- H
- $C_1$-$C_6$-Alkyl
- $SO_2R^5$
- $COR^5$
- $(CH_2)_r$-$R^{10}$
- $(CH_2)_n$-Phenyl,

$R^8$ ausgewählt ist unter
- H
- $C_1$-$C_6$-Alkyl
- Halogen
- $NO_2$
- $CF_3$
- $OR^5$,

$R^9$ ausgewählt ist unter
- H
- $C_1$-$C_6$-Alkyl
- Halogen
- $NO_2$

$NHR^7$
$SO_2NHR^{11}$
$CF_3$
$OR^5$
$CO_2R^5$
$CONR^5R^7$
CN
$(CH_2)_pNR^5R^6$
$C(NR^{14})NR^5R^6$
$NHC(NR^{14})NR^5R^6$
$NHC(NR^{14})H$
$SO_n$-$R^5$
$SO_n$-$CF_3$

Imidazol, Pyrazol, 1,2,3-Triazol, 1,2,4-Triazol und Tetrazol, wobei jede der Gruppen wahlweise mit $CF_3$, Halogen, $NO_2$, $C_1$-$C_5$-Alkyl oder $C_1$-$C_5$-Alkoxy substituiert ist;

$R^{10}$ ausgewählt ist unter

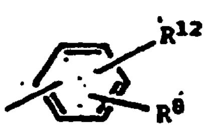 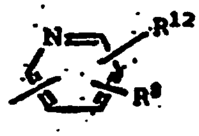 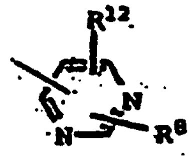 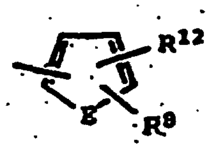

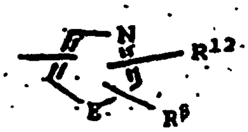 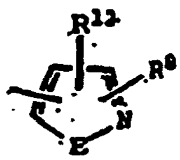 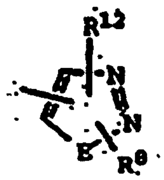 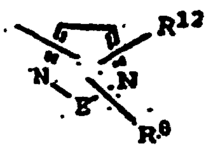

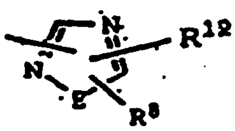 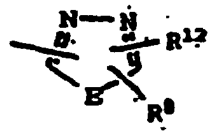 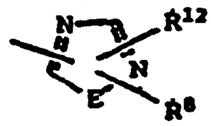 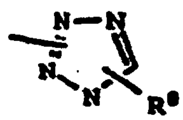

$R^{11}$ ausgewählt ist unter

H
$C_1$-$C_6$-Alkyl
$(CH_2)_n$-Phenyl
$COR^5$
$CO_2R^5$,

$R^{12}$ ausgewählt ist unter

H
$C_1$-$C_6$-Alkyl
$C_1$-$C_6$-Alkoxy
Halogen
$NO_2$
$NHR^7$
CN

$CF_3$
$SONHR^{11}$,

$R^{13}$ ausgewählt ist unter
H
OH
$C_1$-$C_{10}$-Alkyl
$C_1$-$C_{10}$-Alkoxy
Nitro
Halo
$CF_3$,

$R^{14}$ ausgewählt ist unter
H
OH
$C_1$-$C_{10}$-Alkyl
$C_1$-$C_{10}$-Alkoxy
$CO_2$-$C_1$-$C_{10}$-Alkyl
CO-$C_1$-$C_{10}$-Alkyl
CONH-$C_1$-$C_{10}$-Alkyl
CONH-Phenyl
$CO_2(CH_2)_n$-Phenyl;

wobei bei jeder Erwähnung jeder der nachfolgenden Indices unabhängig voneinander
m = 0-2
n = 0-4, außer dass bei $-SO_n-$ n = 0-2 ist;
p = 0-1
q = 0-4
r = 1-2
ist.

**2.** Verbindung nach Anspruch 1, ausgewählt unter folgenden Verbindungen, einschließlich pharmazeutisch akzeptabler Salzformen davon, und aller stereoisomeren Formen und Mischungen solcher stereoisomerer Formen davon:

3-(3-Amidinophenyl)-5-[[(2'-aminosulfonyl-[1,1']-biphenyl-4-yl)-methyl]aminocarbonyl]-5-(carbomethoxymethyl)isoxazolin

3-(3-Amidinophenyl)-5-[[(2'-aminosulfonyl-[1,1']-biphenyl-4-yl)methyl]aminocarbonyl]-5-(aminocarbonylmethyl)isoxazolin

3-(3-Amidinophenyl)-5-[([1,1']-biphenyl-4-yl)aminocarbonyl]-5-(carbomethoxymethyl)isoxazolin

3-(3-Amidinophenyl)-5-[([1,1']-biphenyl-4-yl)aminocarbonyl]-5-(carboxymethyl)isoxazolin

3-(3-Amidinophenyl)-5-[([1,1']-biphenyl-4-yl)aminocarbonyl]-5-(aminocarbonylmethyl)isoxazolin

3-(3-Amidinophenyl)-5-[([1,1']-biphenyl-4-yl)aminocarbonyl]-5-(hydroxymethyl)isoxazolin

3-(3-Amidinophenyl)-5-[(2'-aminosulfonyl-3'-n-propyl-[1,1']-biphenyl-4-yl)aminocarbonyl]-5-(carbomethoxymethyl)isoxazolin

3-(3-Amidinophenyl)-5-[(2'-t-butylaminosulfonyl-[1,1']-biphenyl-4-yl)aminocarbonyl]-5-(carbomethoxymethyl) isoxazolin

3-(3-Amidinophenyl)-5-[(2'-t-butylaminosulfonyl-[1,1']-biphenyl-4-yl)aminocarbonyl]-5-(aminocarbonylmethyl)isoxazolin

3-(3-Amidinophenyl)-5-[(4'-amino-[1,1']-biphenyl-4-yl)aminocarbonyl]-5-(carbomethoxymethyl)isoxazolin

3-(3-Amidinophenyl)-5-[(2'-trifluormethyl-[1,1']-biphenyl-4-yl)aminocarbonyl]-5-(carbomethoxymethyl)isoxazolin

3-(3-Amidinophenyl)-5-[(2'-aminosulfonyl-3-methyl-[1,1']-biphenyl-4-yl)aminocarbonyl]-5-(carbomethoxyethyl)isoxazolin

3-(3-Amidinophenyl)-5-[(2'-aminosulfonyl-3-methyl-[1,1']-biphenyl-4-yl)aminocarbonyl]-5-(carboxyethyl) isoxazolin

3-(3-Amidinophenyl)-5-[(2'-aminosulfonyl-3-methyl-[1,1']-biphenyl-4-yl)aminocarbonyl]-5-(carbomethoxyethylen)isoxazolin

3-(3-Amidinophenyl)-5-[[5-(2'-aminosulfonylphenyl-1-yl)pyrimidin-2-yl]aminocarbonyl]-5-(aminocarbonylmethyl)isoxazolin

3-(3-Amidinophenyl)-5-[[5-(2'-aminosulfonylphenyl-1-yl)pyrimidin-2-yl]aminocarbonyl]-5-(hydroxyethyl)isoxazolin

3-(3-Amidinophenyl)-5-[[5-(2'-aminosulfonylphenyl-1-yl)pyrimidin-2-yl]aminocarbonyl]-5-(methoxyethyl) isoxazolin

3-(3-Amidinophenyl)-5-[[5-(2'-aminosulfonylphenyl-1-yl)pyrimidin-2-yl]aminocarbonyl]-5-(methyl)isoxazolin

3-(3-Amidinophenyl)-5-[(2'-aminosulfonyl-[1,1']-biphenyl-4-yl)aminocarbonyl]-5-(carbomethoxymethyl)isoxazolin

3-(3-Amidinophenyl)-5-[(2'-aminosulfonyl-[1,1']-biphenyl-4-yl)aminocarbonyl]-5-(carboxymethyl)isoxazolin

3-(3-Amidinophenyl)-5-[(2'-aminosulfonyl-[1,1']-biphenyl-4-yl)aminocarbonyl]-5-(aminocarbonylmethyl)isoxazolin

3-(3-Amidinophenyl)-5-[(2'-aminosulfonyl-[1,1']-biphenyl-4-yl)aminocarbonyl]-5-(hydroxyethyl)isoxazolin

3-(3-Amidinophenyl)-5-[(2'-aminosulfonyl-[1,1']-biphenyl-4-yl)aminocarbonyl]-5-(carbomethoxymethylaminocarbonylmethyl)isoxazolin

3-(3-Amidinophenyl)-5-[(2'-aminosulfonyl-[1,1']-biphenyl-4-yl)aminocarbonyl]-5-[(imidazol-4-yl)ethylaminocarbonylmethyl]isoxazolin

3-(3-Amidinophenyl)-5-[(2'-aminosulfonyl-[1,1']-biphenyl-4-yl)aminocarbonyl]-5-(methoxyethyl)isoxazolin

3-(3-Amidinophenyl)-5-[(2'-aminosulfonyl-[1,1']-biphenyl-4-yl)aminocarbonyl]-5-(methyl)isoxazolin

3-(3-Amidinophenyl)-5-[(2'-aminosulfonyl-3-methyl-[1,1']-biphenyl-4-yl)aminocarbonyl]-5-(carbomethoxymethyl)isoxazolin

3-(3-Amidinophenyl)-5-[(2'-aminosulfonyl-3-methyl-[1,1']-biphenyl-4-yl)aminocarbonyl]-5-(carboxymethyl) isoxazolin

3-(3-Amidinophenyl)-5-[(2'-aminosulfonyl-3-methyl-[1,1']-biphenyl-4-yl)aminocarbonyl]-5-(aminocarbonylmethyl)isoxazolin

3-(3-Amidinophenyl)-5-[(2'-aminosulfonyl-3-methyl-[1,1']-biphenyl-4-yl)aminocarbonyl]-5-(carbomethoxymethylaminocarbonylmethyl)isoxazolin

3-(3-Amidinophenyl)-5-[(2'-aminosulfonyl-3-methyl-[1,1']-biphenyl-4-yl)aminocarbonyl]-5-(hydroxyethyl) isoxazolin

3-(3-Amidinophenyl)-5-[(2'-aminosulfonyl-3-methyl-[1,1']-biphenyl-4-yl)aminocarbonyl]-5-(methoxyethyl) isoxazolin

3-(3-Amidinophenyl)-5-[(2'-aminosulfonyl-3-methyl-[1,1']-biphenyl-4-yl)aminocarbonyl]-5-(methyl)isoxazolin

3-(3-Amidinophenyl)-5-[(2'-aminosulfonyl-3-fluor-[1,1']-biphenyl-4-yl)aminocarbonyl]-5-(carbomethoxymethyl)isoxazolin

3-(3-Amidinophenyl)-5-[(2'-aminosulfonyl-3-fluor-[1,1']-biphenyl-4-yl)aminocarbonyl]-5-(carboxymethyl) isoxazolin

3-(3-Amidinophenyl)-5-[(2'-aminosulfonyl-3-fluor-[1,1']-biphenyl-4-yl)aminocarbonyl]-5-(aminocarbonylmethyl)isoxazolin

3-(3-Amidinophenyl)-5-[(2'-aminosulfonyl-3-fluor-[1,1']-biphenyl-4-yl)aminocarbonyl]-5-(hydroxyethyl)isoxazolin

3-(3-Amidinophenyl)-5-[(2'-aminosulfonyl-3-fluor-[1,1']-biphenyl-4-yl)aminocarbonyl]-5-(methaxyethyl)isoxazolin

3-(3-Amidinophenyl)-5-[(2'-aminosulfonyl-3-fluor-[1,1']-biphenyl-4-yl)aminocarbonyl]-5-(methyl)isoxazolin

3-(3-Amidinophenyl)-5-[[2-(2'-aminosulfonylphenyl-1-yl)pyridin-5-yl]aminocarbonyl]-5-(carbomethoxymethyl) isoxazolin

3-(3-Amidinophenyl)-5-[[2-(2'-aminosulfonylphenyl-1-yl)pyridin-5-yl]aminocarbonyl]-5-(carboxymethyl)isoxazolin

3-(3-Amidinophenyl)-5-[[2-(2'-aminosulfonylphenyl-1-yl)pyridin-5-yl]aminocarbonyl]-5-(aminocarbonylmethyl)isoxazolin

3-(3-Amidinophenyl)-5-[[2-(2'-aminosulfonylphenyl-1-yl)pyridin-5-yl]aminocarbonyl]-5-(hydroxyethyl)isoxazolin

3-(3-Amidinophenyl)-5-[[2-(2'-aminosulfonylphenyl-1-yl)pyridin-5-yl]aminocarbonyl]-5-(methoxyethyl)isoxazolin

3-(3-Amidinophenyl)-5-[[2-(2'-aminosulfonylphenyl-1-yl)pyridin-5-yl]aminocarbonyl]-5-(methyl)isoxazolin

3-(3-Amidinophenyl)-5-[[5-(2'-aminosulfonylphenyl-1-yl)pyridin-2-yl]aminocarbonyl]-5-(carbomethoxymethyl) isoxazolin

3-(3-Amidinophenyl)-5-[[5-(2'-aminosulfonylphenyl-1-yl)pyridin-2-yl]aminocarbonyl]-5-(carboxymethyl)isoxazolin

3-(3-Amidinophenyl)-5-[[5-(2'-aminosulfonylphenyl-1-yl)pyridin-2-yl]aminocarbonyl]-5-(aminocarbonylmethyl)isoxazolin

3-(3-Amidinophenyl)-5-[[5-(2'-aminosulfonylphenyl-1-yl)pyridin-2-yl]aminocarbonyl]-5-(hydroxyethyl)isoxazolin

3-(3-Amidinophenyl)-5-[[5-(2'-aminosulfonylphenyl-1-yl)pyridin-2-yl]aminocarbonyl]-5-(methoxyethyl)isoxazolin

3-(3-Amidinophenyl)-5-[[5-(2'-aminosulfonylphenyl-1-yl)pyridin-2-yl]aminocarbonyl]-5-(methyl)isoxazolin

3-(3-Amidinophenyl)-5-[[5-(2'-aminosulfonylphenyl-1-yl)pyrimidin-2-yl]aminocarbonyl]-5-(carbomethoxymethyl)isoxazolin

3-(3-Amidinophenyl)-5-[[5-(2'-aminosulfonylphenyl-1-yl)pyrimidin-2-yl]aminocarbonyl]-5-(carboxymethyl) isoxazolin

3-(3-Amidinophenyl)-5-[5-(2'-aminosulfonylphenyl-1-yl)pyrimidin-2-yl]aminocarbonyl-5-carbomethoxymethyl-isoxazolin

3-(3-Amidinophenyl)-5-[5-(2'-aminosulfonylphenyl-1-yl)pyridin-2-yl]aminocarbonyl-5-carbomethoxymethyl-isoxazolin

3-(3-Amidinophenyl)-5-[2'-aminosulfonyl-[1,1']-biphenyl-4-yl]aminocarbonyl-5-(tetrazol-1-yl)methyl-isoxazolin

3-(3-Amidinophenyl)-5-[5-(2'-aminosulfonyiphenyl-1-yl)pyridin-2-yl]aminocarbonyl-5-(tetrazol-1-yl)methyl-isoxazolin

3-(3-Amidinophenyl)-5-[5-(2'-aminosulfonylphenyl-1-yl)pyrimidin-2-yl]aminocarbonyl-5-(tetrazol-1-yl)methyl-isoxazolin

3-(3-Amidinophenyl)-5-[2'-trifluormethyl-[1,1']-biphenyl-4-yl]aminocarbonyl-5-(tetrazol-1-yl)methyl-isoxazolin

3-(3-Amidinophenyl)-5-[5-(2'-trifluormethylphenyl-1-yl)pyridin-2-yl]aminocarbonyl-5-(tetrazol-1-yl)methyl-isoxazolin

3-(3-Amidinophenyl)-5-[5-(2'-trifluormethylphenyl-1-yl)pyrimidin-2-yl]aminocarbonyl-5-(tetrazol-1-yl)methyl-isoxazolin

3-(3-Amidinophenyl)-5-[5-(2'-trifluormethylsulfonylphenyl-1-yl)pyridin-2-yl]aminocarbonyl-5-(tetrazol-1-yl) methyl-isoxazolin

3-(3-Amidinophenyl)-5-[5-(2'-trifluormethylsulfonylphenyl-1-yl)pyrimidin-2-yl]aminocarbonyl-5-(tetrazol-1-yl) methyl-isoxazolin

3-(3-Amidinophenyl)-5-[2'-Aminosulfonyl-3-fluor-[1,1']-biphenyl-4-yl]aminocarbonyl-5-(tetrazol-1-yl)methyl-isoxazolin

3-(3-Amidinophenyl)-5-[2'-aminosulfonyl-3-chlor-[1,1']-biphenyl-4-yl]aminocarbonyl-5-(tetrazol-1-yl)methyl-isoxazolin

3-(3-Amidinophenyl)-5-[2'-trifluormethyl-3-fluor-[1,1']-biphenyl-4-yl]aminocarbonyl-5-(tetrazol-1-yl)methyl-isoxazolin

3-(3-Amidinophenyl)-5-[2'-trifluormethyl-3-chlor-[1,1']-biphenyl-4-yl]aminocarbonyl-5-(tetrazol-1-yl)methyl-isoxazolin

3-(3-Amidinophenyl)-5-[5-(2'-aminosulfonylphenyl-1-yl)pyridin-2-yl]aminocarbonyl-5-methoxymethyl-isoxazolin

3-(3-Amidinophenyl)-5-[2'-methylaminosulfonyl-[1,1']-biphenyl-4-yl]aminocarbonyl-5-(tetrazol-1-yl)methyl-isoxazolin

3-(3-Amidinophenyl)-5-[5-(2'-methylaminosulfonylphenyl-1-yl)pyridin-2-yl]aminocarbonyl-5-(tetrazol-1-yl)methyl-isoxazolin

3-(3-Amidinophenyl)-5-[2'-methylsulfonyl-[1,1']-biphenyl-4-yl]aminocarbonyl-5-(tetrazol-1-yl)methyl-isoxazolin

3-(3-Amidinophenyl)-5-[2'-methylsulfonyl-fluor-[1,1']-biphenyl-4-yl]aminocarbonyl-5-(tetrazol-1-yl)methyl-isoxazolin

3-(3-Amidinophenyl)-5-[2'-methylsulfonyl-chlor-[1,1']-biphenyl-4-yl]aminocarbonyl-5-(tetrazol-1-yl)methyl-isoxazolin

3-(3-Amidinophenyl)-5-[2'-trifluomethylsulfonyl-[1,1']-biphenyl-4-yl]aminocarbonyl-5-(tetrazol-1-yl)methyl-isoxazolin

3-(3-Amidinophenyl)-5-[2'-aminosulfonyl-[1,1']-biphenyl-4-yl]aminocarbonyl-5-(imidazol-1-yl)methyl-isoxazolin

3-(3-Amidinophenyl)-5-[2'-trifluormethylsulfonyl-3-fluor-[1,1']-biphenyl-4-yl]aminocarbonyl-5-(tetrazol-1-yl)methyl-isoxazolin

3-(3-Amidinophenyl)-5-[2'-trifluormethylsulfonyl-3-chlor-[1,1']-biphenyl-4-yl]aminocarbonyl-5-(tetrazol-1-yl)methyl-isoxazolin

3-(3-Amidinophenyl)-5-[2'-aminosulfonyl-[1,1']-biphenyl-4-yl]aminocarbonyl-5-(imidazol-1-yl)methyl-isoxazolin

3-(3-Amidinophenyl)-4-(2'-aminosulfonyl-[1,1']-biphenyl-4-yl)aminocarbonyl-5-methoxymethyl-isoxazolin

3-(3-Amidinophenyl)-4-(2'-aminosulfonyl-[1,1']-biphenyl-4-yl)aminocarbonyl-5-trifluormethylisoxazolin

3-(3-Amidinophenyl)-5-(2'-aminosulfonyl-[1,1']-biphenyl-4-yl)aminocarbonyl-4-methoxymethyl-isoxazolin

**3.** Pharmazeutische Zusammensetzung, umfassend eine therapeutisch wirksame Menge einer Verbindung nach Anspruch 1 oder 2 und einen pharmazeutisch akzeptablen Träger.

**4.** Verbindung nach Anspruch 1 oder 2 zur Verwendung bei der Behandlung oder Vorbeugung einer thromboembolischen Störung in einem Säugetier.

**5.** Verwendung einer Verbindung nach Anspruch 1 oder 2 zur Herstellung eines Medikaments zur Behandlung oder Vorbeugung einer thromboembolischen Störung in einem Säugetier.

## Revendications

**1.** Composés de formule (V) et (VI) :

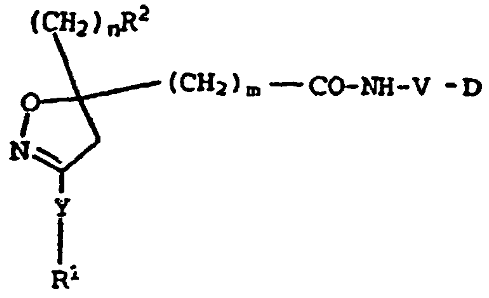

(V)

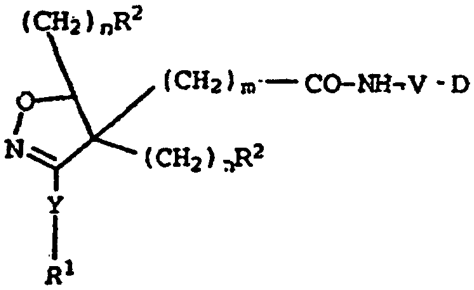

(VI)

y compris les sels pharmaceutiquement acceptables de ceux-ci, et toutes les formes stéréoisomériques de ceux-ci et les mélanges et de telles formes stéréoisomériques, dans lesquels :

$R^1$ est $C(NR^{14})NR^5R^6$ et

D est choisi parmi

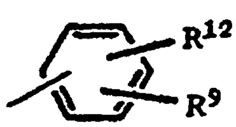 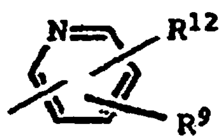 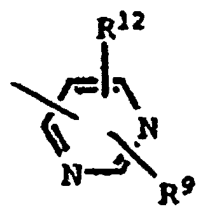

V est choisi parmi ce qui suit :

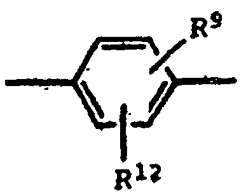 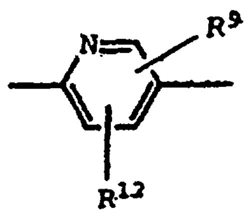 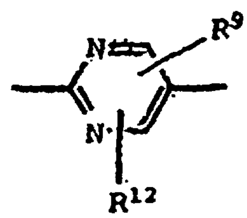

Y est choisi parmi :

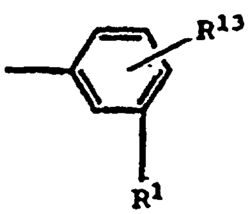 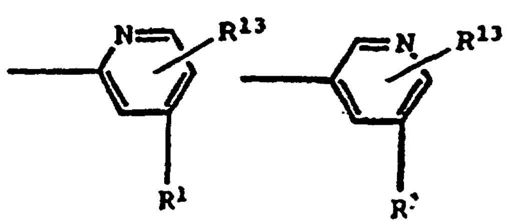 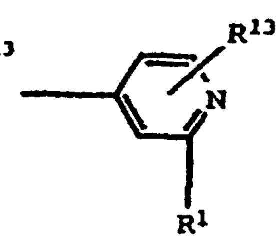

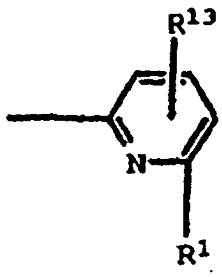 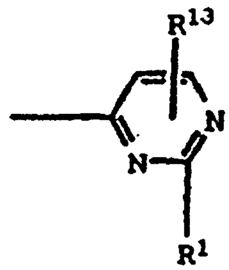 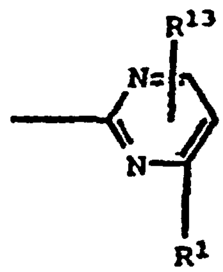

$R^2$ est choisi parmi
- H
- un alkyle $C_1$-$C_6$
- un alcoxy $C_1$-$C_6$
- $CO_2R^5$
- $CONHR^5$
- $CONHCH_2CO_2R^5$
- $CONH(CH_2)_q$-$R^{10}$
- $R^{10}$
- CO-$R^5$
- $COCO_2R^5$
- $COCONHR^5$
- $SO_nR^5$
- $SO_2NHR^5$
- $NHR^7$
- $CH{=}CHCO_2R^5$
- $CH{=}CHCONHR^5$
- O-$(CH_2)_n$-$R^{10}$
- $SO_n$-$(CH_2)_n$-$R^{10}$
- NH-$(CH_2)_n$-$R^{10}$

$R^5$ et $R^6$ à chaque apparition représentent indépendamment
- H
- un alkyle $C_1$-$C_6$
- un $(CH_2)_n$-phényle

$R^7$ est choisi parmi
- H
- un alkyle $C_1$-$C_6$
- $SO_2R^5$
- $COR^5$
- $(CH_2)_r$-$R^{10}$
- un $(CH_2)_n$-phényle

$R^8$ est choisi parmi
- H
- un alkyle $C_1$-$C_6$
- un halogène
- $NO_2$
- $CF_3$
- $OR^5$

$R^9$ est choisi parmi
- H
- un alkyle $C_1$-$C_6$
- un halogène
- $NO_2$
- $NHR^7$
- $SO_2NHR^{11}$
- $CF_3$
- $OR^5$

$CO_2R^5$
$CONR^5R^7$
CN
$(CH_2)_pNR^5R^6$
$C(NR^{14})NR^5R^6$
$NHC(NR^{14})NR^5R^6$
$NHC(NR^{14})H$
$SO_n\text{-}R^5$
$SO_n\text{-}CF_3$
un imidazole, un pyrazole, un 1,2,3-triazole, un 1,2,4-triazole et un tétrazole, chacun facultativement substitué par $CF_3$, un halogène, $NO_2$, un alkyle $C_1$-$C_5$ ou un alcoxy $C_1$-$C_5$ ;

$R^{10}$ est choisi parmi

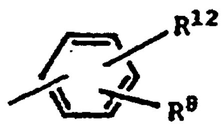
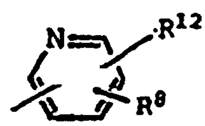
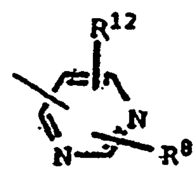
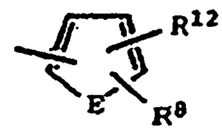

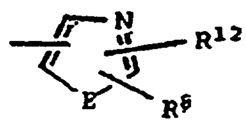
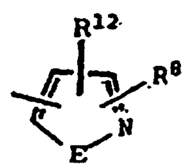
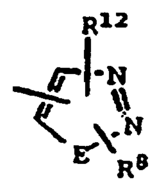
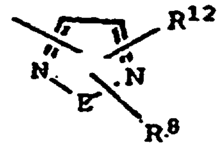

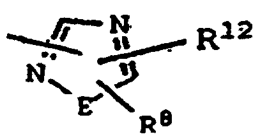
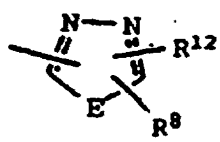
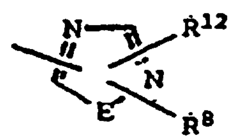
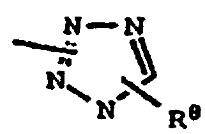

$R^{11}$ est choisi parmi
H
un alkyle $C_1$-$C_6$
un $(CH_2)_n$-phényle
$COR^5$
$CO_2R^5$

$R^{12}$ est choisi parmi
H
un alkyle $C_1$-$C_6$
un alcoxy $C_1$-$C_6$
un halogène
$NO_2$
$NHR^7$
CN
CF3
$SONHR^{11}$

$R^{13}$ est choisi parmi
H
OH
un alkyle $C_1$-$C_{10}$
un alcoxy $C_1$-$C_{10}$
un nitro

un halogène
$CF_3$

$R^{14}$ est choisi parmi
H
OH
un alkyle $C_1$-$C_{10}$
un alcoxy $C_1$-$C_{10}$
un $CO_2$-alkyle $C_1$-$C_{10}$
un CO-alkyle $C_1$-$C_{10}$
un CONH-alkyle $C_1$-$C_{10}$
un CONH-phényle
un $CO_2(CH_2)_n$-phényle

à chaque apparition chacun des éléments suivants est indépendamment :
m = 0 à 2
n = 0 à 4, sauf celui dans $-SO_n-$, n = 0 à 2 ;
p = 0 à 1
q = 0 à 4
r = 1 à 2.

**2.** Composé selon la revendication 1, choisi dans le groupe constitué par les composés suivants, y compris les formes de sels pharmaceutiquement acceptables de ceux-ci et toutes les formes stéréoisomériques de ceux-ci et les mélanges des telles formes stéréoisomériques :

la 3-(3-amidinophényl)-5-[[(2'-aminosulfonyl-[1, 1']-biphényl-4-yl)-méthyl]-aminocarbonyl]-5-(carbométhoxyméthyl)-isoxazoline

la 3-(3-amidinophényl)-5-[[(2'-aminosulfonyl-[1,1']-biphényl-4-yl)-méthyl]-aminocarbonyl]-5-(aminocarbonylméthyl)-isoxazoline

la 3-(3-amidinophényl)-5-[([1,1']-biphényl-4-yl)-aminocarbonyl]-5-(carbométhoxyméthyl)-isoxazoline

la 3-(3-amidinophényl)-5-[([1,1']-biphényl-4-yl)-aminocarbonyl]-5-(carboxyméthyl)-isoxazoline la 3-(3-amidinophényl)-5-[([1,1']-biphényl-4-yl)-aminocarbonyl]-5-(aminocarbonylméthyl)-iso-xazoline

la 3-(3-amidinophényl)-5-[([1,1']-biphényl-4-yl)-aminocarbonyl]-5-(hydroxyméthyl)-isoxazoline

la 3-(3-amidinophényl)-5-[(2'-aminosulfonyl-3'-n-propyl-[1,1']-biphényl-4-yl)-aminocarbonyl]-5-(carbométhoxyméthyl)-isoxazoline

la 3-(3-amidinophényl)-5-[(2'-t-butylaminosulfonyl-[1,1']-biphényl-4-yl)-aminocarbonyl]-5-(carbométhoxyméthyl)-isoxazoline

la 3-(3-amidinophényl)-5-[(2'-t-butylaminosulfonyl-[1,1']-biphényl-4-yl)-aminocarbonyl]-5-(aminocarbonylméthyl)-isoxazoline

la 3-(3-amidinophényl)-5-[(4'-amino-[1,1']-biphényl-4-yl)-aminocarbonyl]-5-(carbométhoxyméthyl)-isoxazoline

la 3-(3-amidinophényl)-5-[(2'-trifluorométhyl-[1,1']-biphényl-4-yl)-aminocarbonyl]-5-(carbométhoxyméthyl)-isoxazoline

la 3-(3-amidinophényl)-5-[(2'-aminosulfonyl-3-méthyl-[1,1']-biphényl-4-yl)-aminocarbonyl]-5-(carbométhoxyéthyl)-isoxazoline

la 3-(3-amidinophényl)-5-[(2'-aminosulfonyl-3-méthyl-[1,1']-biphényl-4-yl)-aminocarbonyl]-5-(carboxyéthyl)-isoxazoline

la 3-(3-amidinophényl)-5-[(2'-aminosulfonyl-3-méthyl-[1,1']-biphényl-4-yl)-aminocarbonyl]-5-(carbométhoxyéthylène)-isoxazoline

la 3-(3-amidinophényl)-5-[[5-(2'-aminosulfonylphényl-1-yl)-pyrimidin-2-yl]-aminocarbonyl]-5-(aminocarbonylméthyl)-isoxazoline

la 3-(3-amidinophényl)-5-[[5-(2'-aminosulfonylphényl-1-yl)-pyrimidin-2-yl]-aminocarbonyl]-5-(hydroxyéthyl)-isoxazoline

la 3-(3-amidinophényl)-5-[[5-(2'-aminosulfonylphényl-1-yl)-pyrimidin-2-yl]-aminocarbonyl]-5-(méthoxyéthyl)-isoxazoline

la 3-(3-amidinophényl)-5-[[5-(2'-aminosulfonylphényl-1-yl)-pyrimidin-2-yl]-aminocarbonyl]-5-(méthyl)-isoxazoline

la 3-(3-amidinophényl)-5-[(2'-aminosulfonyl-[1, 1']-biphényl-4-yl)-aminocarbonyl]-5-(carbométhoxyméthyl)-isoxazoline

la 3-(3-amidinophényl)-5-[(2'-aminosulfonyl-[1, 1']-biphényl-4-yl)-aminocarbonyl]-5-(carboxy-méthyl)-isoxazoline

la 3-(3-amidinophényl)-5-[(2'-aminosulfonyl-[1, 1']-biphényl-4-yl)-aminocarbonyl]-5-(aminocarbonylméthyl)-isoxazoline

la 3-(3-amidinophényl)-5-[(2'-aminosulfonyl-[1, 1']-biphényl-4-yl)-aminocarbonyl]-5-(hydroxy-éthyl)-isoxazoline

la 3-(3-amidinophényl)-5-[(2'-aminosulfonyl-[1, 1']-biphényl-4-yl)-aminocarbonyl]-5-(carbométhoxyméthylaminocarbonylméthyl)-isoxazoline

la 3-(3-amidinophényl)-5-[(2'-aminosulfonyl-[1, 1']-biphényl-4-yl)-aminocarbonyl]-5-[(imidazol-4-yl)-éthylaminocarbonylméthyl]-isoxazoline

la 3-(3-amidinophényl)-5-[(2'-aminosulfonyl-[1, 1']-biphényl-4-yl)-aminocarbonyl]-5-(méthoxyéthyl)-isoxazoline

la 3-(3-amidinophényl)-5-[(2'-aminosulfonyl[1,1']-biphényl-4-yl)-aminocarbonyl]-5-(méthyl)-isoxazoline

la 3-(3-amidinophényl)-5-[(2'-aminosulfonyl-3-méthyl-[1,1']-biphényl-4-yl)-aminocarbonyl]-5-(carbométhoxyméthyl)-isoxazoline

la 3-(3-amidinophényl)-5-[(2'-aminosulfonyl-3-méthyl-[1,1']-biphényl-4-yl)-aminocarbonyl]-5-(carboxyméthyl)-isoxazoline

la 3-(3-amidinophényl)-5-[(2'-aminosulfonyl-3-méthyl-[1,1'] -biphényl-4-yl)-aminocarbonyl]-5-(aminocarbonylméthyl)-isoxazoline

la 3-(3-amidinophényl)-5-[(2'-aminosulfonyl-3-méthyl-[1,1']-biphényl-4-yl)-aminocarbonyl]-5-(carbométhoxyméthylaminocarbonylméthyl)-isoxazoline

la 3-(3-amidinophényl)-5-[(2'-aminosulfonyl-3-méthyl-[1,1']-biphényl-4-yl)-aminocarbonyl]-5-(hydroxyéthyl)-isoxazoline

la 3-(3-amidinophényl)-5-[(2'-aminosulfonyl-3-méthyl-[1,1']-biphényl-4-yl)-aminocarbonyl]-5-(méthoxyéthyl)-isoxazoline

la 3-(3-amidinophényl)-5-[(2'-aminosulfonyl-3-méthyl-[1,1']-biphényl-4-yl)-aminocarbonyl]-5-(méthyl)-isoxazoline

Ia 3-(3-amidinophényl)-5-[(2'-aminosulfonyl-3-fluoro-[1,1']-biphényl-4-yl)-aminocarbonyl]-5-(méthoxyéthyl)-isoxazoline

Ia 3-(3-amidinophényl)-5-[(2'-aminosulfonyl-3-fluoro-[1,1']-biphényl-4-yl)-aminocarbonyl]-5-(carbométhoxyméthyl)-isoxazoline

Ia 3-(3-amidinophényl)-5-[(2'-aminosulfonyl-3-fluoro-[1,1']-biphényl-4-yl)-aminocarbonyl]-5-(carboxyméthyl)-isoxazoline

Ia 3-(3-amidinophényl)-5-[(2'-aminosulfonyl-3-fluoro-[1,1']-biphényl-4-yl)-aminocarbonyl]-5-(aminocarbonylméthyl)-isoxazoline

Ia 3-(3-amidinophényl)-5-[(2'-aminosulfonyl-3-fluoro-[1,1']-biphényl-4-yl)-aminocarbonyl]-5-(hydroxyéthyl)-isoxazoline

Ia 3-(3-amidinophényl)-5-[(2'-aminosulfonyl-3-fluoro-[1,1']-biphényl-4-yl)-aminocarbonyl]-5-(méthyl)-isoxazoline

Ia 3-(3-amidinophényl-5-[[2-(2'-aminosulfonylphényl-1-yl)-pyridin-5-yl]-aminocarbonyl]-5-(carbométhoxyméthyl)-isoxazoline

Ia 3-(3-amidinophényl-5-[[2-(2'-aminosulfonyl--phényl-1-yl)-pyridin-5-yl]-aminocarbonyl]-5-(carboxyméthyl)-isoxazoline

Ia 3-(3-amidinophényl-5-[[2-(2'-aminosulfonylphényl-1-yl)-pyridin-5-yl]-aminocarbonyl]-5-(aminocarbonylméthyl)-isoxazoline

Ia 3-(3-amidinophényl-5-[[2-(2'-aminosulfonylphényl-1-yl)-pyridin-5-yl]-aminocarbonyl]-5-(hydroxyéthyl)-isoxazoline

Ia 3-(3-amidinophényl-5-[[2-(2'-aminosulfonylphényl-1-yl)-pyridin-5-yl]-aminocarbonyl]-5-(méthoxyéthyl)-isoxazoline

Ia 3-(3-amidinophényl-5-[[2-(2'-aminosulfonylphényl-1-yl)-pyridin-5-yl]-aminocarbonyl]-5-(méthyl)-isoxazoline

Ia 3-(3-amidinophényl-5-[[5-(2'-aminosulfonylphényl-1-yl)-pyridin-2-yl]-aminocarbonyl]-5-(carbométhoxyméthyl)-isoxazoline

Ia 3-(3-amidinophényl-5-[[5-(2'-aminosulfonylphényl-1-yl)-pyridin-2-yl]-aminocarbonyl]-5-(carboxyméthyl)-isoxazoline

Ia 3-(3-amidinophényl-5-[[5-(2'-aminosulfonylphényl-1-yl)-pyridin-2-yl]-aminocarbonyl]-5-(aminocarbonylméthyl)-isoxazoline

Ia 3-(3-amidinophényl-5-[[5-(2'-aminosulfonylphényl-1-yl)-pyridin-2- yl]-aminocarbonyl]-5-(hydroxyéthyl)-isoxazoline

Ia 3-(3-amidinophényl-5-[[5-(2'-aminosulfonylphényl-1-yl)-pyridin-2-yl]-aminocarbonyl]-5-(méthoxyéthyl)-isoxazoline

Ia 3-(3-amidinophényl-5-[[5-(2'-aminosulfonylphényl-1-yl)-pyridin-2-yl]-aminocarbonyl]-5-(méthyl)-isoxazoline

Ia 3-(3-amidinophényl-5-[[5-(2'-aminosulfonylphényl-1-yl)-pyrimidin-2-yl]-aminocarbonyl]-5-(carbométhoxyméthyl)-isoxazoline

Ia 3-(3-amidinophényl-5-[[5-(2'-aminosulfonylphényl-1-yl)-pyrimidin-2-yl]-aminocarbonyl]-5-(carboxyméthyl)-isoxazoline

la 3-(3-amidinophényl)-5-[5-(2'-aminosulfonylphényl-1-yl)-pyrimidin-2-yl]-aminocarbonyl]-5-carbométhoxyméthylisoxazoline

la 3-(3-amidinophényl)-5-[5-(2'-aminosulfonylphényl-1-yl)-pyridin-2-yl]-aminocarbonyl]-5-carbométhoxyméthylisoxazoline

la 3-(3-amidinophényl)-5-[2'-aminosulfonyl-[1, 1']-biphényl-4-yl]-aminocarbonyl-5-(tétrazol-1-yl)-méthylisoxazoline

la 3-(3-amidinophényl)-5-[5-(2'-aminosulfonylphényl-1-yl)-pyridin-2-yl]-aminocarbonyl]-5-(tétrazol-1-yl)-méthylisoxazoline

la 3-(3-amidinophényl)-5-[5-(2'-aminosulfonylphényl-1-yl)-pyrimidin-2-yl]-aminocarbonyl]-5-(tétrazol-1-yl)-méthylisoxazoline

la 3-(3-amidinophényl)-5-[2'-trifluorométhyl[1,1']-biphényl-4-yl]-aminocarbonyl-5-(tétrazol-1-yl)-méthyl-isoxazoline

la 3-(3-amidinophényl)-5-[5-(2'-trifluorométhylphényl-1-yl)-pyridin-2-yl]-aminocarbonyl]-5-(tétrazol-1-yl)-méthylisoxazoline

la 3-(3-amidinophényl)-5-[5-(2'-trifluorométhylphényl-1-yl)-pyrimidin-2-yl]-aminocarbonyl]-5-(tétrazol-1-yl)-méthylisoxazoline

la 3-(3-amidinophényl)-5-[5-(2'-trifluorométhylsulfonylphényl-1-yl)-pyridin-2-yl]-aminocarbonyl]-5-(tétrazol-1-yl)-méthylisoxazoline

la 3-(3-amidinophényl)-5-[5-(2'-trifluorométhylsulfonylphényl-1-yl)-pyrimidin-2-yl]-aminocarbonyl]-5-(tétrazol-1-yl)-méthylisoxazoline

la 3-(3-amidinophényl)-5-[2'-aminosulfonyl-3-fluoro-[1,1']-biphényl-4-yl]-aminocarbonyl-5-(tétrazol-1-yl)-méthylisoxazoline

la 3-(3-amidinophényl)-5-[2'-aminosulfonyl-3-chloro-[1,1']-biphényl-4-yl]-aminocarbonyl-5-(tétrazol-1-yl)-méthylisoxazoline

la 3-(3-amidinophényl)-5-[2'-trifluorométhyl-3-fluoro-[1,1']-biphényl-4-yl]-aminocarbonyl-5-(tétrazol-1-yl)-méthylisoxazoline

la 3-(3-amidinophényl)-5-[2'-trifluorométhyl-3-chloro-[1,1']-biphényl-4-yl]-aminocarbonyl-5-(tétrazol-1-yl)-méthylisoxazoline

la 3-(3-amidinophényl)-5-[5-(2'-aminosulfonylphényl-1-yl)-pyridin-2-yl]-aminocarbonyl]-5-méthoxyméthylisoxazoline

la 3-(3-amidinophényl)-5-[2'-méthylaminosulfonyl-[1,1']-biphényl-4-yl]-aminocarbonyl-5-(tétrazol-1-yl)-méthylisoxazoline

la 3-(3-amidinophényl)-5-[5-(2'-méthylaminosulfonylphényl-1-y1)-pyridin-2-yl]-aminocarbonyl]-5-(tétrazol-1-yl)-méthylisoxazoline

la 3-(3-amidinophényl)-5-[2'-méthylsulfonyl-[1, 1']-biphényl-4-yl] aminocarbonyl-5-(tétrazol-1-yl)-méthylisoxazoline

la 3-(3-amidinophényl)-5-[2'-méthylsulfonylfluoro-[1,1']-biphényl-4-yl]-aminocarbonyl-5-(tétrazol-1-yl)-méthylisoxazoline

la 3-(3-amidinophényl)-5-[2'-méthylsulfonylchloro-[1,1']-biphényl-4-yl]-aminocarbonyl-5-(tétrazol-1-yl)-méthylisoxazoline

la 3-(3-amidinophényl)-5-[2'-trifluorométhylsulfonyl-[1,1']-biphényl-4-yl]-aminocarbonyl-5-(tétrazol-1-yl)-méthylisoxazoline

la 3-(3-amidinophényl)-5-[2'-aminosulfonyl-[1,1']-biphényl-4-yl] aminocarbonyl-5-(imidazol-1-yl)-méthylisoxazoline

la 3-(3-amidinophényl)-5-[2'-trifluorométhyl-sulfonyl-3-fluoro-[1,1']-biphényl-4-yl]-aminocarbonyl-5-(tétrazol-1-yl)-méthylisoxazoline

la 3-(3-amidinophényl)-5-[2'-trifluorométhylsulfonyl-3-chloro-[1,1']-biphényl-4-yl]-aminocarbonyl-5-(tétrazol-1-yl)-méthylisoxazoline

la 3-(3-amidinophényl)-5-[2'-aminosulfonyl-[1,1']-biphényl-4-yl]-aminocarbonyl-5-(imidazol-1-yl)-méthylisoxazoline

la 3-(3-amidinophényl)-4-(2'-aminosulfonyl-[1,1']-biphényl-4-yl)-aminocarbonyl-5-méthoxyméthylisoxazoline

la 3-(3-amidinophényl)-4-(2'-aminosulfonyl-[1,1']-biphényl-4-yl)-aminocarbonyl-5-trifluorométhylisoxazoline

la 3-(3-amidinophényl)-5-(2'-aminosulfonyl-[1,1']-biphényl-4-yl)-aminocarbonyl-4-méthoxyméthylisoxazoline.

**3.** Composition pharmaceutique comprenant une quantité pharmaceutiquement efficace d'un composé selon l'une quelconque des revendications 1 ou 2, et un vecteur pharmaceutiquement acceptable.

**4.** Composé selon l'une quelconque des revendications 1 ou 2, pour une utilisation dans le traitement ou la prévention d'un trouble thromboembolique chez un mammifère.

**5.** Utilisation d'un composé selon l'une quelconque des revendications 1 ou 2, dans la fabrication d'un médicament pour le traitement ou la prévention d'un trouble thromboembolique chez un mammifère.